(19) Europäisches Patentamt
European Patent Office
Office européen des brevets

(11) EP 1 678 113 B1

(12) **EUROPÄISCHE PATENTSCHRIFT**

(45) Veröffentlichungstag und Bekanntmachung des Hinweises auf die Patenterteilung:
**16.07.2008 Patentblatt 2008/29**

(21) Anmeldenummer: **04765959.4**

(22) Anmeldetag: **14.10.2004**

(51) Int Cl.:
***C07C 45/50*** *(2006.01)* ***B01J 31/24*** *(2006.01)*
***C07C 47/02*** *(2006.01)*

(86) Internationale Anmeldenummer:
**PCT/EP2004/011530**

(87) Internationale Veröffentlichungsnummer:
**WO 2005/042458 (12.05.2005 Gazette 2005/19)**

(54) **VERFAHREN ZUR KONTINUIERLICHEN HERSTELLUNG VON ALDEHYDEN**

METHOD FOR THE CONTINUOUS PRODUCTION OF ALDEHYDES

PROCEDE DE PRODUCTION EN CONTINU D'ALDEHYDES

(84) Benannte Vertragsstaaten:
**AT BE BG CH CY CZ DE DK EE ES FI FR GB GR HU IE IT LI LU MC NL PL PT RO SE SI SK TR**

(30) Priorität: **21.10.2003 DE 10349482**
**24.08.2004 DE 102004041144**

(43) Veröffentlichungstag der Anmeldung:
**12.07.2006 Patentblatt 2006/28**

(73) Patentinhaber: **BASF SE**
**67056 Ludwigshafen (DE)**

(72) Erfinder:
- **VOLLAND, Martin**
**69117 Heidelberg (DE)**
- **MACKEWITZ, Thomas**
**67354 Römerberg (DE)**
- **AHLERS, Wolfgang**
**67549 Worms (DE)**
- **SCHÄFER, Ansgar**
**76199 Karlsruhe (DE)**
- **RICHTER, Wolfgang**
**67157 Wachenheim (DE)**
- **PACIELLO, Rocco**
**67098 Bad Dürkheim (DE)**

(56) Entgegenhaltungen:
**WO-A-02/068371** **US-A- 5 808 168**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 678 113 B1

Printed by Jouve, 75001 PARIS (FR)

## Beschreibung

Verfahren zur kontinuierlichen Herstellung von Aldehyden mit 5 bis 21 Kohlenstoffatomen durch die isomerisierende Hydroformylierung in homogener Phase von α-Olefine und Olefine mit internen Doppelbindungen enthaltenden Olefinzusammensetzungen mit 4 bis 20 Kohlenstoffatomen mittels Synthesegas in Gegenwart eines mit einem Sauerstoff- und/oder Stickstoffatome enthaltenden Organophosphorliganden komplexierten, homogenen Rhodium-Katalysators und freiem Liganden, bei erhöhter Temperatur und bei erhöhtem Druck in einem mehrstufigen Reaktionssystem aus mindestens zwei Reaktionszonen.

Im Jahre 1990 wurde die Weltjahresproduktion an Produkten aus der Hydroformylierung von Olefinen, auch als Oxo-Synthese bezeichnet, auf ca. 7 Millionen Tonnen geschätzt. Wurden zu Beginn der industriellen Anwendung der Oxo-Synthese im wesentlichen Homogenkatalysatoren auf Kobaltbasis eingesetzt, so setzten sich ab den siebziger Jahren des vergangenen Jahrhunderts immer mehr homogene Hydroformylierungskatalysatoren auf Rhodiumbasis bei der industriellen Anwendung durch, da sie ein wirtschaftliches Arbeiten bei niedrigeren Temperaturen und insbesondere bei einem niedrigeren Druck als Kobaltkatalysatoren ermöglichen und eine hohe n/i-Selektivität bei der Hydroformylierung von Olefinen mit endständiger Doppelbindung (α-Olefine) gewährleisten. Aus diesem Grund hat die sogenannte Niederdruckhydroformylierung mit im allgemeinen Triphenylphosphin-modifizierten Rhodiumkomplexen die sogenannte Hochdruckhydroformylierung mit Kobaltcarbonyl-Katalysatoren bei der Herstellung von $C_3$- bzw. $C_4$-Aldehyden aus Ethen bzw. Propen fast vollständig aus der industriellen Praxis verdrängt. Dennoch ist die Hochdruckhydroformylierung mit Kobaltcarbonyl-Katalysatoren auch heutzutage noch von erheblicher industrieller Bedeutung und zwar insbesondere bei der Hydroformylierung langkettiger Olefine mit internen Doppelbindungen. Solche Olefine stehen in großen Mengen z.B. als Raffinat 11 (aus dem C4-Schnitt eines Steamcrackers nach Abtrennung oder Selektivhydrierung von Dienen, wie 1,3-Butadien und Abtrennung von Isobuten erhaltenes Buten-1 und Buten-2 enthaltendes $C_4$-Gemisch) bzw. aus Olefindimerisierungsprozessen und Trimerisierungsprozessen oder aus Olefinmetatheseanlagen und Fischer-Tropsch-Anlagen zur Verfügung und deren Oxo-Produkte dienen als Ausgangsmaterialien zur Herstellung anderer großtechnischer Produkte, wie Weichmacher- oder Tensidalkoholen. Aufgrund ihrer Genese liegen diese Olefine in der Regel als Isomerengemisch aus dem betreffenden α-Olefin und den entsprechenden internen Doppelbindungsstellungsisomeren vor und werden in dieser Form auch als Ausgangsmaterial in der Oxo-Synthese verwendet. Als "interne" Olefine werden demnach solche Olefine bezeichnet, deren Doppelbindung im Unterschied zu α-Olefinen nicht endständig ist, sondern im Inneren des Olefinmoleküls liegt.

Der Grund für die Bevorzugung von Kobaltkatalysatoren zur Hydroformylierung solcher längerkettigen Olefingemische aber auch zur Hydroformylierung individueller olefinischer Verbindungen mit internen Doppelbindungen trotz der bei Verwendung von Kobaltkatalysatoren benötigten höheren Temperaturen und des erforderlichen höheren Drucks ist im unterschiedlichen katalytischen Verhalten der Katalysatormetalle Kobalt und Rhodium zu suchen. Bei der Hydroformylierung von α-Olefinen können sich, je nach Ort der Addition des CO-Moleküls an die Doppelbindung gemäß Gleichung (1)

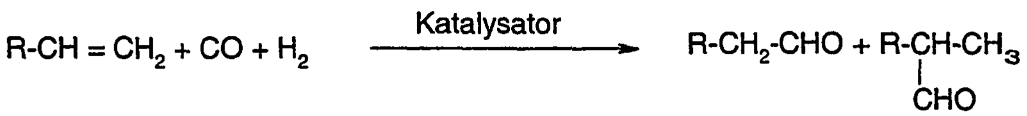


(1)

A B

lineare Aldehyde A, sogenannte n-Aldehyde, oder verzweigte Aldehyde B, auch als iso-Aldehyde bezeichnet, bilden, wobei im Allgemeinen ein hoher Anteil an n-Aldehyden im Hydroformylierungsprodukt ausgedrückt durch das n-/i-Verhältnis - also das Molverhältnis aus n-zu iso-Aldehyden im Reaktionsprodukt, erwünscht ist. Obgleich das Rhodium-Triphenylphosphin-Katalysatorsystem zu hohen n-Selektivitäten bei der Hydroformylierung von kurzkettigen α-Olefinen führt, hat dieses Katalysatorsystem Nachteile bei der Hydroformylierung der genannten Olefingemische bzw. von Olefinen mit internen Doppelbindungen. Einesteils hat das Rhodium-Triphenylphosphin-Katalysatorsystem gegenüber internen Doppelbindungen nur eine sehr geringe Hydroformylierungsaktivität, mit der Folge, dass solche "interne" Olefine entweder gar nicht oder nur unter Anwendung unwirtschaftlich langer Reaktionszeiten in wirtschaftlich hinreichendem Maße umgesetzt werden, ein Nachteil der andernteils dadurch verschlimmert wird, dass dieses Katalysatorsystem auch noch die Eigenschaft hat, endständige Doppelbindungen zu internen Doppelbindungen unter den Bedingungen der Hydroformylierungsreaktion in signifikantem Umfang zu isomerisieren, so dass bei Anwendung des Rhodium-Triphenylphosphin-Katalysatorsystems bei der Hydroformylierung solcher technischen Olefinisomerengemische oder internen Olefine nur unzureichende Olefinumsätze und Olefinausnutzungsgrade der internen Olefine erzielt werden.

Um die der Niederdruckhydroformylierung mit Rhodiumkatalysatoren inhärenten Vorteile auch für die Hydroformylierung längerkettiger Olefingemische aus α- und internen Olefinen bzw. die Hydroformylierung von internen Olefinen nutzbar machen zu können, gibt es seit langem Bestrebungen zur Entwicklung Rhodium-katalysierter, "isomerisierender" Hydroformylierungsverfahren, wobei unter "isomerisierender Hydroformylierung" Verfahren verstanden werden, bei denen bei der Hydroformylierung von internen Olefinen oder von Gemischen aus α- und internen Olefinen ein höherer n-Aldehydanteil im Reaktionsprodukt erzeugt wird, als nach dem Gehalt an endständigen und internen Doppelbindungen im Ausgangsolefin und unter Berücksichtigung der vom Rhodium-Triphenylphosphin-Katalysatorsystems bewirkten Doppelbindungisomerisierung zu erwarten wäre. Die zur Erreichung dieses Zieles bislang in der Forschung verfolgten Ansätze betreffen die Modifizierung der Eigenschaften des Rhodiumkatalysators durch geeignete Liganden und/oder verfahrenstechnische Maßnahmen. Für einen wirtschaftlich erfolgreichen Einsatz in der isomerisierenden Hydroformylierung in der großtechnischen Praxis gibt es aber noch erheblichen Verbesserungsbedarf.

Als zur isomerisierenden Hydroformylierung geeignete Rhodium-Katalysatorsysteme seien an dieser Stelle lediglich beispielhaft die Rhodiumkomplexe mit Organophosphitliganden gemäß US-A 3 527 809, die Rhodiumkomplexe mit Organochelatphosphitliganden gemäß US-A 4,668,651 sowie die Rhodiumkomplexe mit Organophosphoamiditliganden und Organophosphit- bzw. Organophosphonitliganden gemäß WO 01/58589 und WO 02/83695 genannt.

Da die n/i-Selektivität bei der Hydroformylierung von Olefinen von einer Vielzahl von Parametern beeinflusst wird, muss der Einsatz solcher Katalysatoren für die isomerisierende Hydroformylierung durch verfahrenstechnische Maßnahmen und Maßnahmen zur Reaktionsführung flankiert werden, um zu wirtschaftlich zufriedenstellenden Ergebnissen zu gelangen. Als ein Parameter der die n/i-Selektivität bei der Hydroformylierung maßgeblich beeinflusst, wurde schon von d' Oro et al, LA CHIMICA ET L'INDUSTRIA 62, 572 (1980) für das Rhodium-Triphenylphosphin-Katalysatorsystem der CO-Partialdruck im Hydroformylierungsreaktor identifiziert. Dabei wurde festgestellt, dass eine Absenkung des CO-Partialdruckes zwar zu einer Steigerung der n-Selektivität führt, eine solche CO-Partialdruckabsenkung allerdings eine unerwünscht starke Erhöhung der Hydrierrate der eingesetzten Olefine, mithin eine hohe Paraffinbildung nach sich zieht.

Aus US-A 4,885,401 geht hervor, dass eine Absenkung des CO-Partialdrucks im Hydroformylierungsreaktor bei Verwendung von Rhodium-Chelatphosphit-Katalysatorsystemen zu einer Steigerung der Reaktionsgeschwindigkeit führt und das $CO/H_2$-Verhältnis vorzugsweise 1:1 bis 1:10 betragen sollte.

Beispiel 12 von US-A 4,885,401, betreffend die Hydroformylierung von 2-Buten, gibt an, dass bei einer Absenkung des $CO/H_2$-Verhältnisses zwar die Reaktionsgeschwindigkeit erhöht wird, das n/i-Verhältnis der gebildeten Aldehyde aber abnimmt.

US-A 4,885,401 erwähnt allgemein die Möglichkeit Mischungen aus α- und internen Olefinen in einer Reihe von verschiedenen Reaktoren zu hydroformylieren und dabei gewünschtenfalls die optimalen Reaktionsbedingungen in den einzelnen Reaktoren unabhängig voneinander einzustellen, eine spezifische Lehre auf welche Weise dies getan werden soll und mit welcher Zielrichtung der Optimierungsmaßnahmen wird jedoch nicht gegeben.

US-A 4,599,206 enthält Beispiele zur Hydroformylierung von Buten-1/Buten-2-Gemischen mittels eines Rhodium-Organomonophosphitligand-Katalysators in zwei aufeinanderfolgenden Reaktoren, die bei unterschiedlichen $CO/H_2$-Molverhältnissen betrieben werden. Aufgrund der stark variierenden Reaktionsbedingungen dieser Beispiele kann aus den einzelnen Resultaten jedoch keine verallgemeinbare Lehre zur Durchführung der isomerisierenden Hydroformylierung zwecks Erzielung eines hohen n/i-Verhäitnisses der gebildeten Aldehyde bei hohem Butenumsatz entnommen werden.

EP-A 188 426 beschreibt ein Verfahren zur Hydroformylierung von Olefinen bei dem das olefinhaltige Reaktorabgas aus einem ersten Reaktorsystem einem zweiten Reaktorsystem, das vom ersten Reaktorsystem entkoppelt ist, zur Vervollständigung der Hydroformylierung zugeleitet wird. Dabei können im zweiten entkoppelten Reaktorsystem andere Reaktionsbedingungen angewandt werden als im ersten, insbesondere können dort andere Katalysatorsysteme angewandt werden. Durch die Verwendung dieser entkoppelten Reaktorsysteme soll eine effizientere und vollständigere Ausnutzung des eingesetzten Olefins erreicht werden. Von einer Hydroformylierung unter isomerisierenden Bedingungen ist in EP-A 188 426 nicht die Rede und dementsprechend sind die Beispiele dieser Schrift auf die Anwendung des Rhodium-Triphenylphosphin-Katalysatorsystems zur Hydroformylierung von Propen beschränkt. Es wird auch keine Lehre gegeben, wie das n/i-Verhältnis bei Verwendung interner Olefine als Ausgangsmaterial erhöht werden kann.

WO 97/20801 (≙ US-A 5,744,650) befasst sich spezifisch mit dem Zusammenhang zwischen Hydroformylierungsgeschwindigkeit und Kohlenmonoxidpartialdruck bei der Hydroformylierung mit Rhodium-Organopolyphosphit-Komplex-Katalysatoren, der Verhinderung oder Verminderung der Deaktivierung des Rhodium-Organopolyphosphit-Komplex-Katalysators, der Verhinderung und/oder Verminderung von periodischen Schwankungen des Kohlenmonoxidpartialdrucks, des Wasserstoffpartialdrucks, des gesamten Reaktionsdrucks, der Hydroformylierungsgeschwindigkeit und/oder der Temperatur während des Hydroformylierungsverfahrens und schlägt zur Lösung dieser Probleme vor, das Hydroformylierungsverfahren bei einem Kohlenmonoxidpartialdruck durchzuführen, so dass die Hydroformylierungsgeschwindigkeit steigt, wenn der Kohlenmonoxidpartialdruck sinkt und die Hydroformylierungsgeschwindigkeit sinkt, wenn der Kohlenmonoxidpartialdruck steigt, und dass eine oder mehrere der Bedingungen erfüllt werden, nämlich

a) die Anwendung einer Temperatur, so dass die Temperaturdifferenz zwischen der Temperatur der Reaktionsproduktflüssigkeit und der Eintrittstemperatur des Kühlmittels weniger als 25°C beträgt,
b) einen Kohlenmonoxidumsatz von weniger als 90 %,
c) einen Wasserstoffumsatz von mehr als 65 % und
d) einen Umsatz der olefinisch ungesättigten Verbindung von mehr als 50 %.

Bezüglich der Auswirkungen des Kohlenmonoxidpartialdrucks auf die n/i-Seiektivität wird in WO 97/20801 lediglich festgestellt, dass das Arbeiten in einem Bereich bei der die Hydroformylierung eine negative Reaktionsordnung bezüglich des KohlenmonoxidPartialdrucks hat, also bei einem relativ hohen Kohlenmonoxid-Partialdruck, zu einem hohen Isomerenverhältnis führt.

Das Continuation-in-part-Patent US-A 5 874 639 erweitert den Gegenstand von US-A 5,744,650 auf die Verwendung von Katalysatoren aus Organopolyphosphor-Metallkomplexen.

US-A 5 728 893 hat die Verwendung eines speziell konstruierten Mehrstufenreaktors für Hydroformylierungen zum Gegenstand, wobei der Zweck der Verwendung dieses Reaktors ist, Reaktionsbedingungen zu erzielen, bei denen eine Änderung der n-Produktselektivität von 0,2 % n-Produkt pro 1 Pound pro Quadratinch (≙2,9 % pro bar CO Partialdruck) CO-Partialdruck nicht überschritten wird. Die einzigen Beispiele dieses Patents betreffen die herkömmliche Hydroformylierung von Ethylen und Propen mit dem Rhodium-Triphenylphosphin-Katalysatorsystem, wobei von Anfang an ein Wasserstoffüberschuss angewandt wird. Eine Erhöhung des CO-Partialdrucks führt gemäß Angaben der Beispiele zu einer Verringerung der Hydroformylierungsgeschwindigkeit. Über das in Beispiel 2 bei der Hydroformylierung von Propen erzielte n/i-Verhältnis der erzeugten Butyraldehyde werden keine Angaben gemacht.

Laut JP-A 143 572 (2000) soll die Hydroformylierung unter Bedingungen ausgeführt werden, bei denen der Kohlenmonoxidpartialdruck im Reaktor praktisch keine Auswirkungen auf die Reaktionsgeschwindigkeit hat.

Reinius et al (J. Mol. Cat. A: Chemical 158, 499 (2000)) untersuchten die Hydroformylierung von Methylmethacrylat mit einem Rhodium-(Methyl(thiophenyl-) diphenylphosphin-Komplex-Katalysator und kamen dabei zu dem Ergebnis, dass eine Erhöhung des $H_2$-Partialdrucks im Synthesegas die n/i-Selektivität der Hydroformylierungsreaktion im Gegensatz zur Verwendung von Triphenylphosphin-Rhodium-Komplex-Katalysator nicht ändert.

Yang et al beschreiben in Catalysis Today 74, 111 (2002) den Einfluss des $H_2$- und CO-Partialdrucks auf die Aktivität des Rhodium Tris(natrium-m-sulfonatophenyl)-phosphin-Komplex-Katalysators bei der Hydroformylierung von Propen. Sie fanden heraus, dass bei diesem Katalysatorsystem die Hydroformylierungsgeschwindigkeit mit steigendem $H_2$-Partialdruck zunimmt und mit steigendem CO-Partialdruck abnimmt.

EP-A 423 769 betrifft die Verwendung einer Kombination von zwei verschiedenen zu einer Reaktorkaskade miteinander verbundenen Reaktoren bei der mehrstufigen Hydroformylierung von Olefinen. Gemäß den Beispielen dieser Anmeldung werden bei der Hydroformylierung von Propen mit dem herkömmlichen Rhodium-Triphenylphosphin-Katalysatorsystem sowohl in der ersten als auch in der zweiten Reaktionsstufe Synthesegasgemische eingesetzt, in denen der Wasserstoffanteil den Kohlenmonoxidanteil übersteigt. EP-B 423 769 enthält keine Angaben zur isomerisierenden Hydroformylierung.

EP-A 1 008 580 betrifft ebenfalls die Verwendung einer Reaktorkaskade aus mindestens zwei Reaktoren, die in Drucksektionen aufgeteilt sind, für die Hydroformylierung, wobei bezüglich der $H_2$- bzw. CO-Partialdruckverhältnisse in einer ersten (m-1)-ten Drucksektion zu den betreffenden Partialdruckverhältnissen in einer nachfolgenden zweiten (m-ten) Drucksektion mindestens eine der folgenden Beziehungen gelten soll,

$$P_{CO}(m-1)<P_{CO}(m)$$

$$P_{H2}(m-1)<P_{H2}(m)$$

$$P_{CO}(m-1)+P_{H2}(m-1)<P_{CO}(m)+P_{H2}(m)$$

worin $P_{CO}$ und $P_{H2}$ für den CO- bzw. $H_2$-Partialdruck in der jeweils bezeichneten Drucksektion oder Reaktionsstufe stehen. Durch diese Betriebsweise soll sowohl die Hydroformylierungsgeschwindigkeit erhöht als auch die nr-Selektivität verbessert werden. Gemäß Beispielen von EP-A 1 008 580 wird dies bei der Hydroformylierung eines Octengemisches dadurch erreicht, dass bei Verwendung von Synthesegas mit einem $CO/H_2$-Molverhältnis von 1:1 in der ersten Reaktionsstufe ein Druck von 50 $kg/cm^2$ und in der zweiten Reaktionsstufe ein Druck von 170 $kg/cm^2$ eingestellt wird. Als Hydroformylierungskatalysator wird hierbei unkomplexiertes Rhodiumhydridocarbonyl, das in situ im Reaktor erzeugt

wird, eingesetzt. In der Beschreibung von EP-A 1 008 580 wird zwar erwähnt, dass dieses Verfahren auch für Hydroformylierungen mit von verschiedenerlei Phosphit- oder Phosphin-Liganden komplexierten Kobalt- und Rhodiumkatalysatoren geeignet sei, konkrete Beispiele oder Angaben über die Auswirkungen dieses Verfahrens bei Verwendung solcher Katalysatoren, die sich anders verhalten als unkomplexiertes Rhodium, auf das n/i-Verhältnis der damit zur erzeugenden Aldehyde werden jedoch nicht gegeben.

Die Anwendung eines bezüglich des CO-Partialdrucks in der ersten Reaktionsstufe höheren CO-Partialdrucks in einer zweiten Reaktionsstufe wird auch von US-A 4,716,250 offenbart (Beispiel 9), betreffend die Hydroformylierung von Octen-1 mittels eines mit einem monosulfonierten Phosphin komplexierten Rhodiumkatalysators. Hinsichtlich des in den einzelnen Reaktionsstufe eingesetzten $CO/H_2$-Verhältnisses liegt sowohl in der ersten als auch in der zweiten Reaktionsstufe ein Wasserstoffüberschuss vor. Das bezüglich des n/i-Verhältnisses der gebildeten Nonanale beste Ergebnis (n/i-Verhältnis 12,3) wird jedoch in einer anderen Ausgestaltung von Beispiel 9 bei Anwendung eines annähernd gleichen CO-Partialdrucks in der ersten und zweiten Stufe und bei Verwendung eines hohen $H_2/CO$-Molverhältnisses sowohl in der ersten als auch in der zweiten Stufe erzielt.

WO 02/68371 betrifft ein Verfahren zur isomerisierenden Hydroformylierung in einer Reaktorkaskade, wobei in der ersten Reaktionsstufe ein herkömmlicher Rhodium-Triphenylphosphin-Katalysator und in der zweiten Reaktionsstufe ein mit einem chelatisierenden Diphosphin mit Xanthen-Rückgrat modifizierter Rhodiumkatalysator verwendet wird. Bei Einsatz eines Synthesegases mit einem $CO/H_2$-Verhältnis von 1:1 wird auch in dieser Schrift gemäß Beispielen in der zweiten Reaktionsstufe bei einem höheren Druck gearbeitet als in der ersten Stufe, entsprechend einer Erhöhung des CO- und $H_2$-Partialdrucks verglichen mit den Partialdrücken der ersten Stufe. Das Arbeiten mit unterschiedlichen Katalysatorsystemen ist allerdings aufwendig, wodurch dieses Verfahren erheblich verteuert wird.

Der vorliegenden Erfindung lag die Aufgabe zugrunde, ein Verfahren zur Hydroformylierung von Gemischen aus internen und $\alpha$-Olefinen zu finden, das es trotz der vom Stand der Technik geschilderten gegenläufigen Tendenzen der Reaktionsgeschwindigkeit und der n-Selektivität bezüglich deren Abhängigkeit vom CO-Partialdruck ermöglicht, Aldehyde mit hoher n-Selektivität bei hoher Raum-Zeit-Ausbeute aus solchen Olefingemischen wirtschaftlich herzustellen. Dazu sollte die Isomerisierung von internen zu terminalen Doppelbindungen bei den im Olefingemisch vorhandenen internen Olefinen gefördert und gleichzeitig die Doppelbindungsisomerisierung von $\alpha$-Olefinen zu internen Olefinen zurückgedrängt werden, da letztere die Gesamt-Raum-Zeit-Ausbeute des Verfahrens senkt, weil interne Olefine grundsätzlich weniger reaktiv sind als $\alpha$-Olefine.

Dementsprechend wurde ein Verfahren zur kontinuierlichen Herstellung von Aldehyden mit 5 bis 21 Kohlenstoffatomen durch die isomerisierende Hydroformylierung in homogener Phase von $\alpha$-Olefine und Olefine mit internen Doppelbindungen enthaltenden Olefinzusammensetzungen mit 4 bis 20 Kohlenstoffatomen mittels Synthesegas in Gegenwart eines mit einem Sauerstoff- und/oder Stickstoffatome enthaltenden Organophosphorliganden komplexierten, homogenen Rhodium-Katalysators und freiem Liganden, bei erhöhter Temperatur und bei erhöhtem Druck in einem mehrstufigen Reaktionssystem aus mindestens zwei Reaktionszonen gefunden, das dadurch gekennzeichnet ist, dass man die Olefinzusammensetzung zunächst in einer Gruppe aus einer oder mehreren ersten Reaktionszonen bei einem Gesamtdruck von 10 bis 40 bar mit Synthesegas eines $CO/H_2$-Molverhältnis von 4:1 bis 1:2 bis zu einem Umsatz der $\alpha$-Olefine von 40 bis 95 % umsetzt und den Hydroformylierungsaustrag aus dieser Gruppe aus einer oder mehreren ersten Reaktionszonen in einer Gruppe aus einer oder mehreren nachfolgenden Reaktionszonen bei einem Gesamtdruck von 5 bis 30 bar mit Synthesegas eines $CO/H_2$-Molverhältnis von 1:4 bis 1:1000 umsetzt, wobei der Gesamtdruck in der einen oder mehreren nachfolgenden Reaktionszonen jeweils um mindestens 1 bis (G1-Gf) bar niedriger ist als in der jeweils vorausgehenden Reaktionszone, worin G1 für den Gesamtdruck in der vorausgehenden Reaktionszone und Gf für den Gesamtdruck in der der einen oder mehreren ersten Reaktionszonen jeweils nachfolgenden Reaktionszone steht, mit der Maßgabe, dass die Differenz G1-Gf grö-βer als 1 bar ist, und wobei der CO-Partialdruck in der einen oder mehreren nachfolgenden Reaktionszonen jeweils niedriger ist als in der jeweils vorausgehenden Reaktionszone.

Das erfindungsgemäße Verfahren beruht auf einer Reihe von Forschungsergebnissen zur isomerisierenden Hydroformylierung. So wurde u.a. gefunden, dass

a) bei der kontinuierlichen Hydroformylierung von Raffinat II in zwei in Reihe geschalteten Reaktoren unter ansonsten gleichen Bedingungen in beiden Reaktoren durch eine Absenkung des CO-Partialdruckes, entweder durch eine Absenkung des Synthesegasdruckes bei konstantem $CO/H_2$-Molverhältnis bewirkt oder durch eine Verringerung des $CO/H_2$-Molverhältnisses bei konstantem Gesamtdruck, eine Steigerung der Aldehydausbeute und des Anteils an n-Valeraldehyd im Hydroformylierungsaustrag erzielt wird;

b) bei der diskontinuierlichen Hydroformylierung von 2- Buten die Absenkung des CO-Partialdruckes eine Steigerung der Aldehydausbeute und des Anteils an n-Valeraldehyd zur Folge hat; und

c) bei der diskontinierlichen Hydroformylierung von 1-Buten die Absenkung des CO-Partialdruckes zu einer Zunahme der unerwünschten Isomerisierung von 1-Buten zu 2-Buten führt und demzufolge die Raum-Zeit-Ausbeute der

Aldehydbildung als auch das zu n/i-Verhältnis der gebildeten Valeraldehyde abnimmt.

Durch Anwendung der erfindungsgemäßen Maßnahmen und die Einstellung der erfindungsgemäßen Verfahrensparameter wird erfindungsgemäß der in der Olefinzusammensetzung vorliegende α-Olefinanteil in einer Gruppe aus einer oder mehreren ersten Reaktionszonen größtenteils, z.B. bis zu einem Umsatz von 40 bis 95 %, vorzugsweise von 70 bis 95 %, zum entsprechenden n-Aldehyd umgesetzt, ohne dass es in größerem Umfang zu einer Isomerisierung der terminalen Doppelbindung zu innenständigen Doppelbindungen kommt, so dass - stark vereinfachend dargestellt - der Olefinanteil der Olefinzusammensetzung mit internen Doppelbindungen in einer Gruppe aus einer oder mehreren nachfolgenden Reaktionszonen unter für die Isomerisierung von internen Doppelbindungen zu endständigen Doppelbindungen optimalen Hydroformylierungsbedingungen hydroformyliert wird, ohne dass dies in relevantem Umfang zu einer Isomerisierung des ursprünglich in der Olefinzusammensetzung enthaltenen α-Olefinanteils führt, mit der Folge dass schließlich aus der Olefinzusammensetzung ein Aldehydprodukt mit einem hohen n/i-Verhältnis bei einer hohen Raum-Zeit-Ausbeute erhalten wird. Der Umfang der unerwünschten Isomerisierung der α-Olefine zu internen Olefinen sollte in der Gruppe aus einer oder mehreren ersten Reaktionszonen unter den Hydroformylierungsbedingungen so gering wie möglich gehalten werden und max. 80 %, bevorzugt weniger als 50 % und besonders bevorzugt weniger als 30 % betragen.

Der angestrebte Umsatz von im Allgemeinem 50 bis 99 % des in der Olefinzusammensetzung enthaltenen α-Olefinanteils kann also in einer einzigen ersten Reaktionszone als auch in mehreren ersten Reaktionszonen bewerkstelligt werden, wobei zum Zwecke der Erläuterung der Erfindung von dieser einzigen ersten Reaktionszone oder mehreren ersten Reaktionszonen als von einer "Gruppe aus einer oder mehreren ersten Reaktionszonen" gesprochen wird. Entsprechendes gilt für die Bezeichnung der der Gruppe aus einer oder mehreren ersten Reaktionszonen nachfolgenden einzigen oder mehreren Reaktionszonen, in der das hauptsächliche Ziel die isomerisierende Hydroformylierung des Anteils an internen Olefinen in der zu hydroformylierenden Olefinzusammensetzung ist.

Das erfindungsgemäße Verfahren unterscheidet sich somit vom Stand der Technik zur stufenweisen Hydroformylierung von α-Olefine und interne Olefine enthaltenden Olefingemischen mittels bezüglich Druck und $CO/H_2$-Verhältnis kaskadierten, also abgestuften Reaktionszonen (z.B. US-A 4 716 250; EP-A 1 008 580), indem der CO-Partialdruck als auch der Gesamtdruck in der Gruppe aus einer oder mehreren nachfolgenden Reaktionszonen niedriger ist als in der Gruppe aus einer oder mehreren ersten Reaktionszonen, wobei im Allgemeinen in der Gruppe der einen oder mehreren ersten Reaktionszonen mit einem relativ hohen $CO/H_2$-Molverhältnis und bei einem deutlich höheren Gesamtdruck als in der Gruppe der einen oder mehreren nachfolgenden Reaktionszonen gearbeitet wird.

Das erfindungsgemäße Verfahren betrifft die Durchführung der Hydroformylierung von terminale und interne Doppelbindungen enthaltenden Olefinzusammensetzungen in einem bezüglich der Verfahrensparameter Gesamtdruck, $CO/H_2$-Molverhältnis und CO-Partialdruck kaskadierten mehrstufigen Reaktionssystem aus mehreren, z.B. 2 bis 10, vorzugsweise 2 bis 4, und besonders bevorzugt zwei (2) in Reihe geschalteten Reaktionszonen, wobei sich die einzelnen Reaktionszonen voneinander durch den darin vorherrschenden unterschiedlichen Gesamtdruck, den CO-Partialdruck und gegebenenfalls das $CO/H_2$-Molverhältnis unterscheiden. Daraus ergibt sich, dass die einzelnen Reaktionszonen in den einzelnen, in Reihe geschalteten Reaktoren einer Reaktorkaskade gelegen sein können, es kann aber auch sein, dass eine einzelne Reaktionszone mehrere in Reihe oder parallel geschaltete Reaktoren umfasst, die die erfindungsgemäßen Kriterien für eine individuelle Reaktionszone, im wesentlichen gleicher Gesamtdruck und CO-Partialdruck, erfüllen. Umgekehrt kann es sein, dass ein einzelner Hydroformylierungsreaktor durch geeignete Einbauten in mehrere Reaktionskompartimente segmentiert ist, wobei in den einzelnen Reaktionskompartimenten die Reaktionsbedingungen unabhängig voneinander so eingestellt werden können, dass ein oder mehrere dieser Reaktionskompartimente eine Reaktionszone bilden und eines oder mehrere der nachfolgenden Reaktionskompartinente des Reaktors je nach Einstellung der Verfahrensparameter in den einzelnen Kompartimenten eine zweite bzw. mehrere nachfolgende Reaktionszonen bilden. Herrscht also in einem nachfolgenden Kompartiment des Reaktors ein um mindestens 1 bar geringerer Gesamtdruck und ein niedrigerer CO-Partialdruck bei gegebenenfalls geringerem $CO/H_2$-Molverhältnis als im vorausgehenden Kompartiment, so bildet dieses nachfolgende Kompartiment bezüglich des vorausgehenden eine neue Reaktionszone. Analoges gilt bezüglich der Reaktionsbedingungen in einzelnen, in Reihe geschalteten Einzelreaktoren.

Als Reaktoren können im erfindungsgemäßen Verfahren prinzipiell sämtliche für Hydroformylierungsreaktionen geeigneten Reaktortypen verwendet werden, beispielsweise Rührreaktoren, Blasensäulenreaktoren wie sie z.B. in US-A 4 778 929 beschrieben sind, Umlaufreaktoren wie sie z.B. Gegenstand von EP-A 1 114 017 sind, Rohrreaktoren, wobei die einzelnen Reaktoren einer Reihe unterschiedliche Mischungscharakteristiken haben können, wie z.B. in EP-A 423 769 beschrieben, des weiteren können kompartimentierte Reaktoren verwendet werden, wie sie z.B. Gegenstand von EP-A 1 231 198 oder von US-A 5 728 893 sind.

Umfasst eine Reaktionszone mehrere Reaktoren, können in dieser Reaktionszone gleiche oder verschiedene Reaktortypen eingesetzt werden, desgleichen können von Reaktionszone zu Reaktionszone gleiche oder verschiedene Reaktortypen verwendet werden. Vorzugsweise werden in den einzelnen Reaktionszonen gleiche Reaktortypen verwendet, z.B. Umlaufreaktoren oder Rührkessel.

Das erfindungsgemäße Verfahren wird im Allgemeinen in einer Gruppe aus einer oder mehreren ersten Reaktionszonen bei einem Gesamtdruck von im Allgemeinen 10 bis 40 bar, bevorzugt 10 bis 30 bar und besonders bevorzugt 10 bis 25 bar und in einer Gruppe aus einer oder mehreren der Gruppe aus einer oder mehreren ersten Reaktionszonen nachfolgenden Reaktionszonen bei einem Gesamtdruck von im Allgemeinen 5 bis 30 bar, vorzugsweise 5 bis 20 bar, und besonders bevorzugt 9 bis 20 bar durchgeführt. Der Gesamtdruck in den einzelnen Reaktionszonen setzt sich dabei additiv zusammen aus den Partialdrücken der Reaktionsgase Kohlenmonoxid (CO) und Wasserstoff ($H_2$), der Partialdrücke der einzelnen Olefine der zu hydroformylierenden Olefinzusammensetzung sowie der im Verlaufe der Hydroformylierung erzeugten Aldehyde und den Partialdrücken von weiteren in der Reaktionsmischung enthaltenen Komponenten, wie gesättigten Kohlenwasserstoffen, Inertgasen, wie Stickstoff, Hilfsstoffen, wie Lösungsmitteln oder freiem Liganden, wie gegebenenfalls der Reaktionsmischung zugesetzten Stabilisatoren, z.B. tertiären Aminen oder Azinen, die der Stabilisierung der eingesetzten Liganden gegenüber Abbaureaktionen dienen, sowie gegebenenfalls in den Ausgangsmaterialien in Spuren enthaltenen Verunreinigungen, z.B. Kohlendioxid und im Verlaufe der Hydroformylierung gebildeter Nebenprodukte. Im Allgemeinen wird der Gesamtdruck in den einzelnen Reaktionszonen über die Zufuhr der Reaktionsgase CO und $H_2$ und/oder über das Ausgasen leichtflüchtiger Komponenten durch Entspannen der Reaktionsmischung bei der betreffenden Reaktionstemperatur eingestellt.

Der Gesamtdruck in den einzelnen Reaktionszonen wird erfindungsgemäß so eingestellt, dass der Gesamtdruck in der nachfolgenden Reaktionszone bezüglich der ihr vorausgegangenen Reaktionszone um mindestens 1 bis (G1 - Gf) bar niedriger ist, worin G1 für den Gesamtdruck in der vorausgegangenen Reaktionszone $R_m$, der wiederum eine oder mehrere Reaktionszonen, z.B. $R_l$, $R_k$ usw. vorangegangen sein können, und Gf den Gesamtdruck in der dieser Reaktionszone nachfolgenden Reaktionzone $R_n$ steht, der wiederum weitere Reaktionszonen ($R_o$, $R_p$, usw.) folgen können, bezüglich deren Gesamtdruck die gleiche Beziehung gilt, wie zwischen den Reaktionszonen $R_m$ und $R_n$. Dabei gilt die Maßgabe, dass die Differenz G1-Gf größer als 1 bar ist. Im Allgemeinen ist der Gesamtdruck in der nachfolgenden Reaktionszone $R_n$ um 1 bis 25 bar, bevorzugt um 1 bis 15 bar und besonders bevorzugt 1 bis 10 bar niedriger als in der vorausgehenden Reaktionszone. Bezugspunkt für den Gesamtdruck in den einzelnen Reaktionszonen ist dabei jeweils der Gesamtdruck im Reaktor - oder falls mehrere Reaktoren gemeinsam eine Reaktionszone bilden, der Gesamtdruck im letzten Reaktor der vorausgehenden Reaktionszone und im Reaktor - oder falls mehrere Reaktoren gemeinsam eine Reaktionszone bilden, der Gesamtdruck im ersten Reaktor der nachfolgenden Reaktionszone. Entsprechendes gilt bei kompartimentierten Reaktoren, bei denen ein oder mehrere einzelne Reaktionskompartimente eine Reaktionszone bilden. Bei Rohrreaktoren bildet der Gesamtdruck am Ausgang eines ersten Rohrreaktors und der Gesamtdruck am Eingang eines nachfolgenden Rohrreaktors oder anderen Reaktors den Bezugspunkt für die Festlegung der einzelnen Reaktionszonen. Ist die Gesamtdruckdifferenz zwischen zwei oder mehreren in Reihe geschalteten Reaktoren oder Reaktionskompartimenten geringer als 1 bar, so bilden diese gemeinsam eine Reaktionszone.

Die Reaktionstemperatur in den einzelnen Reaktionszonen beträgt im Allgemeinen 50 bis 200°C, vorzugsweise 50 bis 150°C und besonders bevorzugt 70 bis 130°C.

Das $CO/H_2$-Molverhältnis des Synthesegases in der Gruppe aus einer oder mehreren ersten Reaktionszonen des erfindungsgemäßen Verfahren beträgt im Allgemeinen 4:1 bis 1:2, vorzugsweise 4:1 bis 2:3 und besonders bevorzugt 3:2 bis 2:3 ($CO:H_2$) und in der Gruppe aus einer oder mehreren der Gruppe aus einer oder mehreren ersten Reaktionszonen nachfolgenden Reaktionszonen im Allgemeinen 1:4 bis 1:1000, vorzugsweise 1:4 bis 1:100 und besonders bevorzugt 1:9 bis 1:100 ($CO/H_2$), wobei der CO-Partialdruck in einer nachfolgenden Reaktionszone niedriger eingestellt wird als der in der dieser Reaktionszone unmittelbar vorausgegangenen Reaktionszone herrschende CO-Partialdruck. Im Allgemeinen wird der CO-Partialdruck in der einer oder mehreren ersten Reaktionszonen nachfolgenden Reaktionszone um 1 bis 20 bar, vorzugsweise um 1 bis 10 bar und besonders bevorzugt um 2 bis 7 bar niedriger eingestellt als der in der unmittelbar vorausgegangenen Reaktionszone herrschende CO-Partialdruck.

Bezüglich der Bezugspunkte für die Feststellung des in den einzelnen Reaktionszonen vorliegenden CO-Partialdrucks gelten die vorstehenden Ausführungen zur Feststellung des Gesamtdrucks in den einzelnen Reaktionszonen entsprechend.

Der optimale Gesamtdruck, das optimale $CO/H_2$-Molverhältnis als auch der optimale CO-Partialdruck in den einzelnen Reaktionszonen des erfindungsgemäßen Verfahrens ist von der Art und Zusammensetzung der zu hydroformylierenden Olefinzusammensetzung abhängig, beispielsweise der Kettenlänge der zu hydroformylierenden Olefine, des Anteils an terminalen und internen Doppelbindungen, der Lage der internen Doppelbindungen und gegebenenfalls dem Verzweigungsgrad der Olefine. Dementsprechend werden diese Verfahrensparameter für eine bestimmte Olefinzusammensetzung und den zum Einsatz gelangenden Hydroformylierungskatalysator zweckmäßigerweise in routinemäßigen Vorversuchen oder rechnerischen Verfahrenssimulationen erfindungsgemäß optimiert, um die wirtschaftlich im Hinblick auf die Erzielung eines möglichst hohen n/i-Anteils im Aldehydprodukt bei einer möglichst hohen Raum-Zeit-Ausbeute optimalen Bedingungen zu eruieren.

Entsprechendes gilt für die Anzahl der Reaktionszonen im erfindungsgemäßen Verfahren. So kann es sinnvoll sein zur Erzielung einer wirtschaftlich optimalen Beziehung von n/i-Verhältnis und Raum-Zeit-Ausbeute erfindungsgemäß bis zu 10 in Reihe geschaltete Reaktionszonen einzurichten. Aufgrund des dadurch bedingten höheren Investitionsaufwands und/oder mess- und regeltechnischen Aufwands und möglicherweise höherer Betriebskosten kann dadurch der durch die Verbesserung des n/i-Verhältnisses und der Raum-Zeit-Ausbeute gewonnene wirtschaftliche Vorteil neutralisiert werden. Zweckmäßigerweise wird das erfindungsgemäße Verfahren daher im allgemeinen in 2 bis 8, vorzugsweise in 2 bis 4 und besonders bevorzugt in 2 in Reihe geschalteten Reaktionszonen durchgeführt, wobei die wirtschaftliche optimale Anzahl der Reaktionszonen zweckmäßigerweise abhängig von der Art und Zusammensetzung der zu hydroformylierenden Olefinzusammensetzung und dem eingesetzten Hydroformylierungskatalysator im Einzelfall durch Routineversuche oder rechnerische Verfahrenssimulationen ermittelt wird.

Der Hydroformylierungsaustrag aus einer Reaktionszone kann vor Eintritt in die nachfolgende Reaktionszone entspannt, die gebildeten Aldehyde abgetrennt und die nicht umgesetzten Olefine zur Hydroformylierung der nachfolgenden Reaktionszone zugeführt werden. Vorzugsweise wird erfindungsgemäß keine derartige Aufarbeitung des Hydroformylierungsaustrags aus einer Reaktionszone vor dessen Eintritt in die nächste Reaktionszone vorgenommen und der Hydroformylierungsaustrag aus einer Reaktionszone direkt in die nachfolgende Reaktionszone entspannt. Diese vorteilhafte Ausgestaltung des erfindungsgemäßen Verfahrens wird dadurch möglich, dass der Gesamtdruck in der nachfolgenden Reaktionszone niedriger ist als in der vorausgehenden Reaktionszone.

Die Einstellung des Gesamtdrucks in den einzelnen Reaktionszonen erfolgt im Allgemeinen unter Berücksichtigung der Menge des frisch zugeführten Synthesegases über die Regelung des Abgasstroms aus den einzelnen Reaktionszonen, der sich im Allgemeinen im Wesentlichen aus nicht umgesetztem Synthesegas, Inertgasen und Kohlenwasserstoffen zusammensetzt. Zur Einstellung des in den einzelnen Reaktionszonen gewünschten $CO/H_2$-Molverhältnissen kann, falls das $CO/H_2$-Gemisch nicht mit dem gewünschten, vorgeformten $CO/H_2$-Molverhältnis den einzelnen Reaktionszonen zugeführt wird, vorteilhaft Synthesegas eines herkömmlichen $CO/H_2$-Molverhältnisses von ca. 1:1 verwendet und das gewünschte $CO/H_2$-Molverhältnis in den einzelnen Reaktionszonen durch zudosieren von zusätzlichen Mengen an CO oder $H_2$ eingestellt werden. Hierbei besteht die wirtschaftlich vorteilhafte Möglichkeit das erfindungsgemäße mit anderen Verfahren zu integrieren, indem man im wesentlichen CO- oder $H_2$-enthaltende Abgase aus anderen Verfahren zur Einstellung des $CO/H_2$-Mol-verhältnisses verwendet. So kann z.B. das CO-haltige Abgas aus der Methylformiatherstellung zur Erhöhung des $CO/H_2$-Molverhältnisses bzw. das $H_2$-haltige Abgas aus der Hydrierung von Aldehyden oder daraus erzeugter Enale, z.B. 2-Ethylhexenal oder 2-Propylheptenal, zu den entsprechenden gesättigten Alkoholen als $H_2$-Quelle zur Erniedrigung des $CO/H_2$-Molverhältnisses in einzelnen Reaktionszonen eingesetzt werden. Der CO-Partialdruck kann auch durch eine Erniedrigung des $CO/H_2$-Drucks in einer nachfolgenden Reaktionszone verringert werden.

Das erfindungsgemäße Verfahren eignet sich zur Hydroformylierung von Olefinzusammensetzungen aus vorzugsweise aliphatischen $C_4$- bis $C_{20}$-Olefinen, die interne und terminale Doppelbindungen enthalten und geradkettig oder verzweigt sein können. Beispiele für solche Olefinzusammensetzungen sind 1-Buten/2-Butengemische wie sie z.B. großtechnisch als Raffinat II anfallen. Als Raffinat II wird die nach Abtrennung von Butadienen und Acetylenen und nachfolgenden Abtrennung von Isobuten aus dem $C_4$-Schnitt von Steamcrackern erhaltene $C_4$-Olefinfraktion bezeichnet, die im Allgemeinen folgende Zusammensetzung hat:

0,5 bis 5 Gew.-% Isobutan
5 bis 20 Gew.-% n-Butan
20 bis 40 Gew.-% trans-2-Buten
10 bis 20 Gew.-% cis-2-Buten
25 bis 55 Gew.-% 1-Buten
0,5 bis 5 Gew.-% Isobuten

sowie Spurengase, wie 1,3-Butadien, Propadien, Propen, Cyclopropan, Methylcyclopropan und Vinylacetylen.

Andere geeignete Olefinzusammensetzungen sind z.B. Pentengemische, wie sie z.B. bei der säurekatalysierten Codimerisierung von Ethen und Propen gebildet werden, Hexengemische aus der Säure- oder Nickel-katalysierten (Dimersol-Verfahren) Propendimerisierung oder aus Metatheseprozessen, wie sie z.B. in EP-A 1 069 101 und EP-A 1 134 271 beschrieben sind, Heptengemische, z.B. aus der Säure- oder Nickel-katalysierten Codimerisierung von Propen und Buten, Octengemische aus der Dimerisierung von Butenen, beispielsweise gemäß US-A 5 849 972, Nonengemische z.B. aus der säurekatalysierten Propentrimerisierung, Decengemische, z.B. aus der säurekatalysierten Dimerisierung von Pentenen, Undecengemische z.B. aus der Codimerisierung von Pentenen und Hexenen, Dodecengemische, z.B. aus der säurekatalysierten Tetramerisierung von Propen, der Trimerisierung von Butengemischen, z.B. gemäß US-A 5 849 972 und/oder der Dimerisierung von Hexengemischen z.B. gemäß WO 00/53546, Hexadecengemische, z.B. aus der Tetramerisierung von Butengemischen, z.B. gemäß US-A 5 849 972, Octadecengemischen z.B. aus der Dimerisierung von Nonengemischen und/oder Eicosengemische aus der Dimerisierung von Decengemischen oder der Tetramerisierung von Pentengemischen. Es bedarf keiner weiteren Erläuterung, dass selbstverständlich auch Zusammensetzungen aus Olefinen unterschiedlicher Kohlenstoffanzahl im erfindungsgemäßen Verfahren eingesetzt werden können. Solche Olefinzusammensetzungen mit unterschiedlicher Kohlenstoffanzahl können z.B. durch Metathese aus Butenen,

wie in EP-A 1 134 271 und EP-A 1 069 101 beschrieben, oder über den SHOP-Prozess oder das Fischer-Tropsch-Verfahren erhalten werden. Eine Zusammenstellung verschiedener geeigneter Verfahren zur Herstellung von Zusammensetzungen aus höheren Olefinen findet sich z.B. in Weissermel, Arpe: Industrielle Organische Chemie, Seiten 82-99, 5. Auflage, Wiley-VCH, Weinheim 1998. Die erfindungsgemäß aus diesen Olefinzusammensetzungen erhältlichen Aldehyde dienen z.B. als Ausgangsmaterial zur Herstellung von Weichmacher- und Tensidalkoholen, die daraus entweder direkt z.B. durch Hydrierung des Aldehyds zum Alkohol oder über die Stufen der Aldolisierung und Hydrierung gewonnen werden können, wie z.B. 2-Propylheptanol, das durch die Aldolisierung von Vateraldehyd (erhalten durch die Hydroformylierung von Raffinat II) und anschließende Hydrierung des Aldolisierungsprodukts 2-Propylheptenal erzeugt werden können.

Lediglich beispielhaft zur Veranschaulichung und keinesfalls abschließend seien im Folgenden repräsentativ einzelne olefinische Verbindungen, wie sie in solchen Olefinzusammensetzungen vorkommen können, aufgezählt:

Butene, wie 1-Buten, 2-Buten und -isobuten, Penten, wie 1-Penten, 2-Penten, 2-Methyl-1-buten und 2-Methyl-2-buten, Hexene, wie 1-Hexen, 2-Hexen, 3-Hexen, 2-Methyl-1-penten, 2-Methy-2-penten, 3-Methyl-2-penten und 3-Methyl-1-penten, Heptene, wie 1-Hepten, 2-Hepten, 3-Hepten und die verschiedenen Isomere von Methyl-Hexen, Octene, wie 1-Octen, 2-Octen, 3-Octen, 4-Octen sowie verzweigte Octene mit innenständiger bzw. endständiger Doppelbindung, Nonene, wie 1-Nonen, 2-Nonen, 3-Nonen, 4-Nonen sowie verzweigte Nonene mit innenständiger bzw. endständiger Doppelbindung, Decene, wie 1-Decen, 2-Decen, 3-Decen, 4-Decen, 5-Decen sowie verzweigte Decene mit innenständiger bzw. endständiger Doppelbindung, Undecene, wie 1-Undecen, 2-Undecen, 3-Undecen, 4-Undecen, 5-Undecen sowie verzweigte Undecene mit innenständiger bzw. terminaler Doppelbindung, Dodecene, wie 1-Dodecen, 2-Dodecen, 3- Dodecen, 4-Dodecen, 5-Dodecen, 6-Dodecen sowie verzweigte Dodecene mit innenständiger bzw. endständiger Doppelbindung. Desgleichen Tetradecene, Pentadecene, Hexadecene, Heptadecene, Octadecene, Nonadecene und Eicosene.

Die im erfindungsgemäßen Verfahren einsetzbaren Olefinzusammensetzungen können auch Diolefine enthalten, beispielsweise $\alpha$-w-Diolefine, wie 1,3-Butadien, 1,4-Pentadien, 1,5-Hexadien, 1,6-Heptadien, 1,7-Octadien, 1,8-Nonadien oder 1,9-Decadien, als auch Diolefine mit sowohl innenständigen und endständigen Doppelbindungen, wie 1,3-Pentadien, 1,3-Hexadien, 1,4-Hexadien, 1,3-Heptadien, 1,4-Heptadien, 1,5-Heptadien, 1,3-Octadien, 1,4-Octadien, 1,5-Octadien, 1,6-Octadien, 1,3-Nonadien, 1,4-Nonadien, 1,5-Nonadien, 1,6-Nonadien, 1,7-Nonadien, 1,3-Decadien, 1,4-Decadien, 1,5-Decadien, 1,6-Decadien, 1,7-Decadien, 1,8-Decadien.

Das erfindungsgemäße Hydroformylierungsverfahren wird mit homogen im Reaktionsmedium gelösten Rhodiumkatalysatoren durchgeführt, die mit einem phosphorhaltigen Liganden komplexiert sind, der die Fähigkeit hat unter den erfindungsgemäßen Reaktionsbedingungen Olefine mit internen Doppelbindungen isomerisierend zu hydroformylieren und nur eine relativ geringe Neigung zur Isomerisierung von terminalen zu internen Doppelbindungen unter den erfindungsgemäßen Reaktionsbedingungen hat. Zweckmäßigerweise wird der Ligand bezüglich des Rhodiums im Überschuss eingesetzt und zwar im Allgemeinen mit einem Ligand/Rh-Molverhältnis von 2 bis 300, vorzugsweise von 2 bis 20 und besonders bevorzugt von 2 bis 10.

Die Rhodiumkonzentration im flüssigen Reaktionsgemisch beträgt im Allgemeinen 10 bis 500 Gew.-ppm, vorzugsweise 30 bis 350 Gew.-ppm und besonders bevorzugt 50 bis 300 Gew.-ppm. Als Rhodiumquelle können z.B. Rhodiumsalze, wie Rhodiumacetat, Rhodiumchlorid oder Rhodiumnitrat, Rhodiumkomplexe, wie Rhodiumacetylacetonat und/oder Rhodiumcarbonylverbindungen, wie $Rh(CO)_2acac$, $Rh_4(CO)_{12}$ oder $Rh_6(CO)_{16}$ verwendet werden (acac: Acetylacetonat). Die Art der verwendeten Rhodiumquelle ist im Allgemeinen für das Ergebnis des erfindungsgemäßen Verfahrens nicht kritisch.

Zur Hydroformylierung wird die als Rhodiumquelle dienende Rhodiumverbindung im Allgemeinen im Reaktionsgemisch gelöst oder suspendiert, desgleichen der einzusetzende Ligand. Der hydroformylierungsaktive Rhodiumkomplexkatalysator bildet sich dann in situ im Hydroformylierungsreaktor, unter den Bedingungen der Hydroformylierungsreaktion, d.h. unter der Einwirkung von Kohlenmonoxid und Wasserstoff auf die mit dem Phosptior-haltigen Liganden komplexierte Rhodiumverbindung. Es versteht sich von selbst, dass auch präformierte Rhodium-Katalysatorkomplexe der Reaktionsmischung zugesetzt werden können.

Das erfindungsgemäße Verfahren wird in homogener Phase durchgeführt, d.h. das zu hydroformylierende Olefin, der homogen in der Reaktionsmischung gelöste Rhodiumhydroformylierungskatalysator, freier Ligand als auch der gebildete Aldehyd befinden sich in einer flüssigen Phase, d.h. das erfindungsgemäße Verfahren betrifft nicht die Hydroformylierung in zwei nebeneinander vorliegenden flüssigen Phasen.

Das erfindungsgemäße Hydroformylierungsverfahren kann vorteilhaft in Gegenwart von Lösungsmitteln ausgeführt werden. Als Lösungsmittel werden vorzugsweise diejenigen Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, die auch zum Verdünnen der oben genannten Aldehyde und der Folgeprodukte der Aldehyde

dienen können. Weitere Lösungsmittel sind Ester aliphatischer Carbonsäuren mit Alkanolen, beispielsweise Essigester oder Texanol®, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch sogenannte "Ionische Flüssigkeiten" verwendet werden. Hierbei handelt es sich um flüssige Salze, beispielsweise um N,N'-Di-alkylimidazoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z. B. die Tetrafluoroborate, Acetate, Tetrachloroaluminate, Hexafluorophosphate, Chloride und Tosylate.

Das erfindungsgemäße Verfahren wird zweckmäßigerweise nach dem Flüssigaustragsverfahren durchgeführt. Dabei wird das flüssige Hydroformylierungsgemisch kontinuierlich aus dem Hydroformylierungsreaktor einer ersten Reaktionszone abgezogen und dem Hydroformylierungsreaktor der nachfolgenden Reaktionszone zugeführt. Da die nachfolgende Reaktionszone bei einem geringeren Gesamtdruck betrieben wird als vorausgehende, kann der Hydroformylierungsaustrag aus der vorausgehenden Reaktionszone zweckmäßigerweise in den, Hydroformylierungsreaktor der nachfolgenden Reaktionszone entspannt werden. Im Allgemeinen findet keine Aufarbeitung des Hydroformylierungsaustrags aus einer ersten Reaktionszone vor dessen Eintritt in die nachfolgende Reaktionszone statt. Die nachfolgende Reaktionszone wird unter den gewählten, von den Reaktionsbedingungen der vorausgehenden Reaktionszone verschiedenen Reaktionsbedingungen betrieben. Aus dieser nachfolgenden Reaktionszone wird im Allgemeinen das flüssige Hydroformylierungsgemisch kontinuierlich dem Hydroformylierungsreaktor entnommen und entweder zur Abtrennung darin gelöster Gase, wie nicht umgesetztes $CO/H_2$-Gemisch, auf einen Druck, der im Allgemeinen um 1 bis 35 bar, vorzugsweise 3 bis 10 bar niedriger ist, als der im Hydroformylierungsreaktor vorherrschende, in ein Entspannungsgefäß entspannt oder gegebenenfalls einer weiteren nachfolgenden Reaktionszone, wie beschrieben, zugeführt. Die im Entspannungsgefäß freigesetzten Gase, insbesondere nicht-umgesetztes $CO/H_2$-Gemisch, können gewünschtenfalls wieder in eine der vorausgegangenen Reaktionszonen zur weiteren Umsetzung zurückgeführt werden, wobei es zweckmäßig sein kann, diesen gasförmigen Rückführstrom einer Wäsche oder einer Zwischenkondensation in einem Wärmetauscher zur Entfernung mitgerissener Aldehyde und/oder Olefine zu unterziehen oder einen Teilstrom dieses Rückführstroms zur Vermeidung der Anreicherung von Inertgasen im Reaktor auszuschleusen. Der im Entspannungsgefäß verbleibende flüssige, das Hydroformylierungsprodukt, darin gelöste Olefine, Hochsieder, den Katalysator und freien Liganden enthaltende Hydroformylierungsaustrag kann anschließend einer Entgasungskolonne zur Rückgewinnung nicht umgesetzter Olefine zugeführt werden. Aus dem das Hydroformylierungsprodukt, Hochsieder, Katalysator und freien Liganden enthaltenden Sumpfprodukt der Entgasungskolonne kann anschließend in einer Destillationskolonne das Hydroformylierungsprodukt von den Hochsiedern, dem Katalysator und freiem Liganden abgetrennt und weiterverwendet werden, wohingegen die Hochsieder, der Katalysator und freier Ligand, gegebenenfalls nach Konzentrierung und Ausschleusung eines Teilstroms zwecks Vermeidung des Aufpegelns von Hochsiedern im Reaktor zweckmäßigerweise wieder in den Reaktor einer der vorausgegangenen Reaktionszonen, vorzugsweise der ersten Reaktionszone, zurückgeleitet wird. Zur Aufarbeitung des Aldehydproduktes kann nach verschiedenen Verfahren des Standes der Technik verfahren werden, beispielsweise nach den Verfahren gemäß US-A 4,148,830, US-A 6,100,432 oder WO 01/58844.

Als doppelbindungsisomerisierungsaktive Katalysatoren werden erfindungsgemäß vorzugsweise Komplexe des Rhodiums mit chelatisierenden Organophosphoramidit- bzw. Organophosphit- oder Organophosphonit-Liganden verwendet. In diesen Liganden kann eines oder mehrere der Phosphoratome durch Arsen- und/oder Antimonatome ersetzt sein, bevorzugt werden allerdings phosphorhaltige Liganden verwendet.

Als isomerisierende Hydroformylierungskatalysatoren sind im erfindungsgemäßen Verfahren beispielsweise Rhodiumkomplexe mit Phosphoramiditliganden der allgemeinen Formel I geeignet,

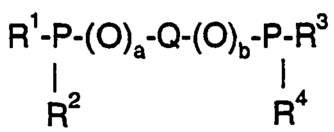

I

in der

Q eine Brückengruppe der Formel

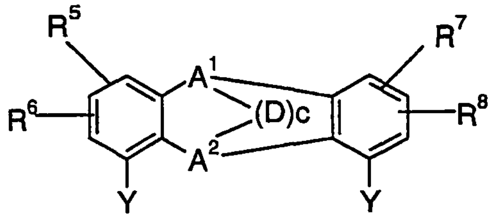

ist,
worin

$A^1$ und $A^2$ unabhängig voneinander für O, S, $SiR^aR^b$, $NR^c$ oder $CR^dR^e$ stehen, wobei

$R^a$, $R^b$ und $R^c$ unabhängig voneinander für Wasserstoff, Alkyl, Cycloalkyl, Heterocycloalkyl, Aryl oder Hetaryl stehen,

$R^d$ und $R^e$ unabhängig voneinander für Wasserstoff, Alkyl, Cycloalkyl, Heterocycloalkyl, Aryl oder Hetaryl stehen oder gemeinsam mit dem Kohlenstoffatom, an das sie gebunden sind, eine Cycloalkylidengruppe mit 4 bis 12 Kohlenstoffatomen bilden oder die Gruppe $R^d$ gemeinsam mit einer weiteren Gruppe $R^d$ oder die Gruppe $R^e$ gemeinsam mit einer weiteren Gruppe $R^e$ eine intramolekulare Brückengruppe D bilden,

D eine zweibindige Brückengruppe, ausgewählt aus den Gruppen

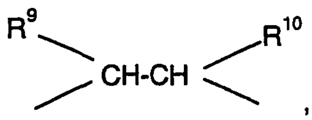 , 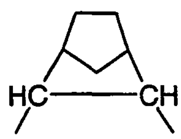 , 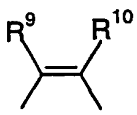 , 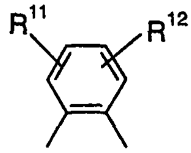 , 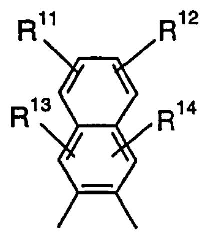

ist, in denen

$R^9$ und $R^{10}$ unabhängig voneinander für Wasserstoff, Alkyl, Cycloalkyl, Aryl, Halogen, Trifluormethyl, Carboxyl, Carboxylat oder Cyano stehen oder miteinander zu einer $C_3$- bis $C_4$-Alkylenbrücke verbunden sind,

$R^{11}$, $R^{12}$, $R^{13}$ und $R^{14}$ unabhängig voneinander für Wasserstoff, Alkyl, Cycloalkyl, Aryl, Halogen, Trifluormethyl, COOH, Carboxylat, Cyano, Alkoxy, $SO_3H$, Sulfonat, $NE^1E^2$, Alkylen-$NE^1E^2E^{3+}X^-$, Acyl oder Nitro stehen,

c 0 oder 1 ist,

Y eine chemische Bindung darstellt,

$R^5$, $R^6$, $R^7$ und $R^8$ unabhängig voneinander für Wasserstoff, Alkyl, Cycloalkyl, Heterocycloalkyl, Aryl, Hetaryl, $COOR^f$, $COO^-M^+$, $SO_3R^f$, $SO^-_3M^+$, $NE^1E^2$, $NE^1E^2F^{3+}X^-$, Alkylen-$NE^1E^2E^{3+}X^-$, $OR^f$, $SR^f$, $(CHR^9CH_2O)_xR^f$, $(CH_2N(E^1))_xR^f$, $(CH_2CH_2N(E^1))_xR^f$, Halogen, Trifluormethyl, Nitro, Acyl oder Cyano stehen,

worin

$R^f$, $E^1$, $E^2$ und $E^3$ jeweils gleiche oder verschiedene Reste, ausgewählt unter Wasserstoff, Alkyl, Cycloalkyl oder Aryl bedeuten,

$R^9$ für Wasserstoff, Methyl oder Ethyl steht,

$M^+$ für ein Kation steht,

$X^-$ für ein Anion steht, und

x für eine ganze Zahl von 1 bis 120 steht,

oder

$R^5$ und/oder $R^7$ zusammen mit zwei benachbarten Kohlenstoffatomen des Benzolkerns, an den sie gebunden sind, für ein kondensiertes Ringsystem, mit 1, 2 oder 3 weiteren Ringen stehen,

a und b unabhängig voneinander die Zahl 0 oder 1 bedeuten

P für Phosphor

und

$R^1$, $R^2$, $R^3$, $R^4$ unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine

$NE^1E^2$-Gruppe stehen, mit der Maßgabe, dass $R^1$ und $R^3$ über das Stickstoffatom an das Phosphor P gebundene Pyrrolgruppen sind oder worin $R^1$ gemeinsam mit $R^2$ und/oder $R^3$ gemeinsam mit $R^4$ eine mindestens eine über das pyrrolische Stickstoffatom an das Phosphoratom P gebundene Pyrrolgruppe enthaltende zweibindige Gruppe E der Formel

Py-I-W

bilden, worin

Py eine Pyrrolgruppe ist,

I für eine chemische Bindung oder für O, S, $SiR^aR^b$, $NR^c$ oder $CR^hR^i$ steht,

W für Cycloalkyl, Cycloalkoxy, Aryl, Aryloxy, Hetaryl oder Hetaryloxy steht,

und

$R^h$ und $R^i$ unabhängig voneinander für Wasserstoff, Alkyl, Cycloalkyl, Heterocycloalkyl, Aryl oder Hetaryl stehen,

oder eine über die Stickstoffatome an das Phosphoratom P gebundene Bispyrrolgruppe der Formel

Py-I-Py

bilden.

Bevorzugte Phosphoramidit-Liganden sind solche der Formel Ia

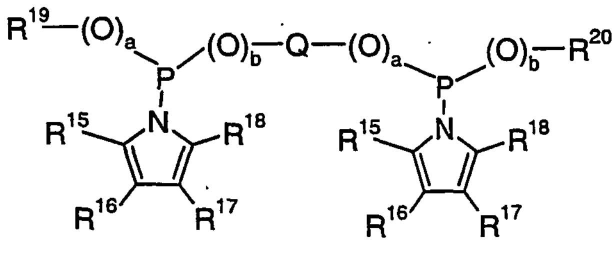

Ia

worin

$R^{15}$, $R^{16}$, $R^{17}$ und $R^{18}$ unabhängig voneinander für Wasserstoff, Alkyl, Cycloalkyl, Heterocycloalkyl, Aryl, Hetaryl, $W'COOR^k$, $W'COO^-M^+$, $W'(SO_3)R^k$, $W'(SO_3)^-M^+$, $W'PO_3(R^k)(R^l)$, $W'(PO_3)_2^-(M^+)_2$, $W'NE^4E^5$, $W'(NE^4E^5E^6)^+X^-$, $W'OR^k$, $W'SR^k$, $(CHR^lCH_2O)_yR^k$, $(CH_2NE^4)_yR^k$, $(CH_2CH_2NE^4)_yR^k$, Halogen, Trifluormethyl, Nitro, Acyl oder Cyano stehen,

worin

W' für eine Einfachbindung, ein Heteroatom oder eine zweiwertige verbrückende Gruppe mit 1 bis 20 Brückenatomen steht,

$R^k$, $E^4$, $E^5$, $E^6$ jeweils gleiche oder verschiedene Reste, ausgewählt unter Wasserstoff, Alkyl, Cycloalkyl oder Aryl bedeuten,

$R^l$ für Wasserstoff, Methyl oder Ethyl steht,

$M^+$ für ein Kationäquivalent steht,

$X^-$ für ein Anionäquivalent steht und

y für eine ganze Zahl von 1 bis 240 steht,

wobei jeweils zwei benachbarte Reste $R^{15}$, $R^{16}$, $R^{17}$ und $R^{18}$ zusammen mit den Kohlenstoffatomen des Pyrrolrings, an die sie gebunden sind, auch für ein kondensiertes Ringsystem mit 1, 2 oder 3 weiteren Ringen stehen können, mit der Maßgabe, dass wenigstens einer der Reste $R^{15}$, $R^{16}$, $R^{17}$ oder $R^{18}$ nicht für Wasserstoff steht, und dass $R^{19}$ und $R^{20}$ nicht mit einander verknüpft sind,

$R^{19}$ und $R^{20}$ unabhängig voneinander für Cycloalkyl, Heterocyloalkyl, Aryl oder Hetaryl stehen,

a und b unabhängig voneinander die Zahl 0 oder 1 bedeuten

P für ein Phosphoratom steht,

Q eine Brückengruppe der Formel

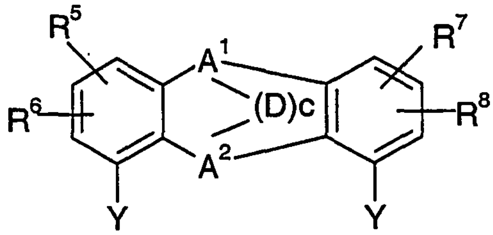

ist,
worin

$A^1$ und $A^2$ unabhängig voneinander für O, S, $SiR^aR^b$, $NR^c$ oder $CR^dR^e$ stehen, wobei

$R^a$, $R^b$ und $R^c$ unabhängig voneinander für Wasserstoff, Alkyl, Cycloalkyl, Heterocycloalkyl, Aryl oder Hetaryl stehen,

$R^d$ und $R^e$ unabhängig voneinander für Wasserstoff, Alkyl, Cycloalkyl, Heterocycloalkyl, Aryl oder Hetaryl stehen oder gemeinsam mit dem Kohlenstoffatom, an das sie gebunden sind, eine Cycloalkylidengruppe mit 4 bis 12 Kohlenstoffatomen bilden oder die Gruppe $R^d$ gemeinsam mit einer weiteren Gruppe $R^d$ oder die Gruppe $R^e$ gemeinsam mit einer weiteren Gruppe $R^e$ eine intramolekulare Brückengruppe D bilden,

D eine zweibindige Brückengruppe, ausgewählt aus den Gruppen

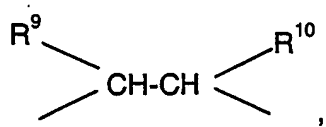, 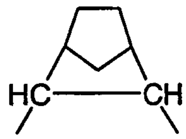, 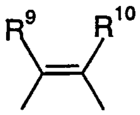, 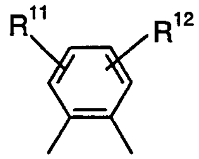, 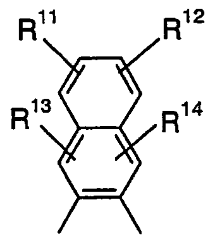

ist, in denen

| | |
|---|---|
| $R^9$ und $R^{10}$ | unabhängig voneinander für Wasserstoff, Alkyl, Cycloalkyl, Aryl, Halogen, Trifluormethyl, Carboxyl, Carboxylat oder Cyano stehen oder miteinander zu einer $C_3$- bis $C_4$-Alkylenbrücke verbunden sind, |
| $R^{11}$, $R^{12}$, $R^{13}$ und $R^{14}$ | unabhängig voneinander für Wasserstoff, Alkyl, Cycloalkyl, Aryl, Halogen, Trifluormethyl, COOH, Carboxylat, Cyano, Alkoxy, $SO_3H$, Sulfonat, $NE^1E^2$, Alkylen-$NE^1E^2E^{3+}X^-$, Acyl oder Nitro stehen, |
| c | 0 oder 1 ist, |
| $R^5$, $R^6$, $R^7$ und $R^3$ | unabhängig voneinander für Wasserstoff, Alkyl, Cycloalkyl, Heterocycloalkyl, Aryl, Hetaryl, $COOR^f$, $COO^-M^+$, $SO_3R^f$, $SO^-_3M^+$, $NE^1E^2$, $NE^1E^2E^{3+}X^-$, Alkylen-$NE^1E^2E^{3+}X^-$, $OR^f$, $SR^f$, $(CHR^9CH_2O)_xR^f$, $(CH_2N(E^1))_xR^f$, $(CH_2CH_2N(E^1))_xR^f$, Halogen, Trifluormethyl, Nitro, Acyl oder Cyano stehen, |

worin

| | |
|---|---|
| $R^f$, $E^1$, $E^2$ und $E^3$ | jeweils gleiche oder verschiedene Reste, ausgewählt unter Wasserstoff, Alkyl, Cycloalkyl oder Aryl bedeuten, |
| $R^9$ | für Wasserstoff, Methyl oder Ethyl steht, |
| $M^+$ | für ein Kation steht, |
| $X^-$ | für ein Anion steht, und |
| x | für eine ganze Zahl von 1 bis 120 steht, |

oder

$R^5$ und/oder $R^7$ zusammen mit zwei benachbarten Kohlenstoffatomen des Benzolkerns, an den sie gebunden sind, für ein kondensiertes Ringsystem, mit 1, 2 oder 3 weiteren Ringen stehen.

Solche Liganden sind Gegenstand von WO 02/083695, auf die hiermit in vollem Umfang Bezug genommen wird und wo auch die Herstellung dieser Liganden beschrieben wird. Bevorzugte Liganden aus dieser Klasse sind z.B. die folgenden Verbindungen, wobei diese Aufzählung lediglich der Veranschaulichung dient und keinen einschränkenden Charakter bezüglich der anwendbaren Liganden hat.

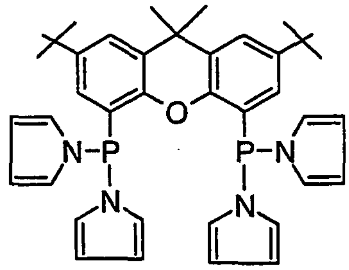

1

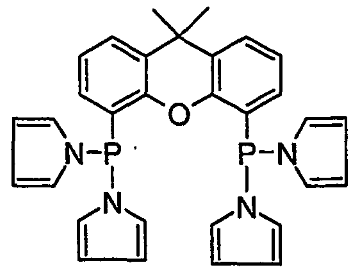

2

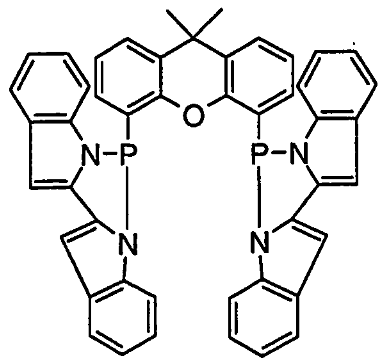

3

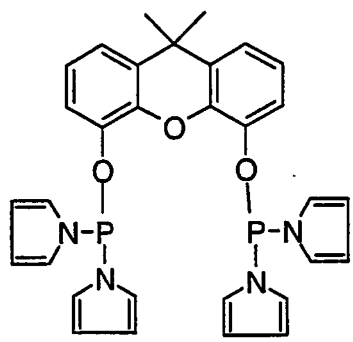

4

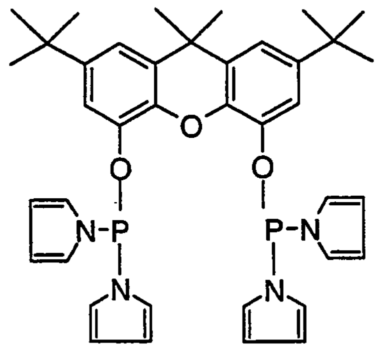

5

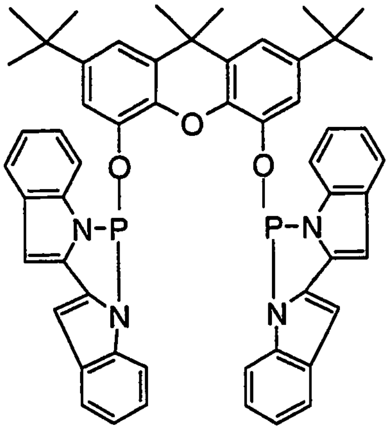

6

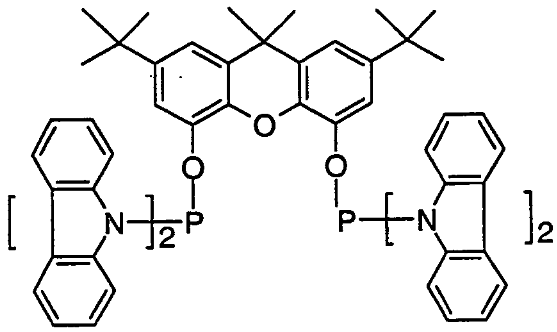

7

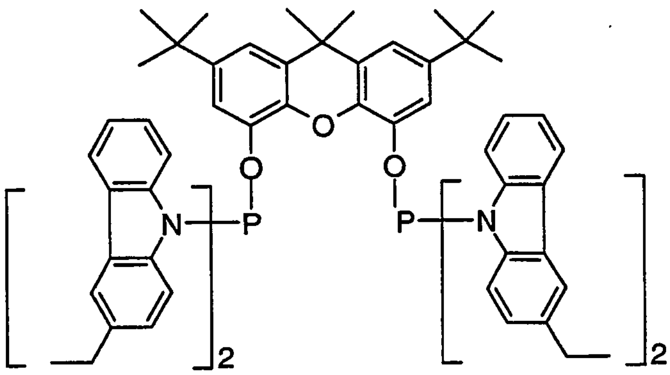

8

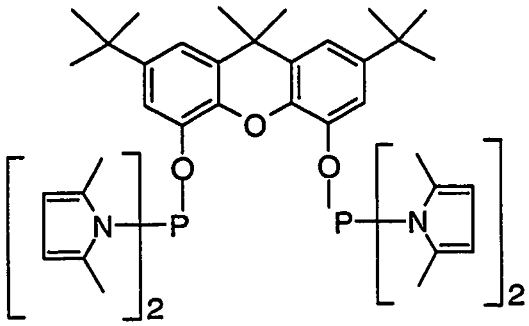

9;

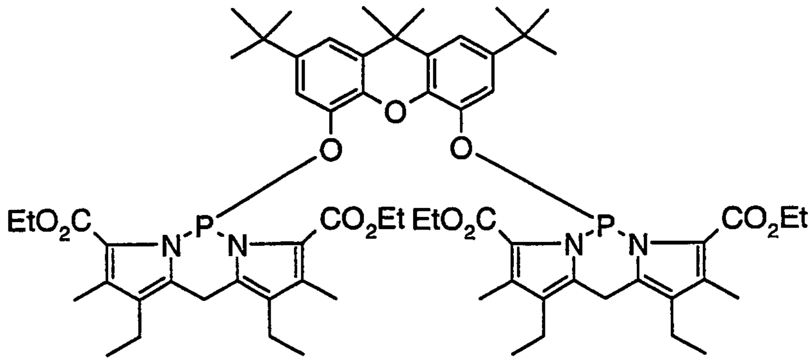

10

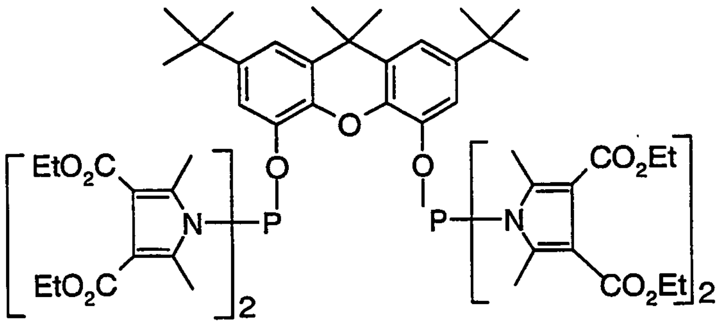

11

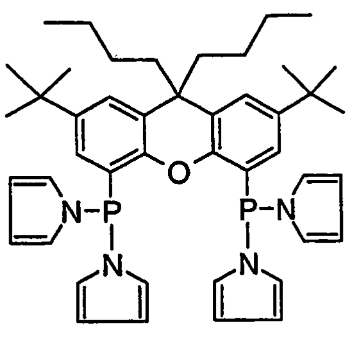

12

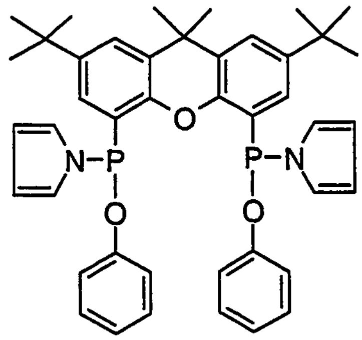

13

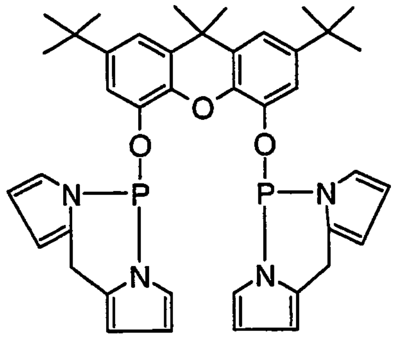

14

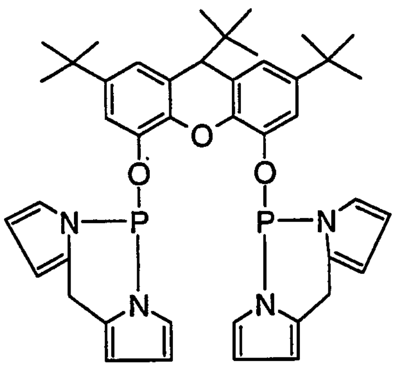

15

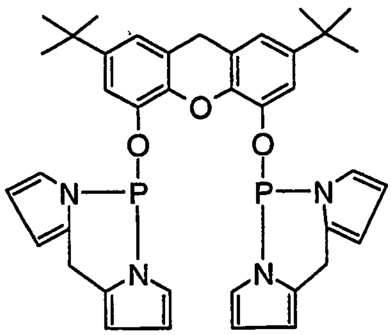

16

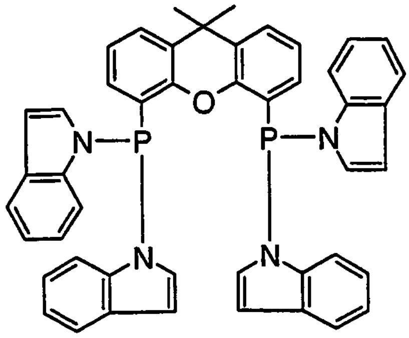

17

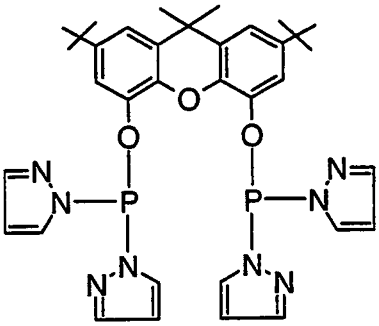

18

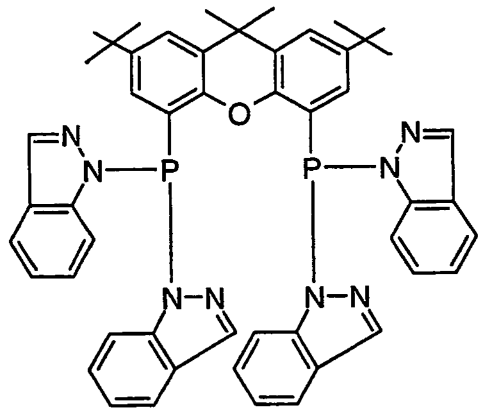

19

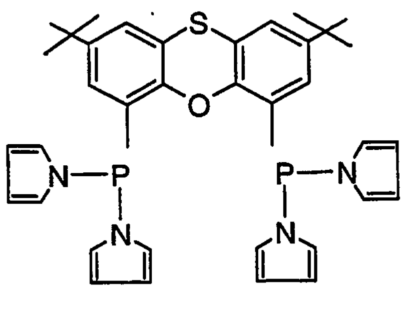

20

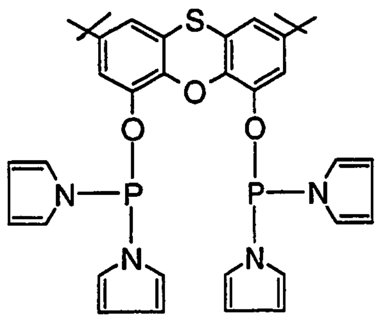

21

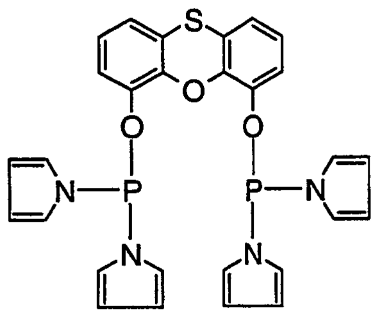

22

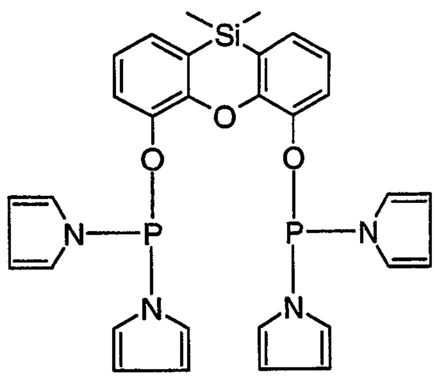

23

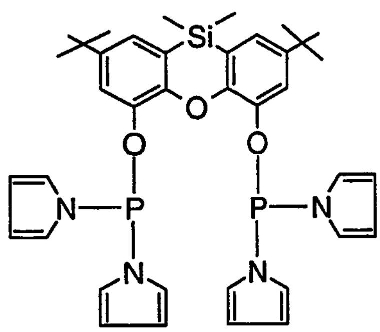

24

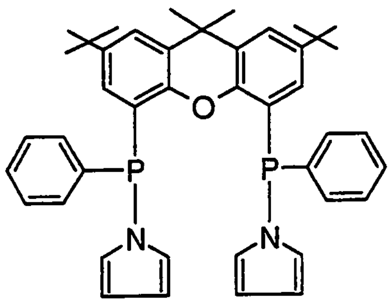

25

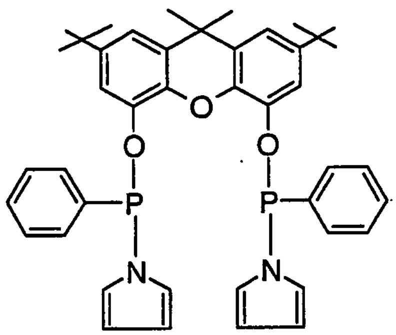

26

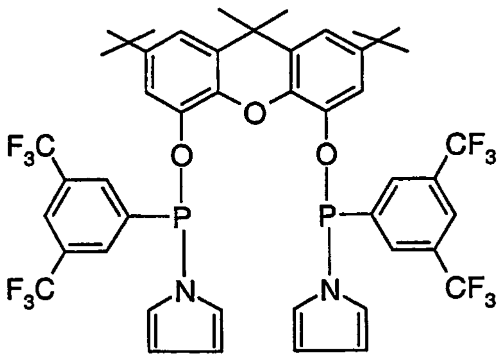

27

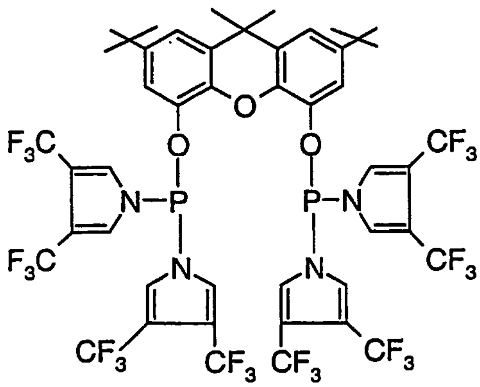

28

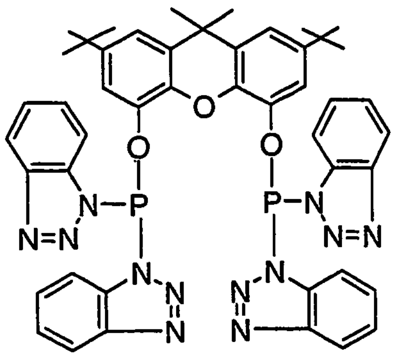

29

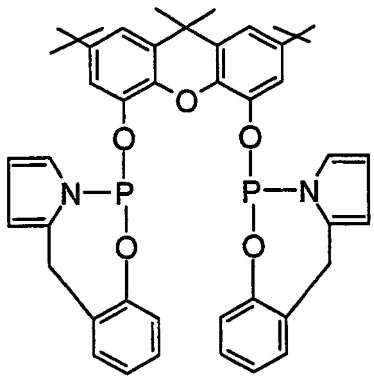

30

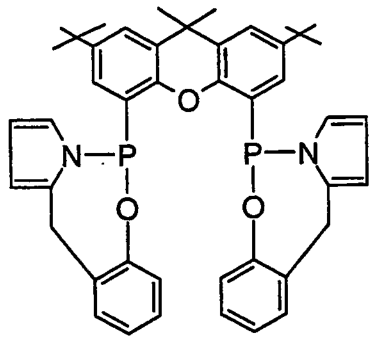

31

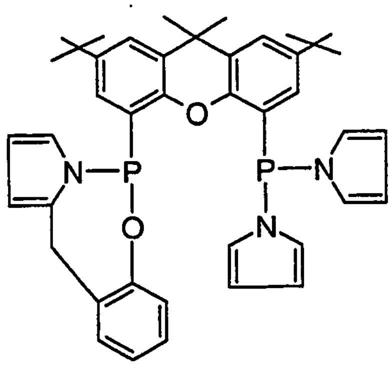

32

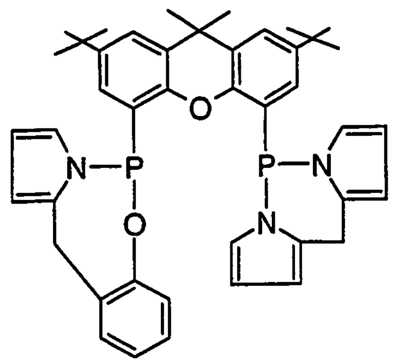

33

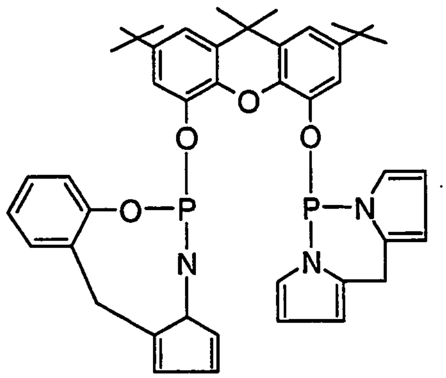

34

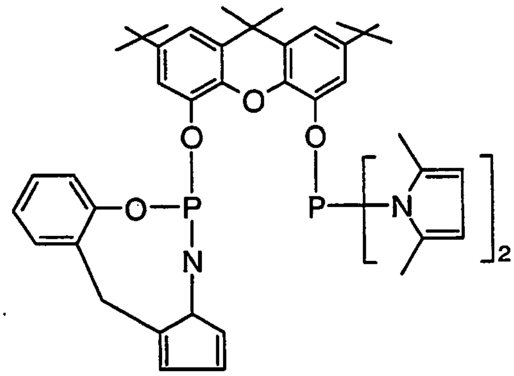

35

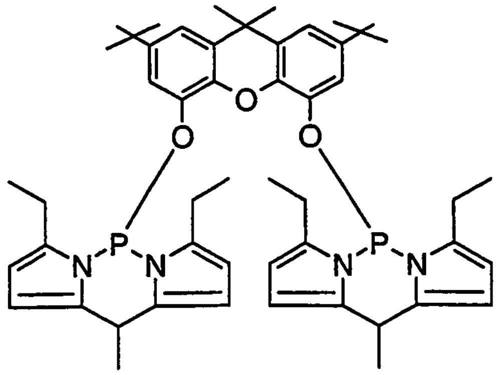

36

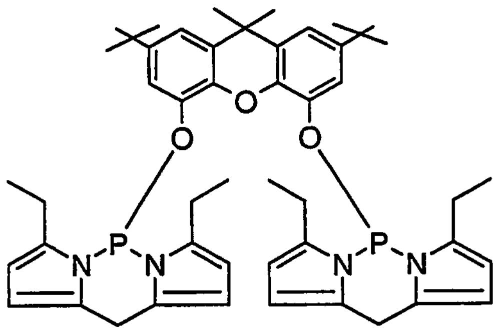

37

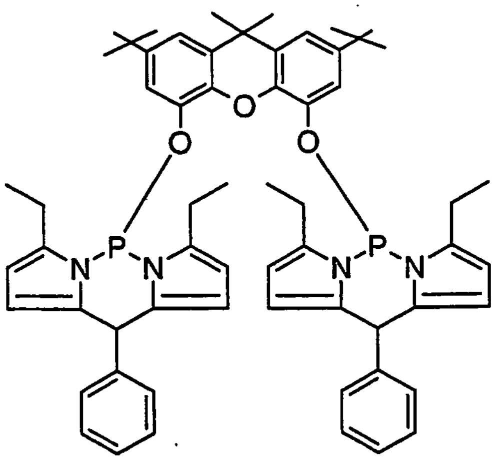

38

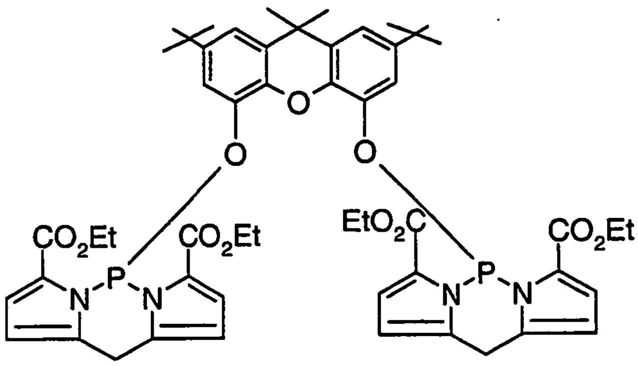

39

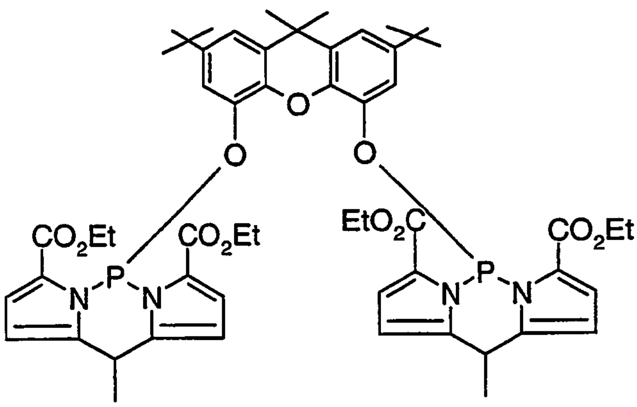

40

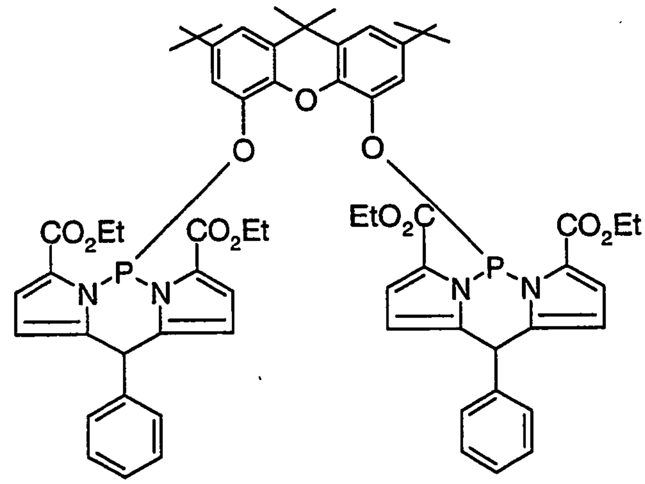

41

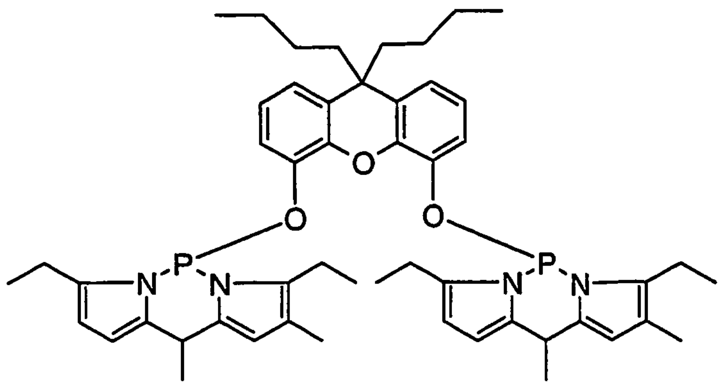

42

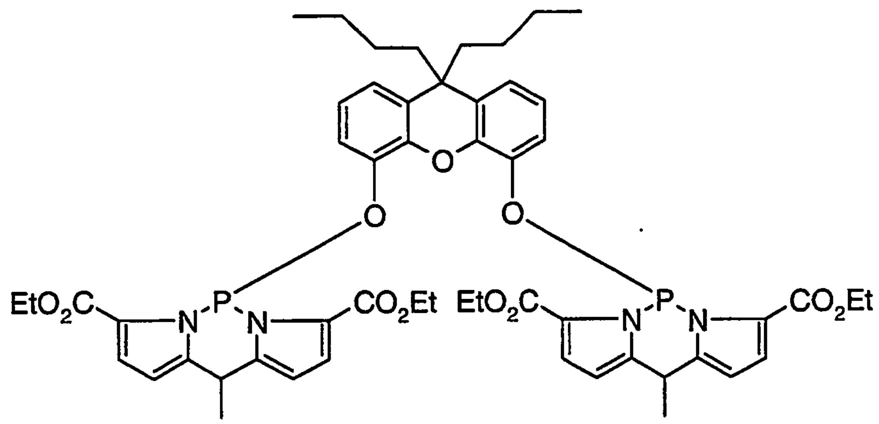

43

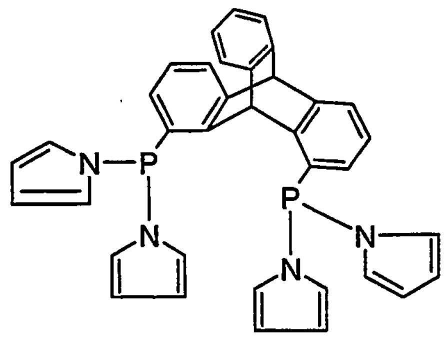

44

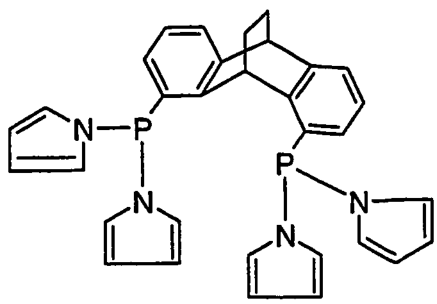

45

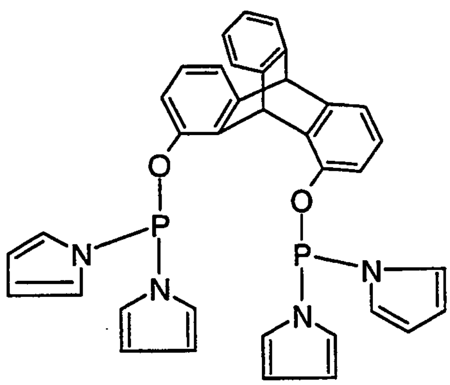

46

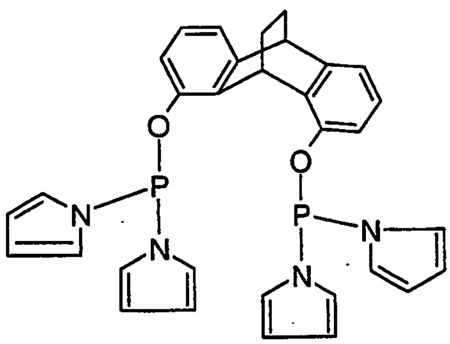

47

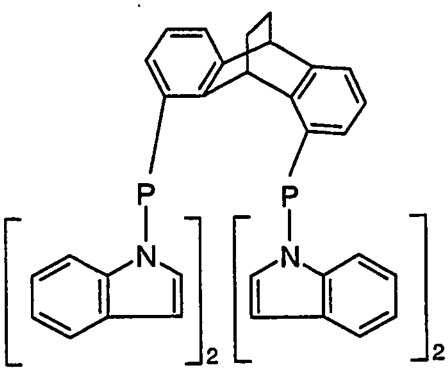

48

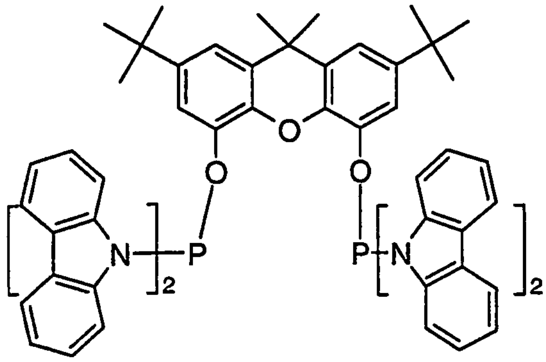

49

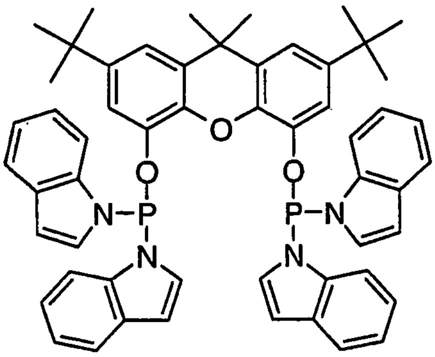

50

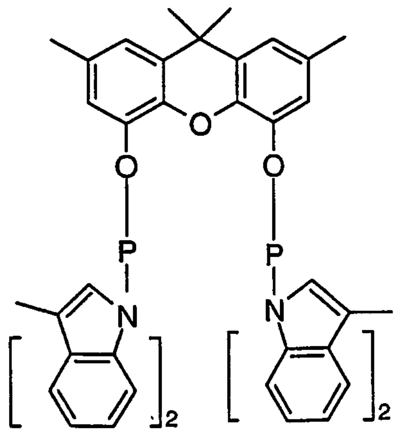

51

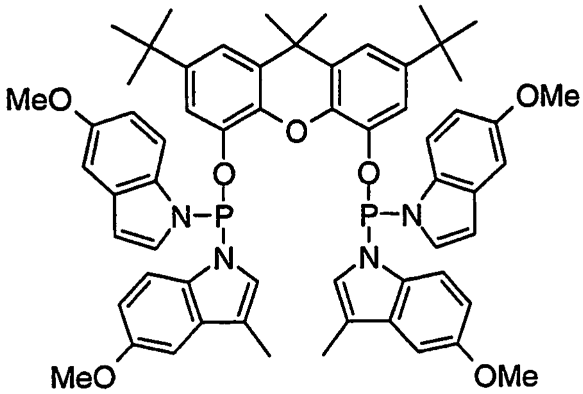

52

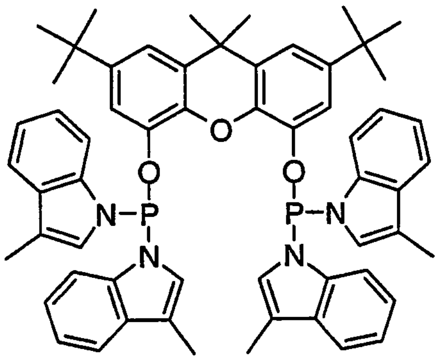

53

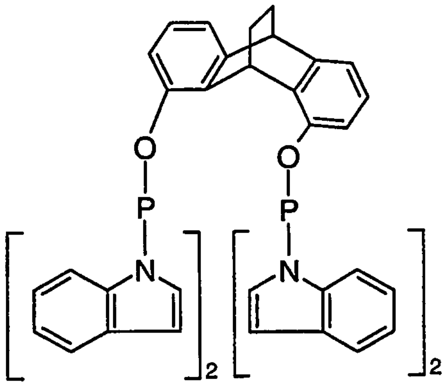

54

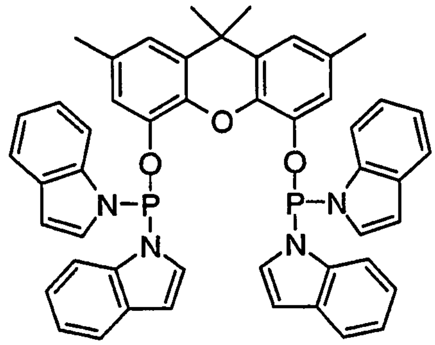

55

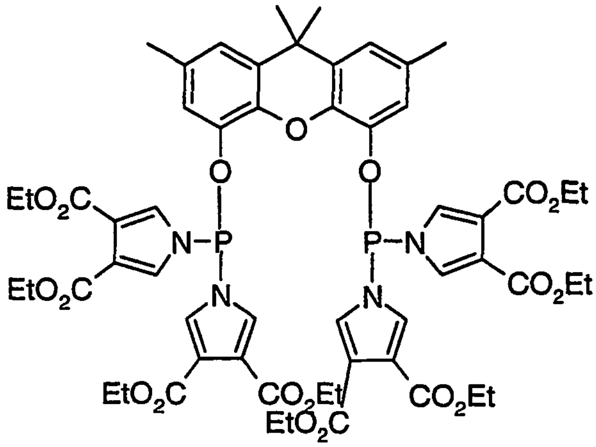

56

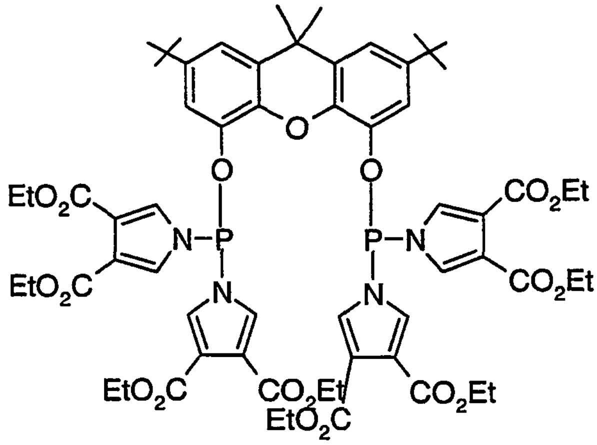

57

Et: Ethyl
Me: Methyl

Geeignete Phosphoramiditliganden für die isomerisierende Hydroformylierung mit Rhodiumkomplexkatalysatoren sind auch die Phosphoramiditliganden gemäß WO 98/19985 und WO 99/52632, mit 2,2'-Dihydroxy-1,1'-biphenylen- oder 2,2'-Dihydroxy-1,1'-binaphtylen-Brückengruppen, die an das Phosphoratom über das Stickstoffatom verknüpfte Heteroarylgruppen, wie Pyrrolyl- oder Indolyl-Gruppen, tragen, beispielsweise die Liganden.

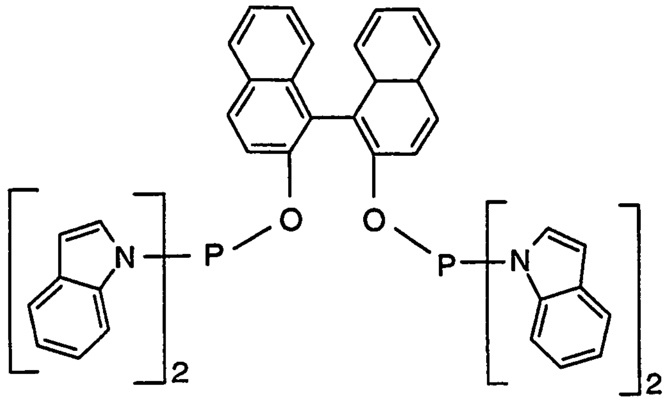

58

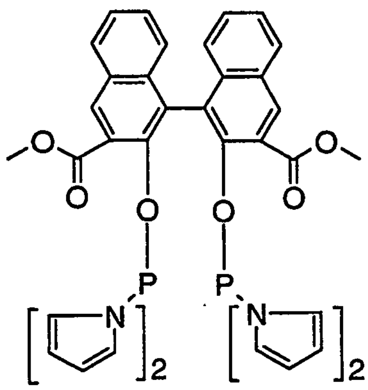

59

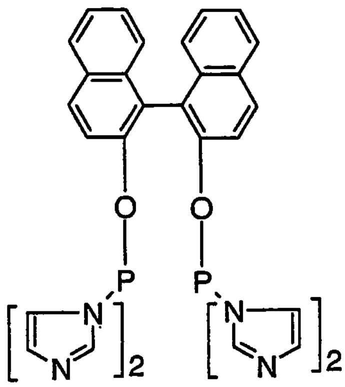

60

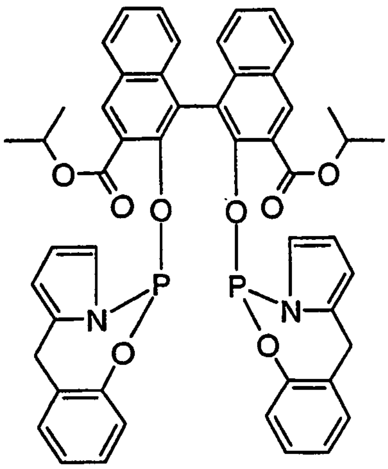

61

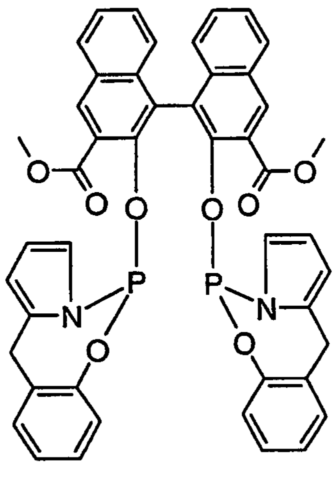

62

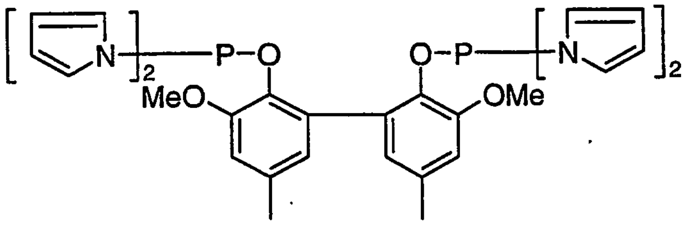

63

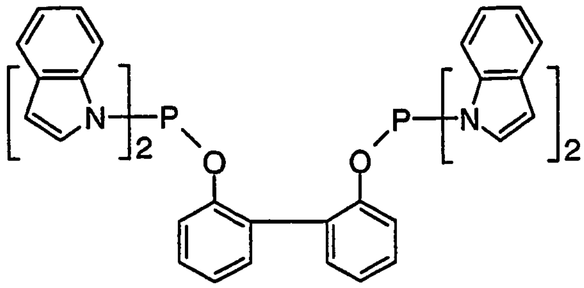

64

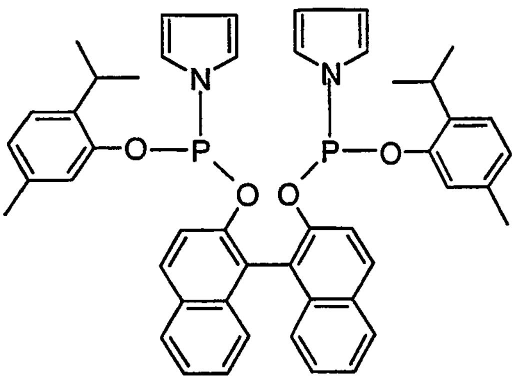

65

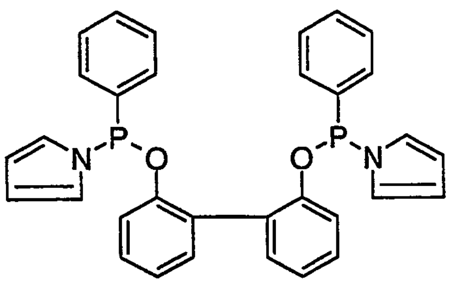

66

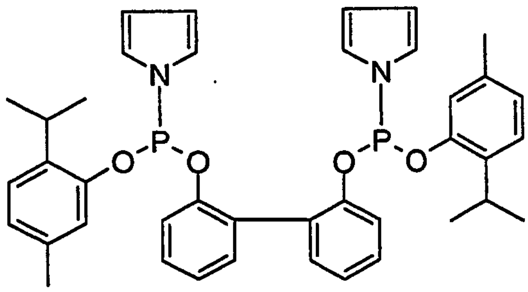

67

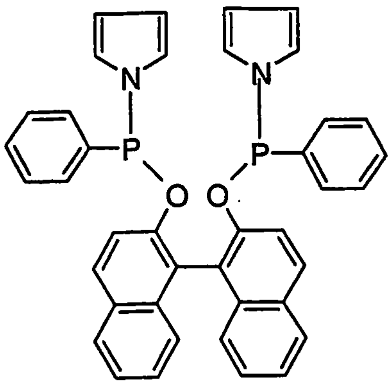

68

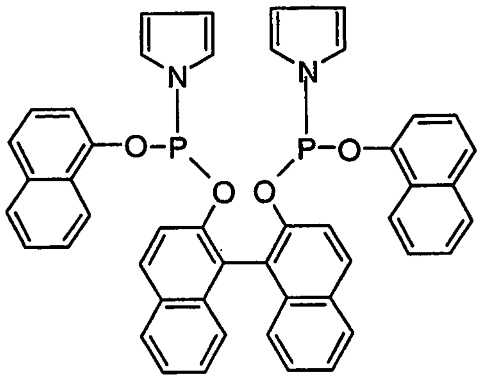

69

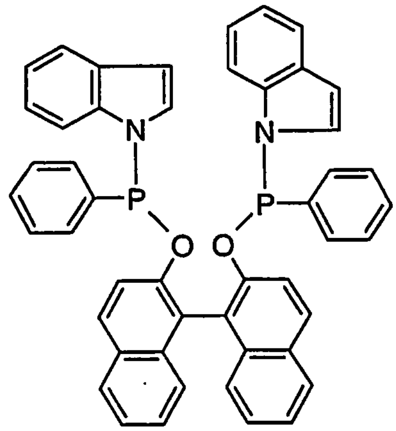

70

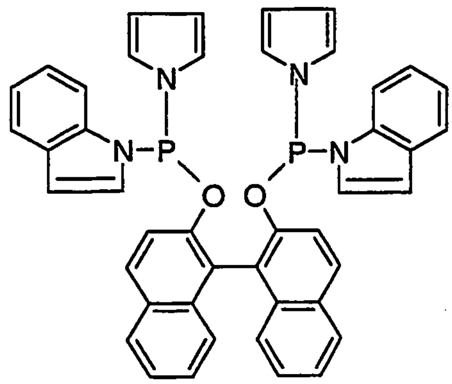

71

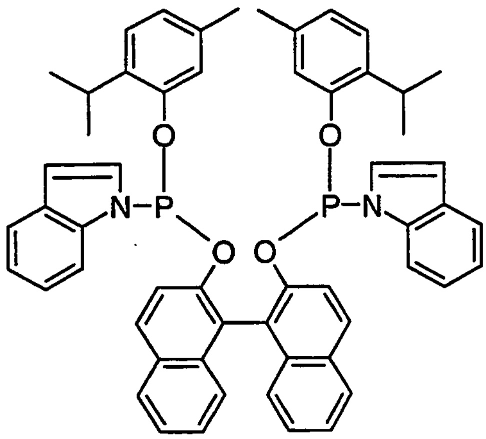

72

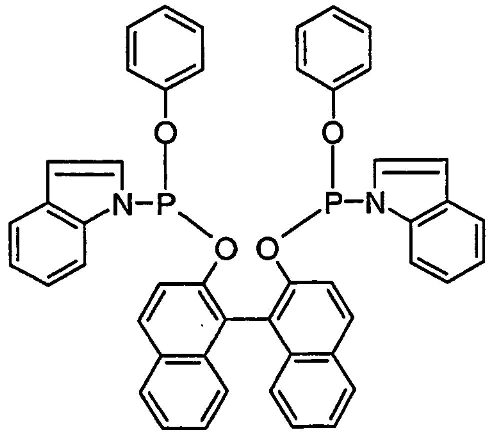

73

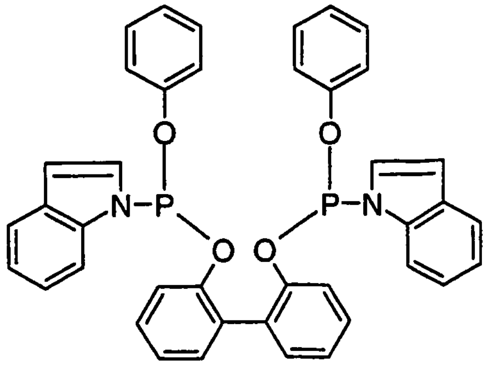

74

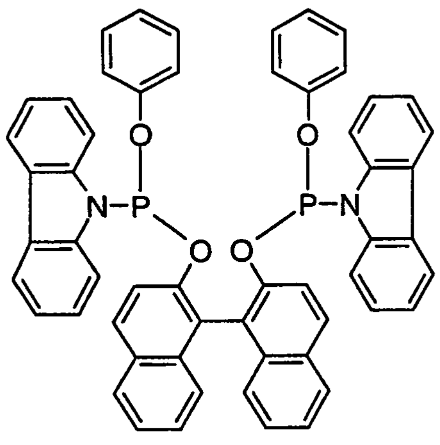

75

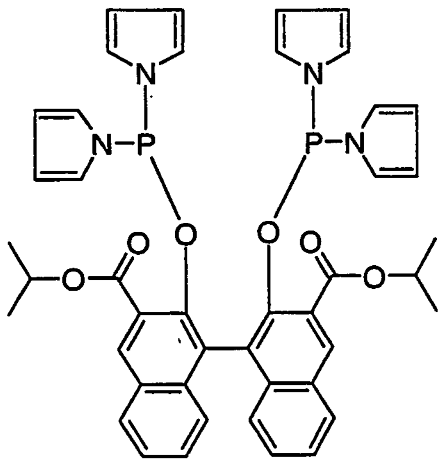

76

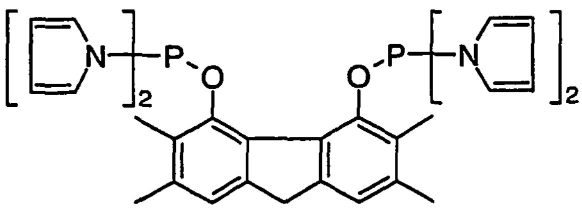

77

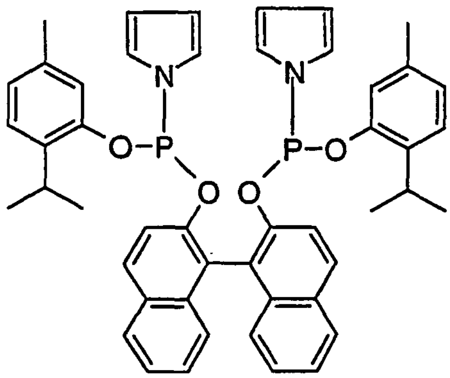

78

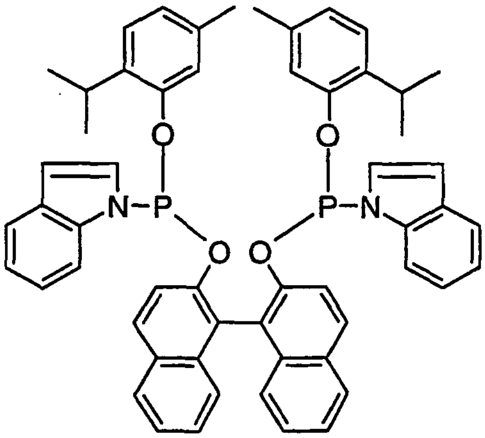

79

Die 1,1'-Biphenylen- bzw. 1,1'Binaphthylenbrückengruppen dieser Liganden können über die 1,1-Position noch durch eine Methylen- ($CH_2$-), eine 1,1-Ethyliden- ($CH_3$-CH<) oder eine 1,1-Propylidengruppe ($CH_3$-$CH_2$-HC<) verbrückt sein.

Geeignete Phosphinitliganden für die isomerisierende Hydroformylierung mit Rhodiumkomplexkatalysatoren sind u.a. die in WO 98/19985 beschriebenen Liganden, beispielsweise

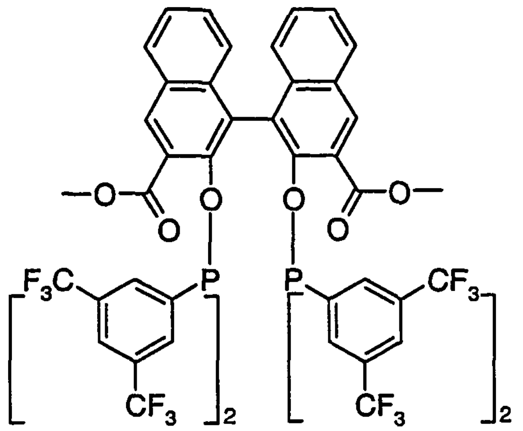

80

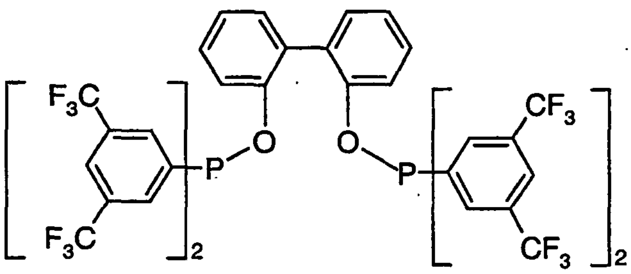

81

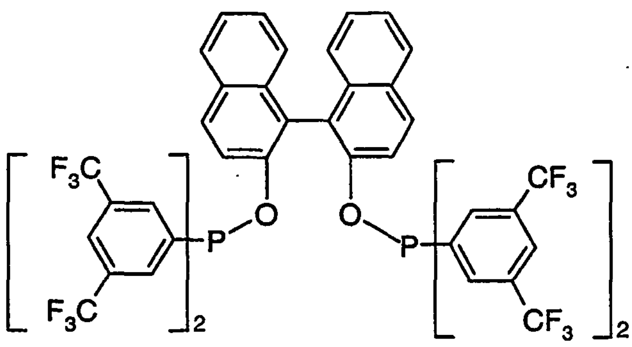

82

Geeignet als Liganden für die isomerisierende Hydroformylierung mit Rhodiumkomplexkatalysatoren sind auch Phosphit- und Phosphonit-Liganden, wie sie beispielsweise in WO 01/58589 beschrieben werden. Lediglich zur Erläuterung seien beispielhaft die folgenden Liganden genannt.

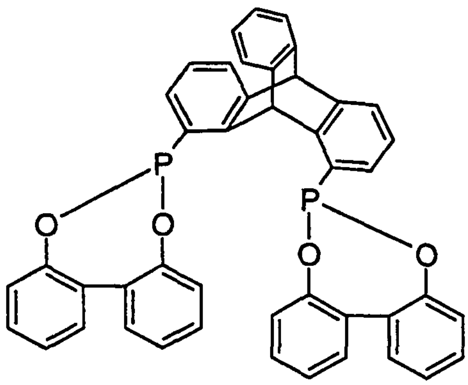

83

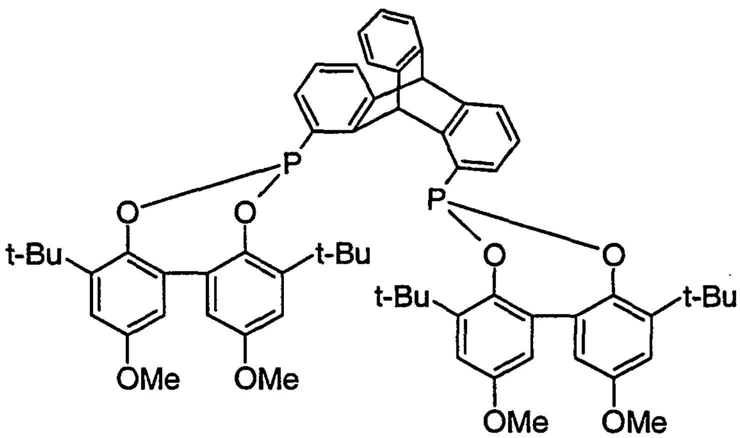

84

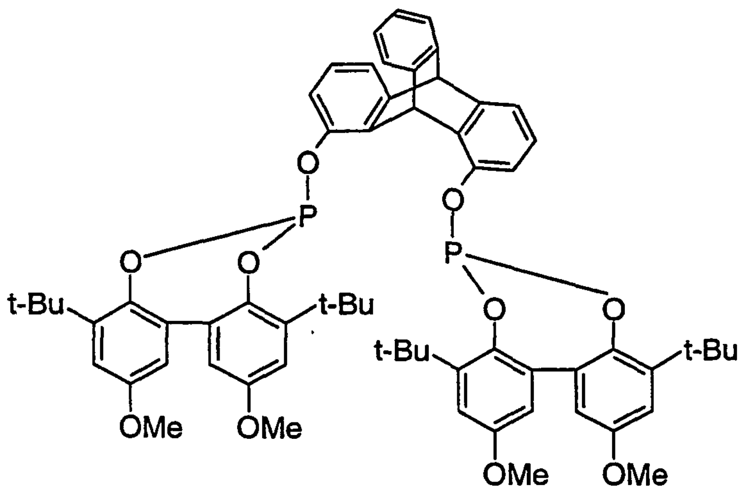

85

Gut geeignet als Liganden für die isomerisierende Hydroformylierung mit Rhodiumkomplexkatalysatoren sind auch Phosphinliganden mit dem Xanthenyl-bisphosphoxanthenyl-Gerüst, wie sie beispielsweise in WO 02/068371 und EP-A 982314 beschrieben werden. Lediglich zur Veranschaulichung sind im folgenden einige dieser Liganden exemplarisch aufgeführt.

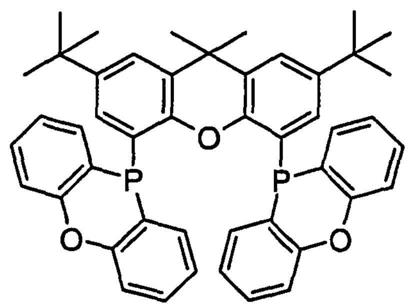

86

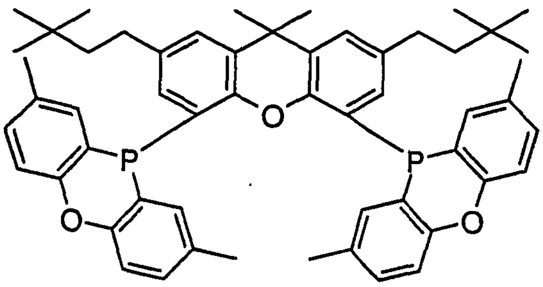

87

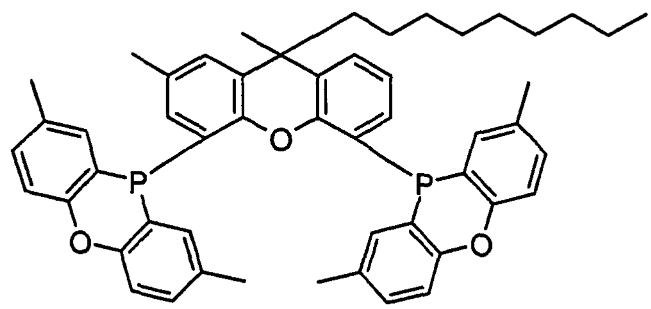

88

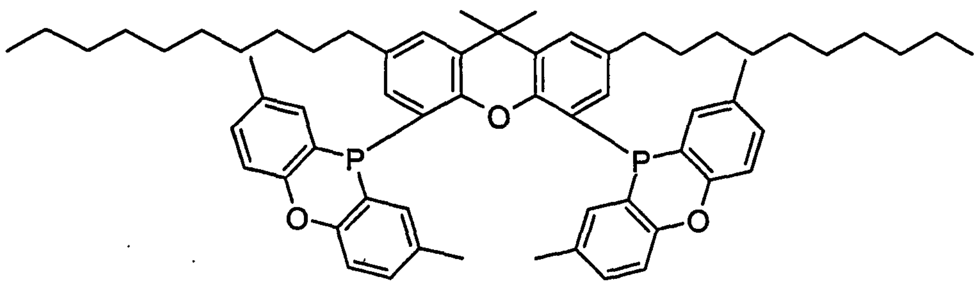

89

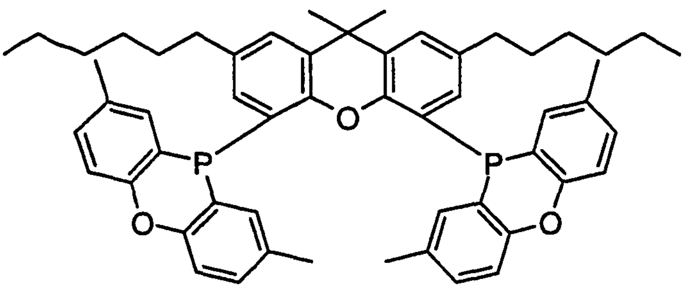

90

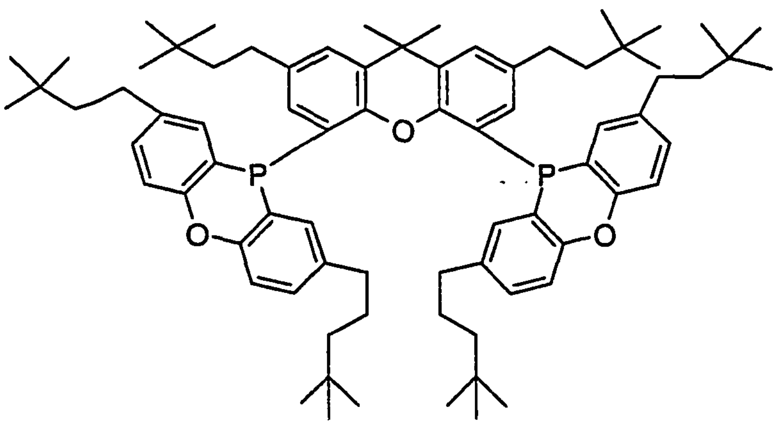

91

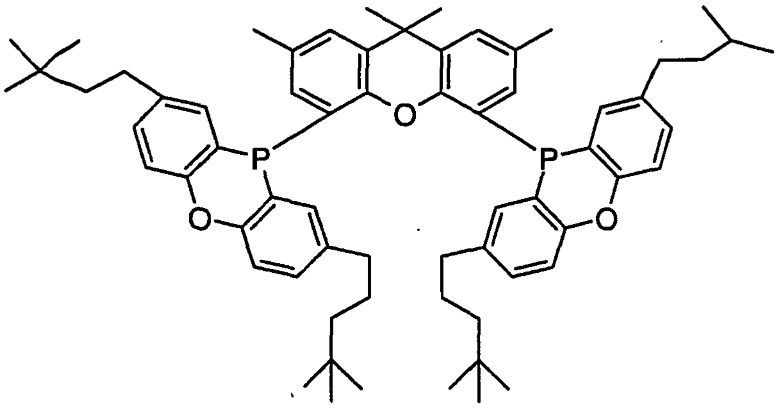

92

Geeignete Chelatphosphitliganden für die isomerisierende Hydroformylierung mit Rhodiumkomplexkatalysatoren dieser Liganden sind z.B. solche der allgemeinen Formel II, III und IV

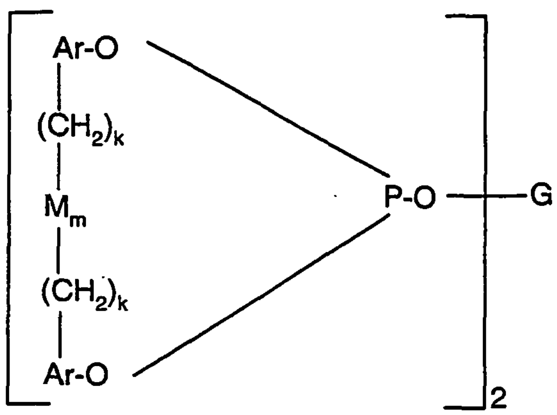

II

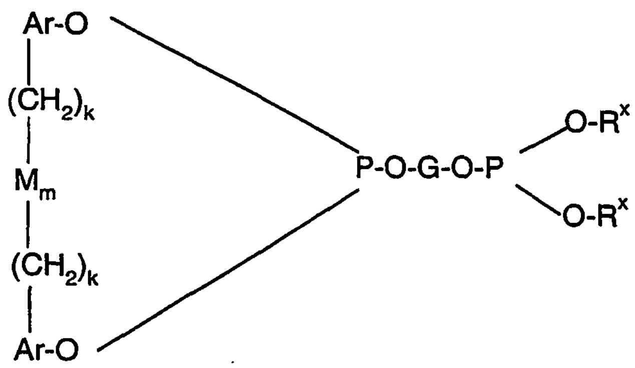

III

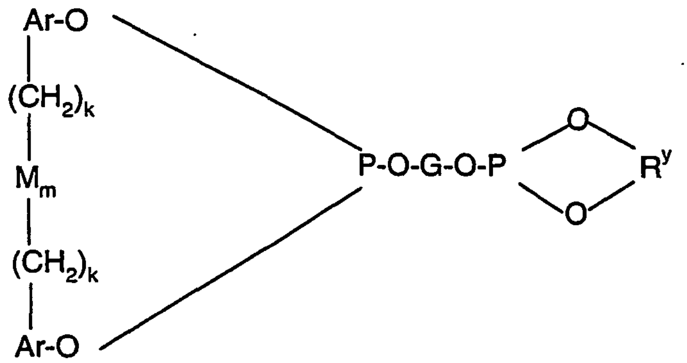

IV

in denen G eine substituierte oder unsubstituierte zweiwertige organische Brückengruppe mit 2 bis 40 Kohlenstoffatomen ist, M für eine zweiwertige Brückengruppe steht, ausgewählt aus $-C(R^w)_2-$, -O-, -S-, $NR^v$, $Si(R^t)_2-$ und -CO-, worin die Gruppen $R^w$ gleich oder verschieden sind und für Wasserstoff, eine Alkylgruppe mit 1 bis 12 Kohlenstoffatomen, eine Phenyl-, Tolyl- und Anisyl-Gruppen stehen, die Gruppen $R^v$ Wasserstoff oder eine substituierte oder unsubstituierte Kohlenwasserstoffgruppe mit 1 bis 12 Kohlenstoffatomen ist, die Gruppen $R^t$ gleich oder verschieden sind und für Wasserstoff oder die Methylgruppe stehen, m die Zahl 0 oder 1 bedeutet, die Gruppen Ar gleich oder verschieden sind und für eine unsubstituierte oder substituierte Arylgruppe stehen, der Index k einen Wert von 0 oder 1 hat, die Gruppen $R^x$ gleich oder verschieden sind und für unsubstituierte oder substituierte einwertige Alkyl- oder Arylgruppen stehen und $R^Y$ ein zweiwertiger organischer Rest ist, ausgewählt aus unsubstituierten oder substituierten Alkylen-, Arylen-, Arylen-alkylen-arylen und Bis-arylen-Gruppen. Lediglich zur Veranschaulichung jedoch ohne beschränkenden Charakter seien beispielhaft die folgenden im erfindungsgemäßen Verfahren verwendbaren Chelatphosphit-Liganden genannt:

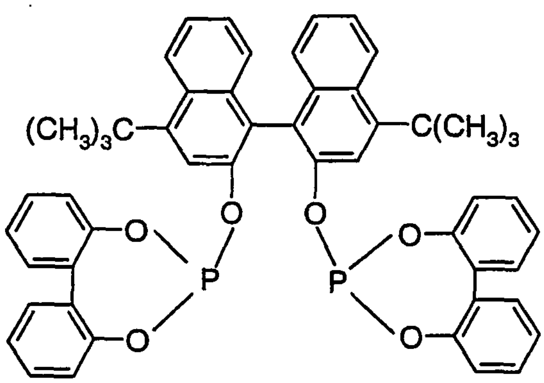

93

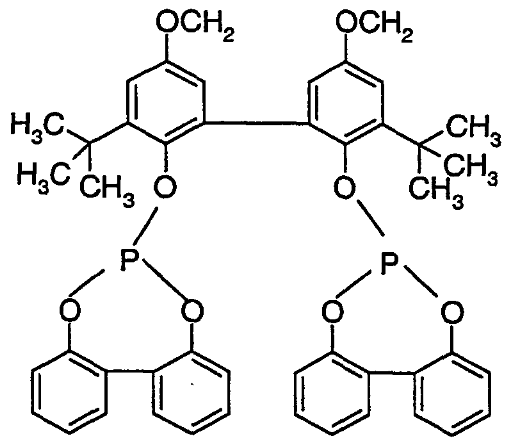

94

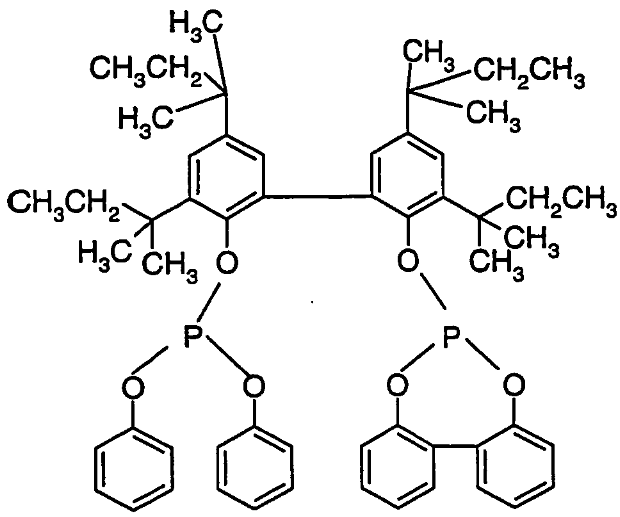

95

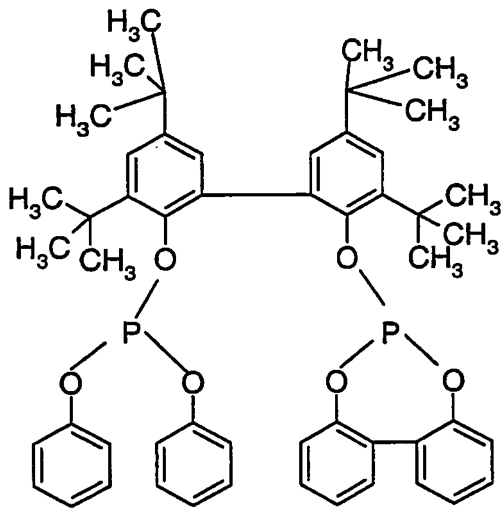

96

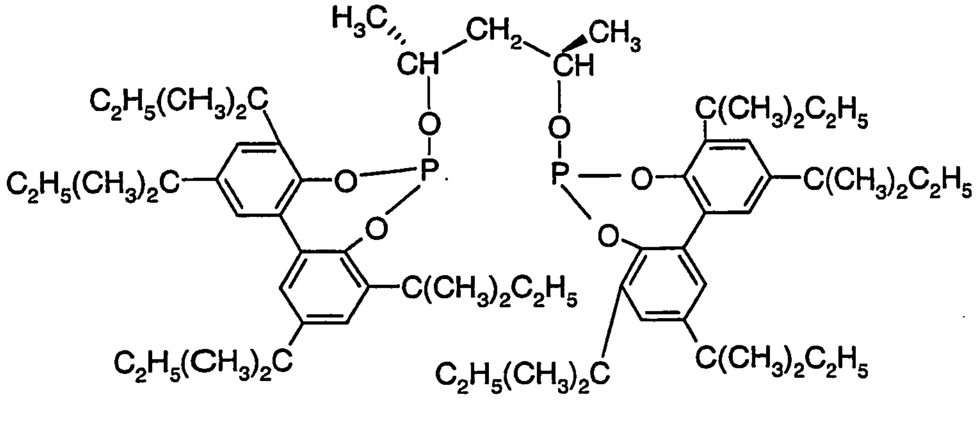

97

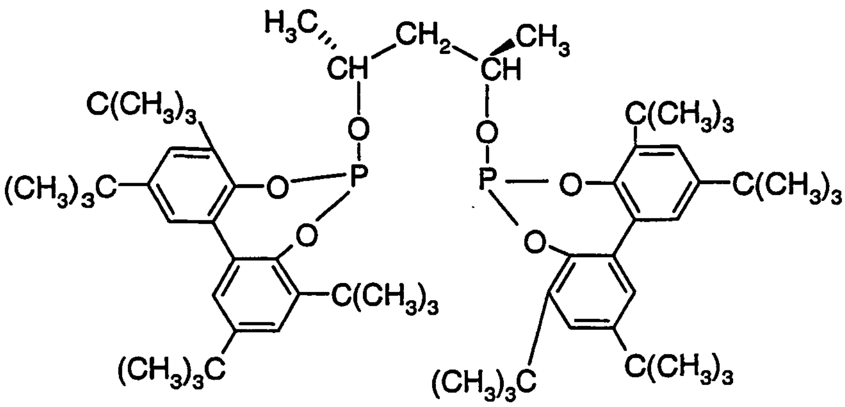

98

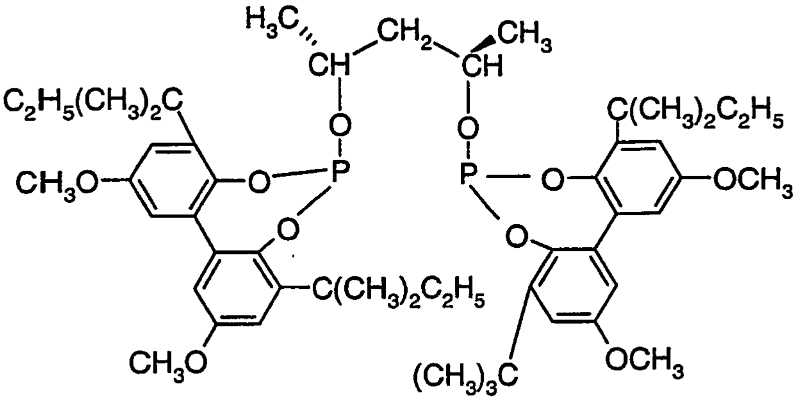

99

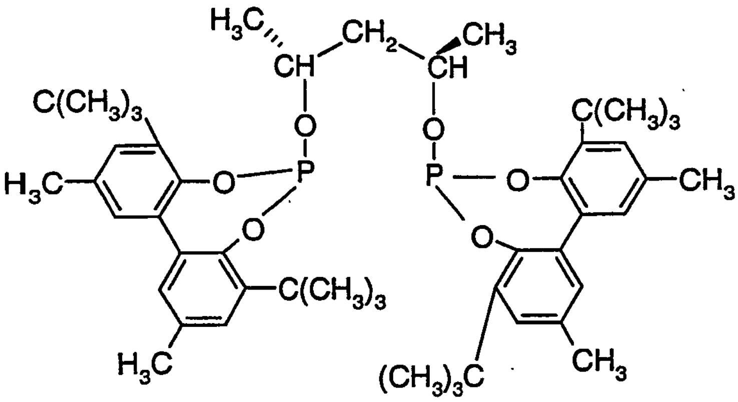

100

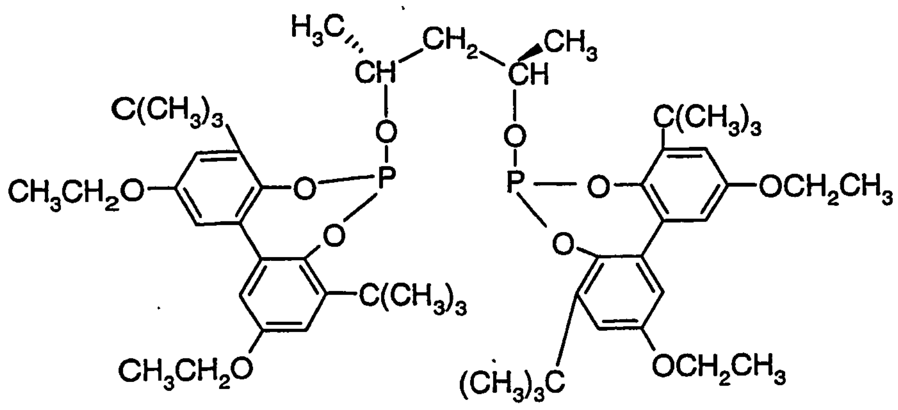

101

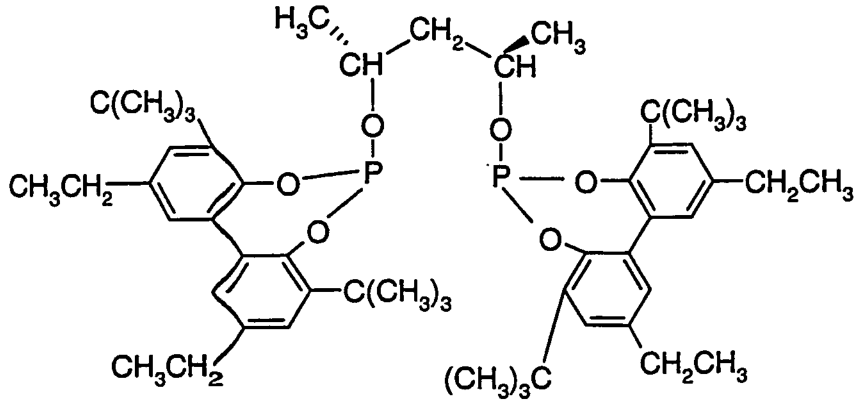

102

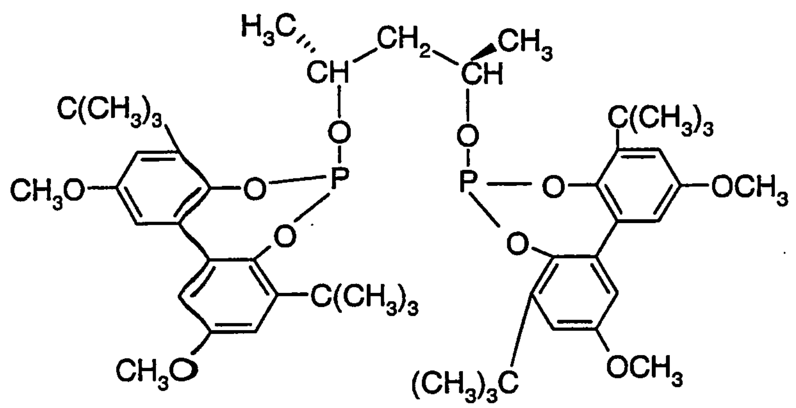

103

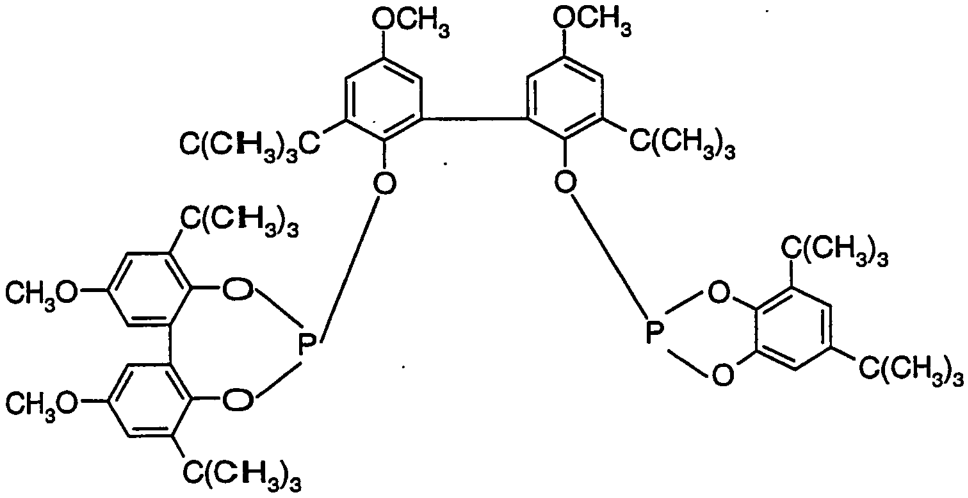

104

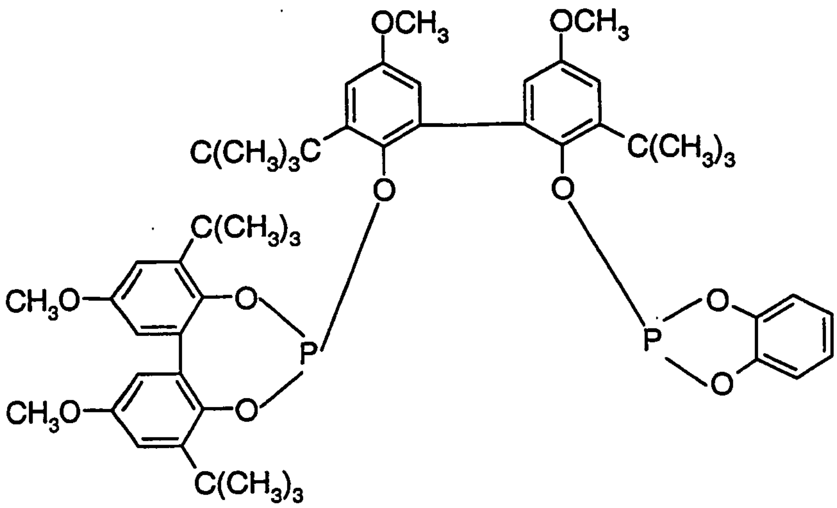

105

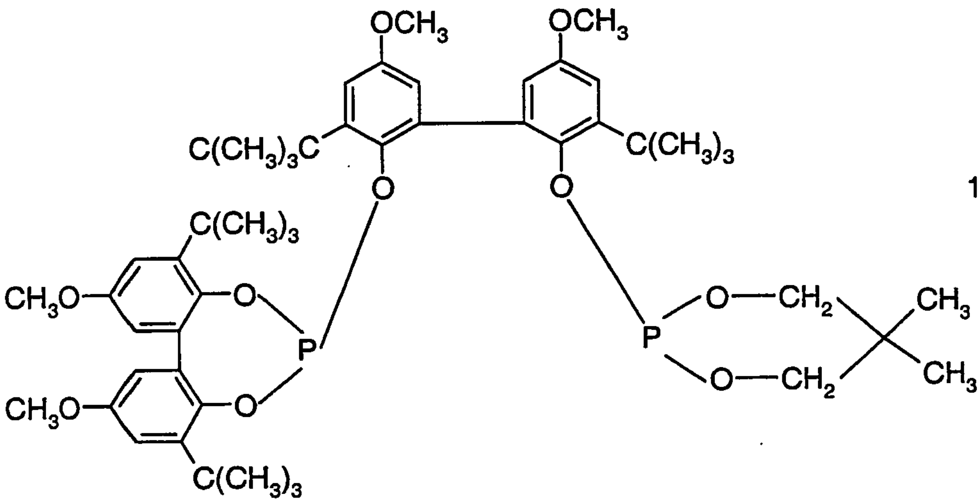

106

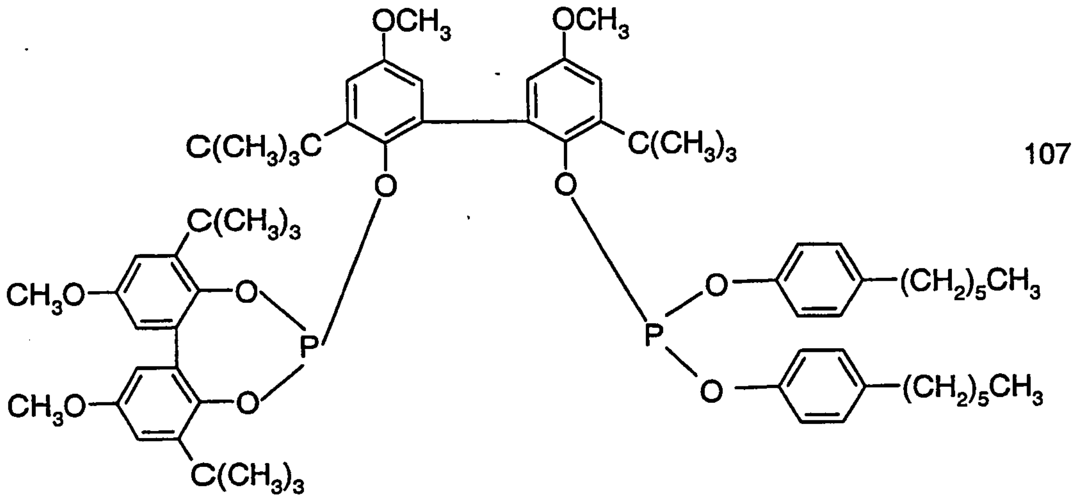

107

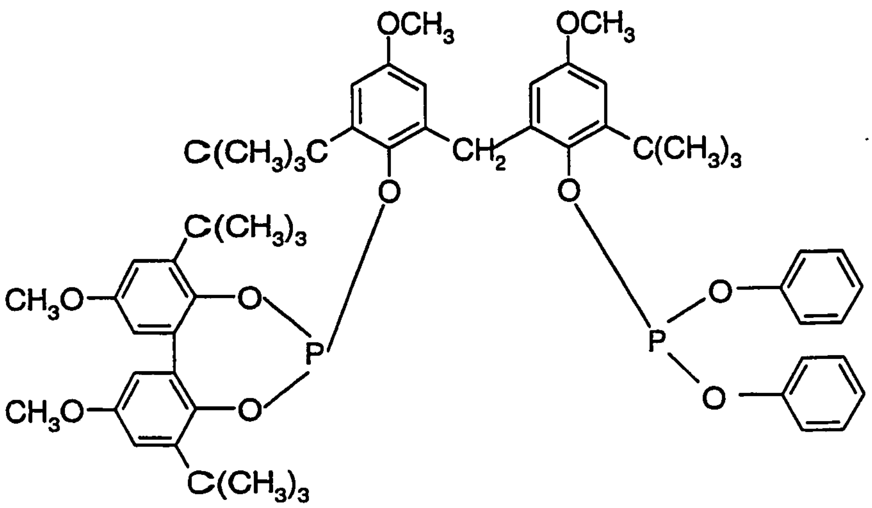

108

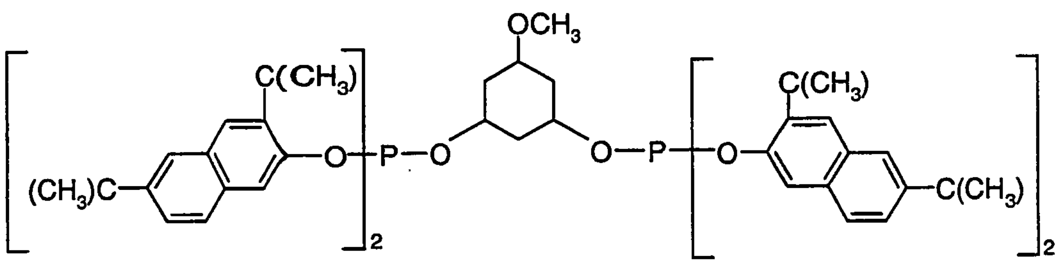

109

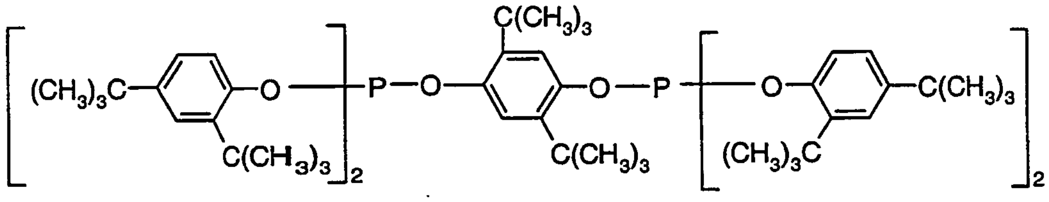

110

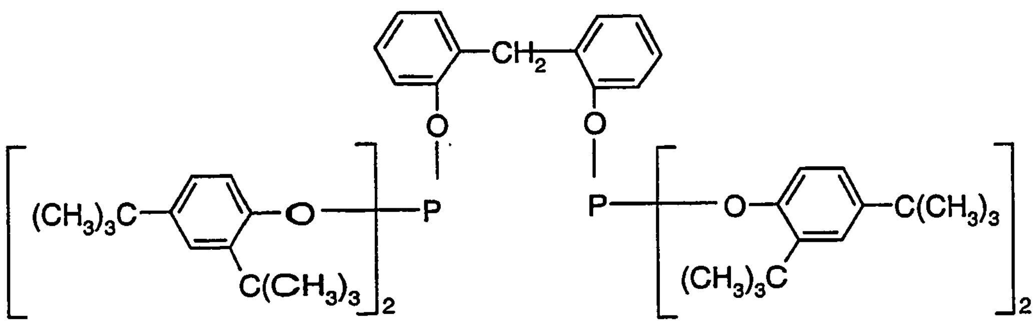

111

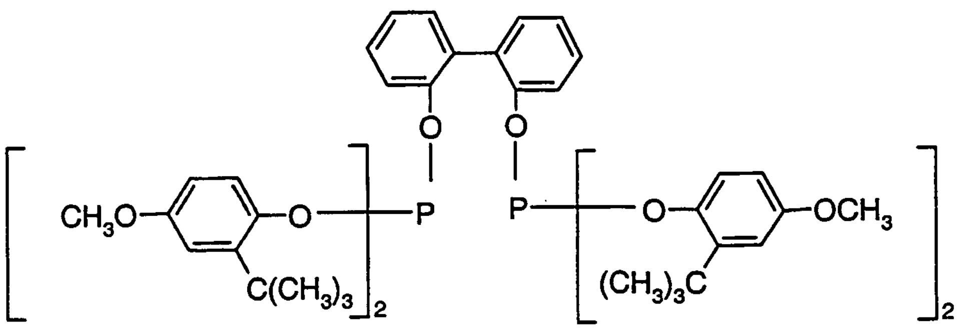

112

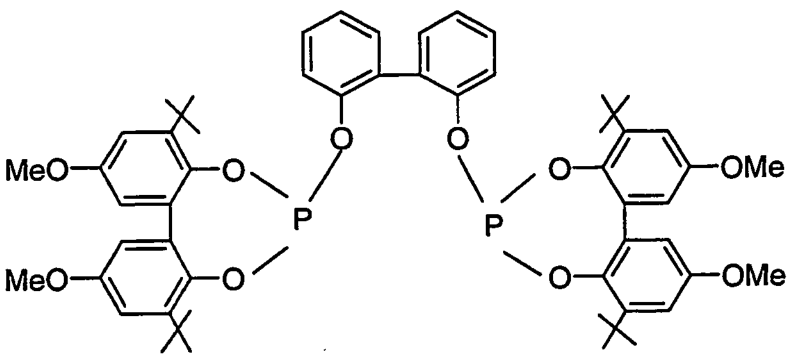

113

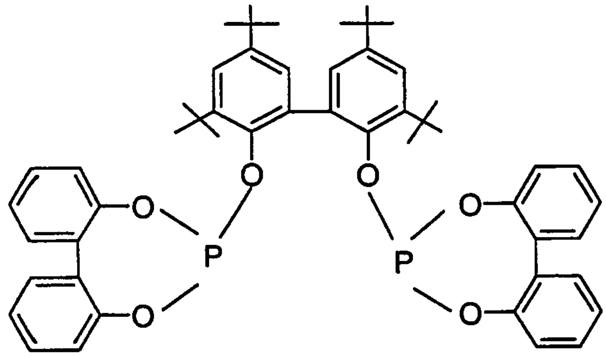

114

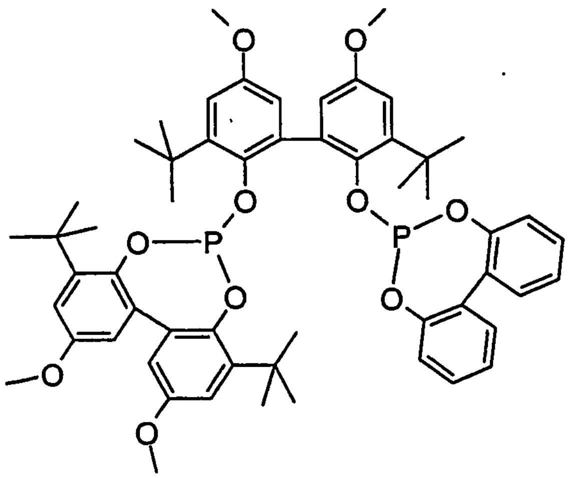

115

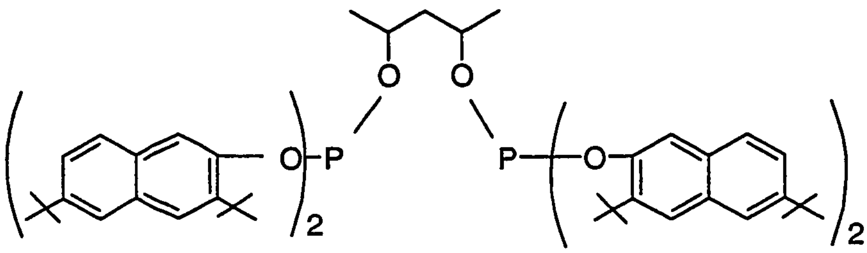

116

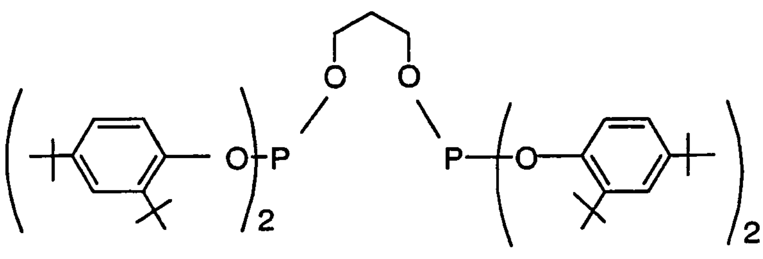

117

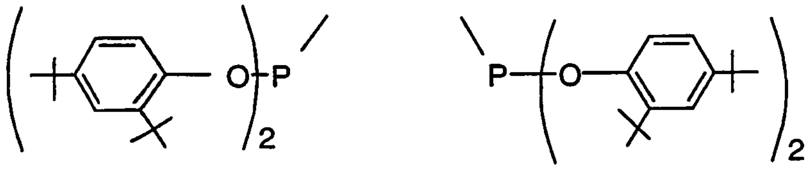

118

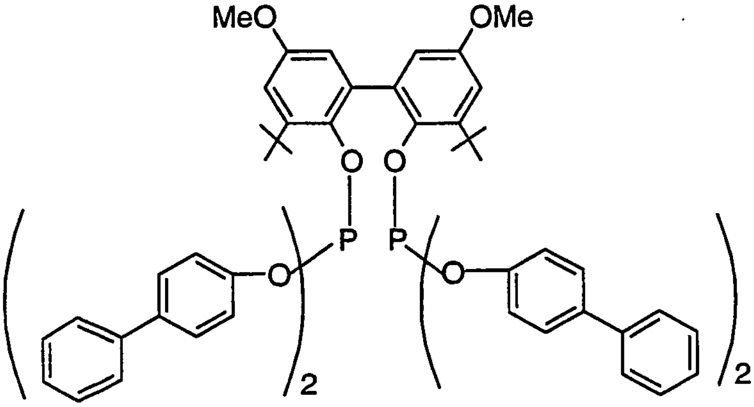

119

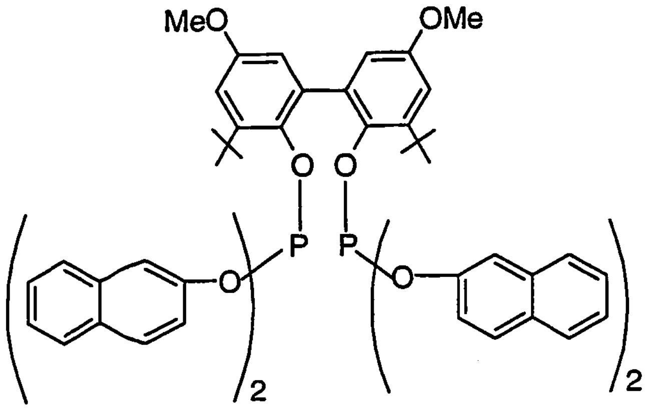

120

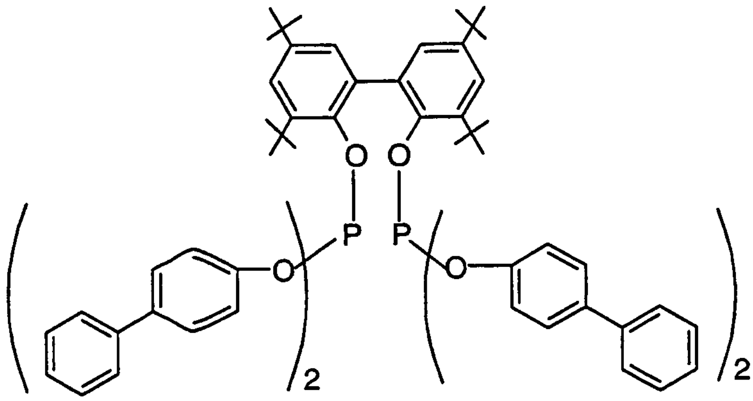

121

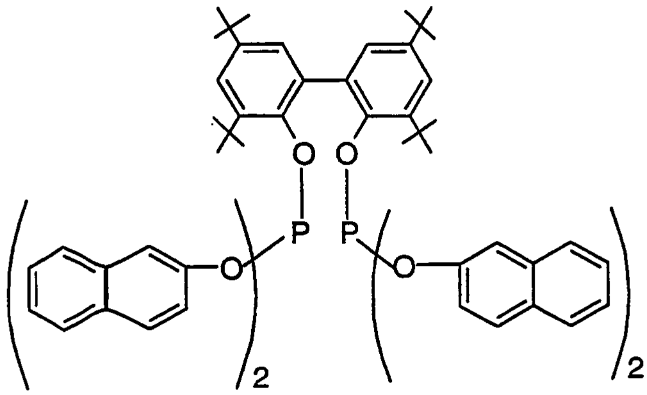

122

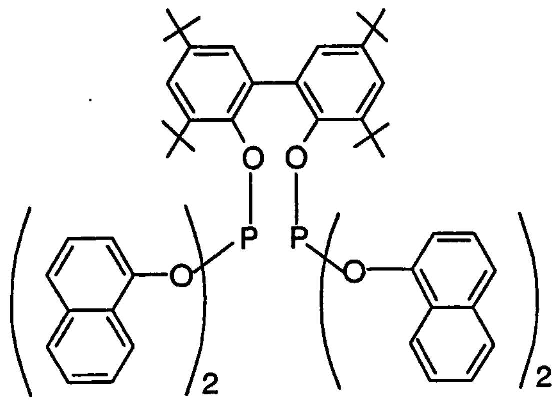

123

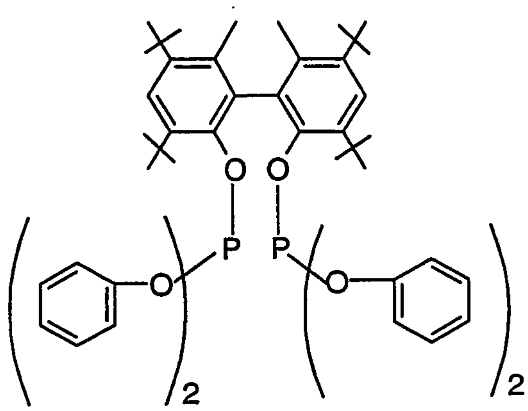

124

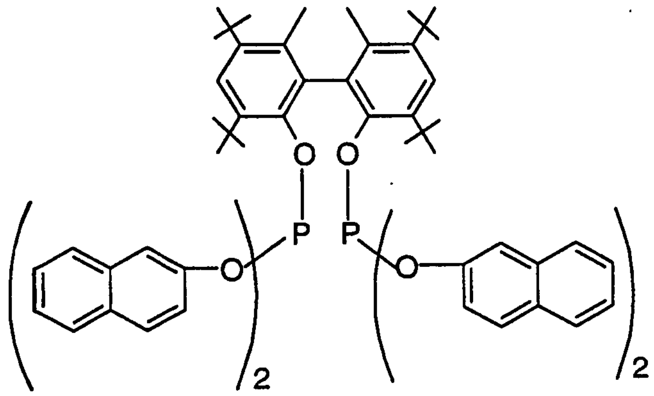

125

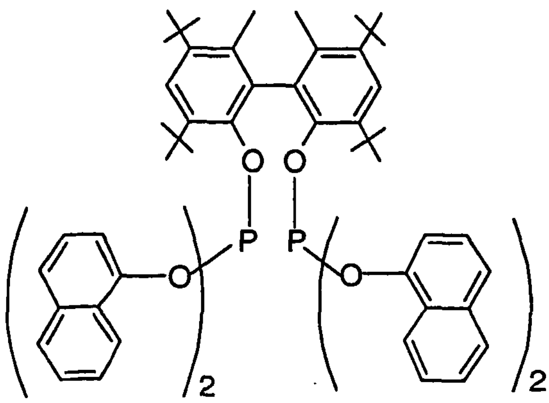

126

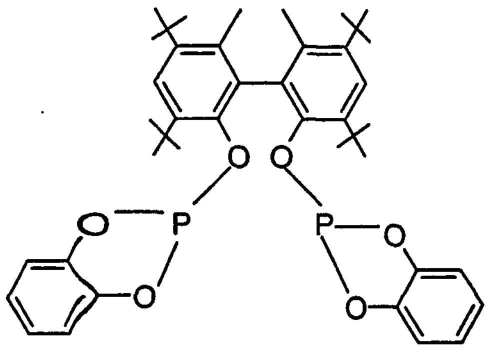

127

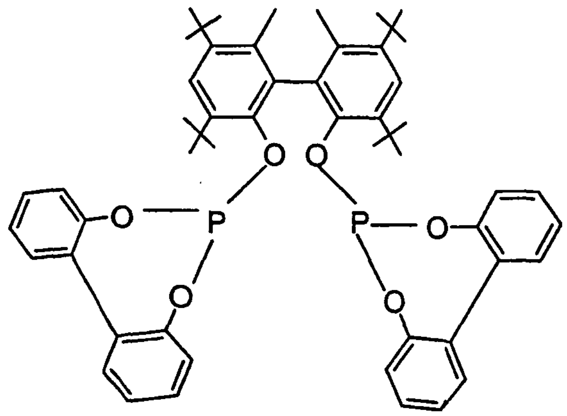

128

Solche und andere Bisphosphit-Chelatliganden sind Gegenstand von EP-A 213 369 und US-A 4 769 498 und ihre Herstellung wird dort beschrieben.

Anstelle der vorstehend genannten Bisphosphit-Chelatliganden können im erfindungsgemäßen Verfahren auch einzähnige Monophosphit-Liganden der allgemeinen Formel V

$$P\,(OR^S)\,(OR^T)\,(OR^U) \qquad V$$

zur Komplexierung des Rhodium-Hydroformylierungskatalysators und als freier Ligand verwendet werden. Die Eignung solcher Liganden und deren Komplexen mit Rhodium als Katalysatoren für die isomerisierende Hydroformylierung ist bekannt. In den Monophosphit-Liganden der allgemeinen Formel IV stehen die Reste $R^S$, $R^T$ und $R^U$ unabhängig voneinander für gleiche oder verschiedene organische Gruppen mit im Allgemeinen 1 bis 30, vorzugsweise 5 bis 30 Kohlenstoffatomen, beispielsweise substituierte oder unsubstituierte Alkyl-, Aryl-, Arylalkyl-, Cycloalkyl- und/oder Heteroarylgruppen. Wegen ihrer erhöhten Hydrolyse- und Abbaustabillität werden hierfür insbesondere sterisch gehinderte Monophosphit-Liganden bevorzugt, wie sie beispielsweise in EP-A 155 508 beschrieben sind. Lediglich zum Zwecke der Veranschaulichung seien beispielhaft die folgenden Monophosphit-Ligandstrukturen genannt:

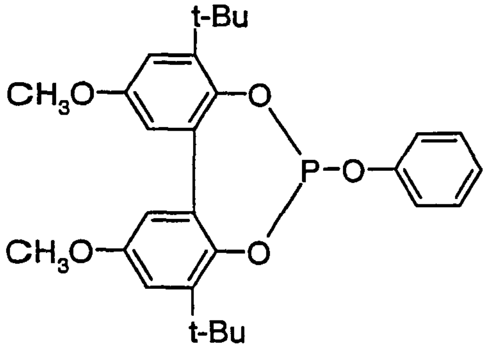

129

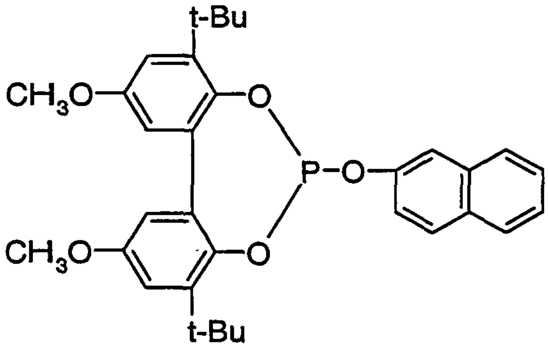

130

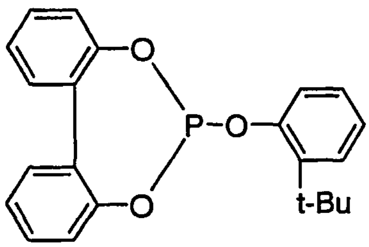

131

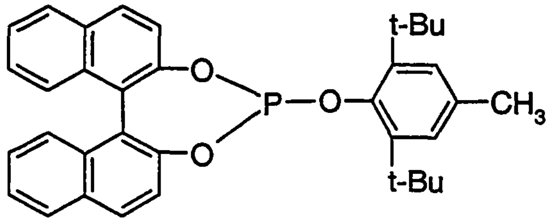

132

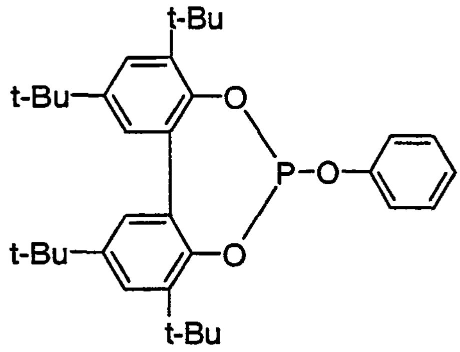

133

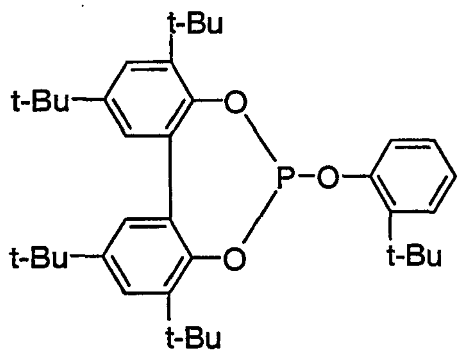

134

Bekannte Liganden zur isomerisierenden Hydroformylierung mit Rhodiumkomplexkatalysatoren sind auch solche zweizähnigen Liganden, die im Ligandmolekül neben einer Phosphitgruppe noch eine Phosphinit- oder Phosphingruppe tragen. Solche Liganden werden u.a. in WO 99/50214 beschrieben. Lediglich zur Erläuterung seien im Folgenden einige solcher Liganden exemplarisch aufgeführt:

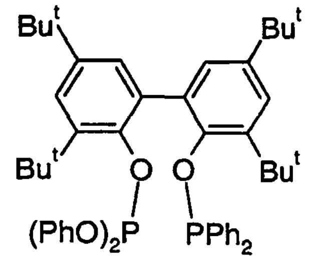

135

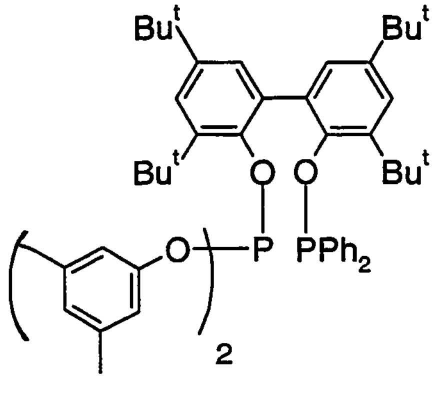

136

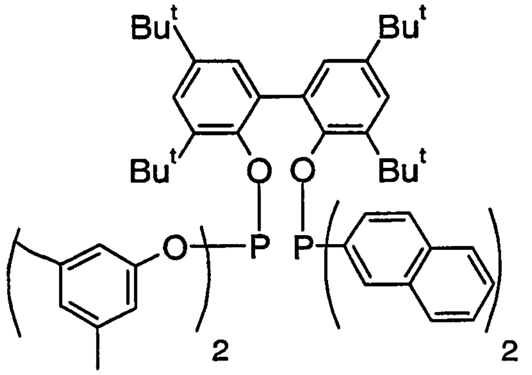

137

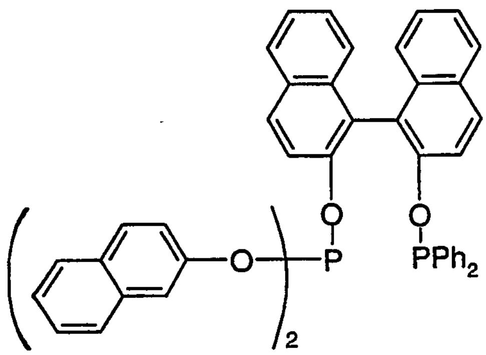

138

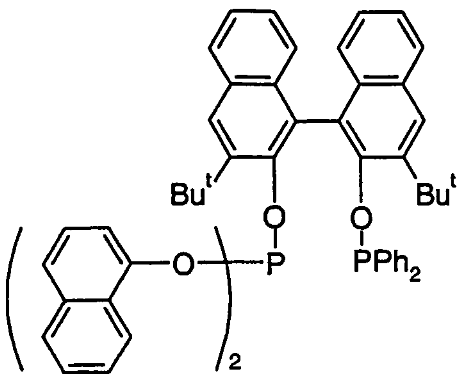

139

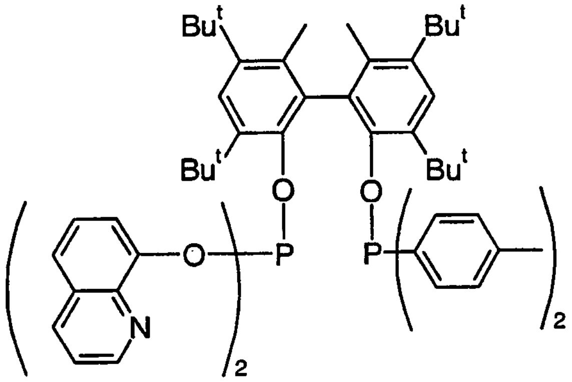

140

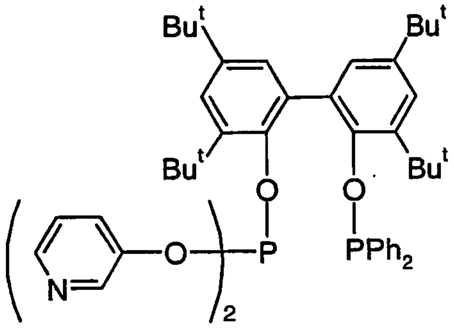

141

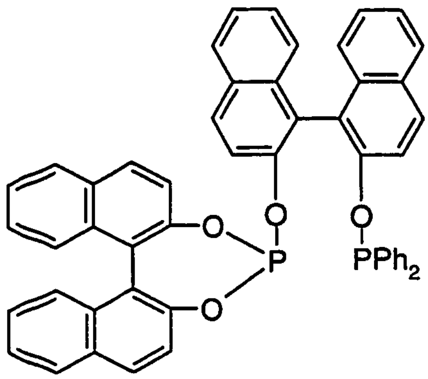

142

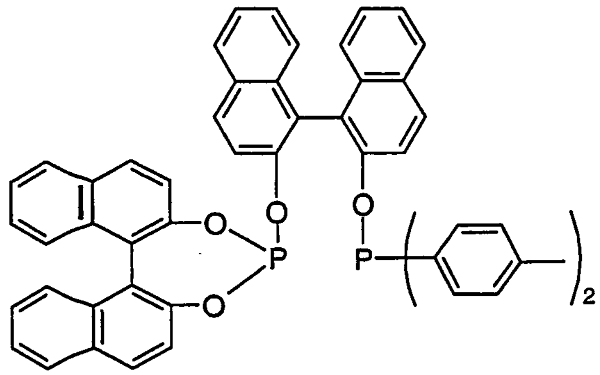

143

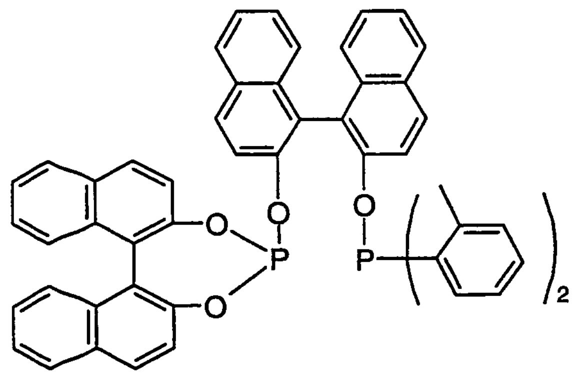

144

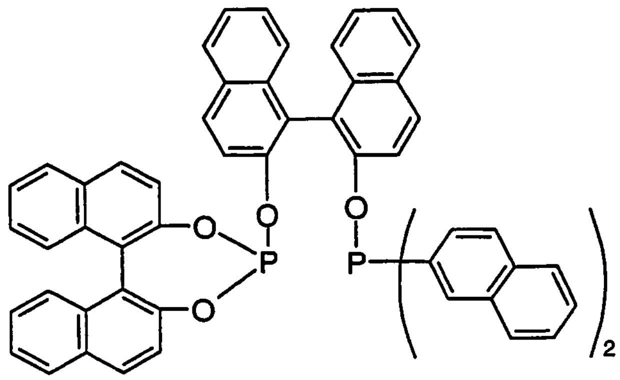

145

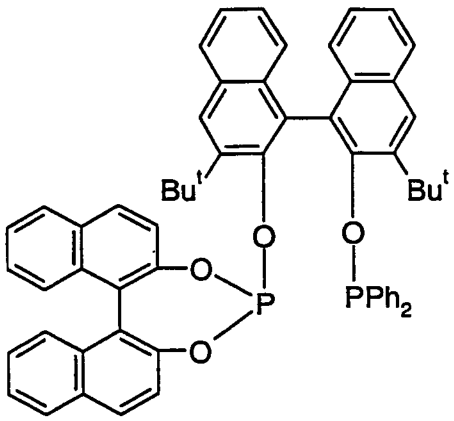

146

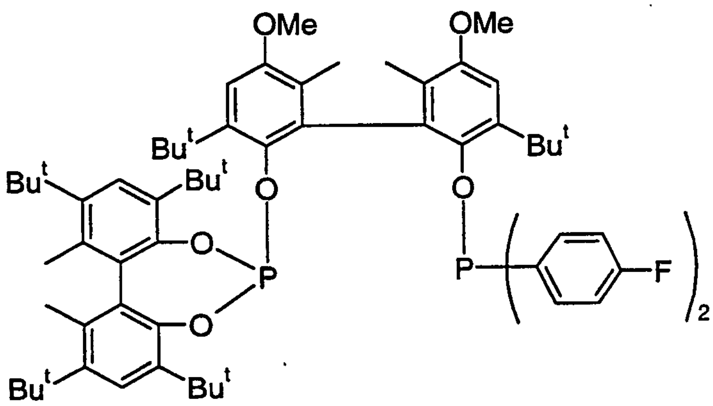

147

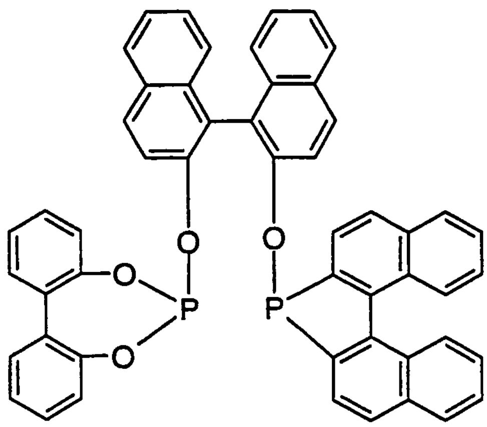

148

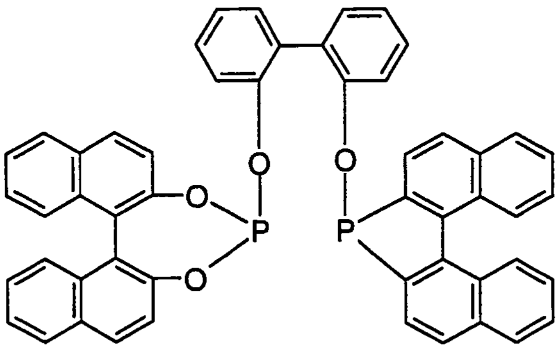

149

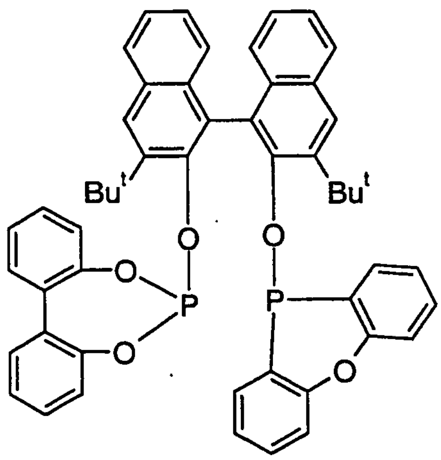

150

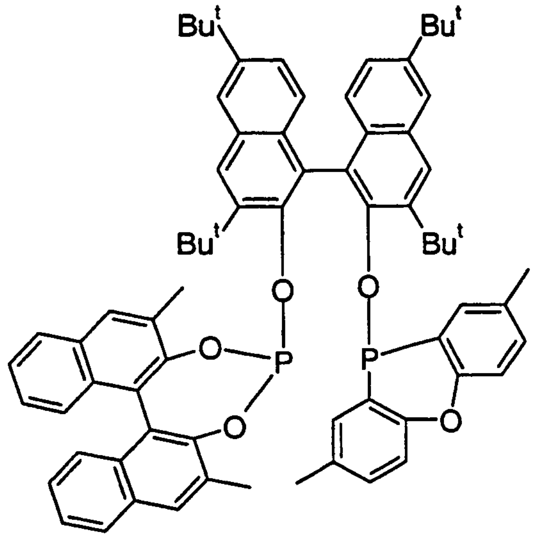

151

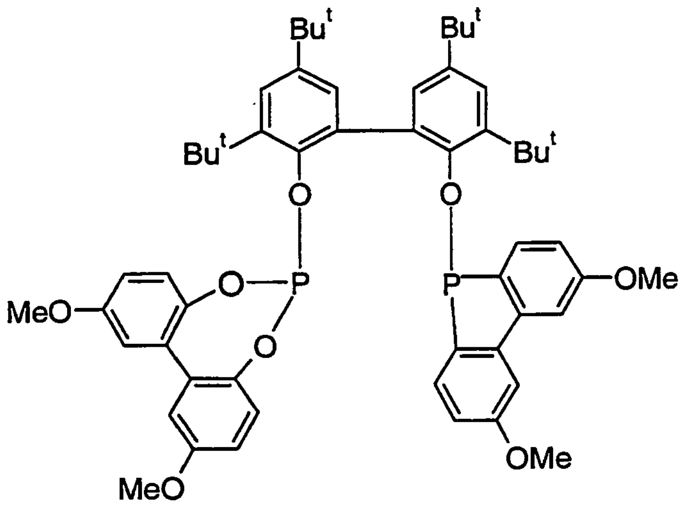

152

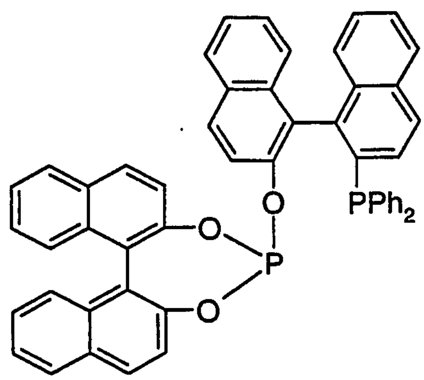

153

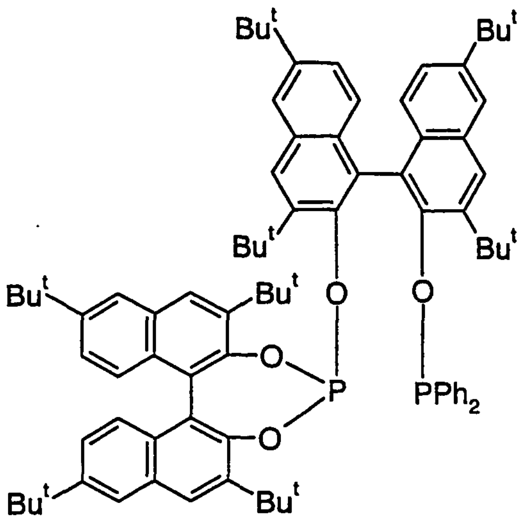

154

$Bu^t$: tertiär Butyl
Ph: Phenyl

Die vorstehend beschriebenen Ligandtypen können in Form ihrer Komplexe mit Rhodium und/oder als freie Liganden im erfindungsgemäßen Verfahren verwendet werden, bevorzugt werden im erfindungsgemäßen Verfahren zur isomerisierenden Hydroformylierung Rhodiumkomplexkatalysatoren mit Phosphoramiditliganden mit Xanthen-Rückgrat, wie sie Gegenstand der WO 02/083695 sind, eingesetzt.

Mittels des erfindungsgemäßen Verfahrens lassen sich bei der Hydroformylierung von Olefinzusammensetzungen, die sowohl α-Olefine als auch Olefine mit internen Doppelbindungen enthalten, die entsprechenden Aldehyde mit einem höheren n/i-Verhältnis erhalten, als dies dem Anteil an α-Olefinen in der Olefinzusammensetzung entspricht und dies mit einer höheren Raum-Zeit-Ausbeute als sie mit herkömmlichen Verfahren ohne die erfindungsgemäße Kaskadierung in zwei oder mehrere Reaktionszonen erhältlich ist.

Das erfindungsgemäße Verfahren wird im Folgenden anhand von Beispielen erläutert.

Beispiele

Synthese von Ligand 53:

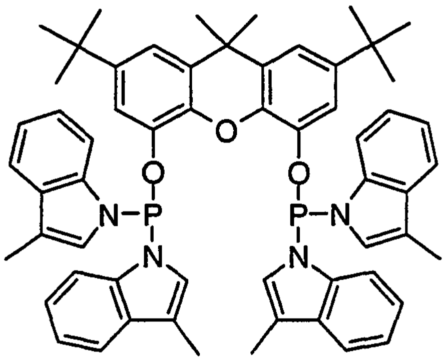

53

28.5 g (218 mmol) 3-Methylindol (Skatol) wurden in ca. 50 ml getrocknetem Toluol vorgelegt und das Lösungsmittel im Vakuum abdestilliert, um Wasserspuren azeotrop zu entfernen. Dieser Vorgang wurde noch einmal wiederholt. Der Rückstand wurde anschließend in 700 ml getrocknetem Toluol unter Argon aufgenommen und auf -65°C abgekühlt. Dann wurden 14.9 g (109 mmol) $PCl_3$ und danach 40 g (396 mmol) Triethylamin bei -65°C langsam zugegeben. Das Reaktionsgemisch wurde innerhalb von 16 h auf Raumtemperatur gebracht und danach 16 h unter Rückfluss erhitzt. Zum Reaktionsgemisch wurden 19.3 g (58 mmol) 4,5-Dihydroxy-2,7-di-tert-butyl-9,9-dimethylxanthen in 300 ml getrocknetem Toluol zum Reaktionsgemisch gegeben, danach 16 Stunden unter Rückfluss erhitzt und nach Abkühlung auf Raumtemperatur der ausgefallene farblose Feststoff (Triethylamin-Hydrochlorid) abgesaugt, das Lösungsmittel abdestilliert und der Rückstand zweimal aus heißem Ethanol umkristallisiert. Nach Trocknung im Vakuum wurden 36.3 g (71% der Theorie) eines farblosen Feststoffes erhalten. 31 P-NMR (298K): δ = 105.

Beispiel 1

Hydroformylierung von 1-Buten $CO:H_2$-Molverhältnis =1:1

5.5 mg $Rh(CO)_2acac$ (acac=Acetylacetonat) und 200 mg Ligand 53 wurden separat eingewogen, in je 5 g Toluol gelöst, vermischt und bei 90°C mit 10 bar Synthesegas ($CO:H_2$ =1:1) begast (Präformierung). Nach 1 h wurde entspannt. Dann wurden 9.9 g 1-Buten mittels Druckschleuse zugegeben, mit Synthesegas ($CO:H_2$ =1:1) ein Gesamtdruck von 17 bar eingestellt und 2 h bei 90°C hydroformyliert (109 ppm Rh; Ligand 53:Rh-Molverhältnis ≈ 10: 1). Nach der angegeben Reaktionszeit wurde der Autoklav abgekühlt, vorsichtig über eine Kühlfalle entspannt und die gesammelten Reaktionsausträge (Reaktor und Kühlfalle) mittels Gaschromatographie analysiert. Der Umsatz betrug 99%, die Ausbeute an Valeraldehyd 92% und die Linearität (n-Anteil) 98.5%. Die Ausbeute an 2-Buten (Isomerisierungsprodukt) betrug 7%.

Der CO- und $H_2$-Partialdruck zu Beginn der Reaktion betrugen jeweils 6 bar.

Die Linearität (n-Anteil) ist definiert als der Quotient aus n-Valeraldehyd zu der Summe aus n- Valeraldehyd und i-Valeraldehyd multipliziert mit 100.

Beispiel 2

Hydroformylierung von 1-Buten $CO:H_2$-Molverhältnis = 1:2

5.4 mg $Rh(CO)_2acac$ (acac=Acetylacetonat) und 200 mg Ligand 53 wurden separat eingewogen, in je 5 g Toluol gelöst, vermischt und bei 90°C mit 10 bar Synthesegas (CO:H2 =1:2) begast (Präformierung). Nach 1 h wurde entspannt. Dann wurden 10.1 g 1-Buten mittels Druckschleuse zugegeben, mit Synthesegas ($CO:H_2$ = 1:2) ein Gesamtdruck von 17 bar eingestellt. Die Gaszufuhr wurde dann auf Synthesegas ($CO:H_2$ = 1:1) umgestellt, um ein konstantes Molverhältnis von $CO:H_2$ von 1:2 im Reaktor zu gewährleisten. Anschließend wurde 2 h bei 90°C hydroformyliert (105 ppm Rh; Ligand 53:Rh-Molverhältnis ≈ 10:1). Der Umsatz betrug 98%, die Ausbeute an Valeraldehyd 49% und die Linearität (n-Anteil) 95.8%. Die Ausbeute an 2-Buten (isomerisierungsprodukt) betrug 46%.

Der CO-Partialdruck zu Beginn der Reaktion betrug 4 bar, der $H_2$-Partialdruck zu Beginn der Reaktion betrug 8 bar.

Beispiel 3

Hydroformylierung von 2-Buten $CO:H_2$-Molverhältnis = 1:1

5.0 mg $Rh(CO)_2acac$ (acac=Acetylacetonat) und 176 mg Ligand 53 wurden separat eingewogen, in je 5 g Toluol gelöst, vermischt und bei 90°C mit 10 bar Synthesegas ($CO:H_2$ = 1:1) begast (Präformierung). Nach 1 h wurde entspannt. Dann wurden 11.3 g 2-Buten mittels Druckschleuse zugegeben, mit Synthesegas ($CO:H_2$ =1:1) ein Gesamtdruck von 17 bar eingestellt. Anschließend wurde 4 h bei 90°C hydroformyliert (93 ppm Rh; Ligand 53:Rh-Molverhältnis = 10:1). Der Umsatz betrug 12%, die Ausbeute an Valeraldehyd 10% und die Linearität (n-Anteil) 88.5%.

Der CO- und $H_2$-Partialdruck zu Beginn der Reaktion betrugen jeweils 6 bar.

Beispiel 4

Hydroformylierung von 2-Buten $CO:H_2$-Molverhältnis = 1:2

5.0 mg $Rh(CO)_2acac$ (acac=Acetylacetonat) und 176 mg Ligand 53 wurden separat eingewogen, in je 5 g Toluol gelöst, vermischt und bei 90°C mit 10 bar Synthesegas ($CO:H_2$ = 1:2) begast (Präformierung). Nach 1 h wurde entspannt. Dann wurden 11.2 g 2-Buten mittels Druckschleuse zugegeben, mit Synthesegas ($CO:H_2$ =1:2) ein Gesamtdruck von 17 bar eingestellt. Die Gaszufuhr wurde dann auf Synthesegas ($CO:H_2$ = 1:1) umgestellt, um ein konstantes Molverhältnis von $CO:H_2$ von 1:2 im Reaktor zu gewährleisten. Anschließend wurde 4 h bei 90°C hydroformyliert (93 ppm Rh; Ligand 53:Rh-Molverhältnis ≈ 10:1). Der Umsatz betrug 34%, die Ausbeute an Valeraldehyd 32% und die Linearität (n-Anteil) 93%.

Der CO-Partialdruck zu Beginn der Reaktion betrug 4 bar, der $H_2$-Partialdruck zu Beginn der Reaktion betrug 8 bar.

Beispiel 5

Hydroformylierung von 2-Buten $CO:H_2$-Molverhältnis =1:9

5.0 mg $Rh(CO)_2acac$ (acac=Acetylacetonat) und 176 mg Ligand 53 wurden separat eingewogen, in je 5 g Toluol gelöst, vermischt und bei 90°C mit 10 bar Synthesegas ($CO:H_2$ = 1:9) begast (Präformierung). Nach 1 h wurde entspannt. Dann wurden 11.2 g 2-Buten mittels Druckschleuse zugegeben, mit Synthesegas ($CO:H_2$ = 1:9) ein Gesamtdruck von 17 bar eingestellt. Die Gaszufuhr wurde dann auf Synthesegas ($CO:H_2$ = 1:1) umgestellt, um ein konstantes Molverhältnis von $CO:H_2$ von 1:9 im Reaktor zu gewährleisten. Anschließend wurde 4 h bei 90°C hydroformyliert (93 ppm Rh; Ligand 53:Rh-Molverhältnis ≈ 10:1). Der Umsatz betrug 64%, die Ausbeute an Valeraldehyd 46% und die Linearität (n-Anteil) 96%.

Der CO-Partialdruck zu Beginn der Reaktion betrug 1,2 bar, der $H_2$-Partialdruck zu Beginn der Reaktion betrug 10,8 bar.

Beispiel 6

Hydroformylierung von trans-2-Buten $CO:H_2$ =1:1

5,0 mg $Rh(CO)_2acac$ (acac= Acetylacetonat) und 187 mg Ligand 53 wurden separat eingewogen, in je 5 g

Toluol gelöst, vermischt und bei 90°C mit 10 bar Synthesegas ($CO:H_2$ = 1:1) begast (Präformierung). Nach 1 h wurde entspannt. Dann wurden 10,4 g trans-2-Buten mittels Druckschleuse zugegeben und mit Synthesegas ($CO:H_2$ = 1:1) ein Gesamtdruck von 12 bar eingestellt. Anschließend wurde 4 h bei 90°C hydroformyliert (97 ppm Rh; Ligand 53:Rh = 10:1). Der.Umsatz betrug 33 %, die Ausbeute an Valeraldehyd 30 % und die Linearität (n-Anteil) 95 %.

Der CO- und $H_2$-Partialdruck zu Beginn der Reaktion betrugen jeweils 3,5 bar.

Beispiel 7

Kontinuierlich betriebene Hydroformylierung von Raffinat 11 mit Rh/Ligand 53

In einer kontinuierlich betriebenen Apparatur gemäß Zeichnung, bestehend aus zwei in Reihe geschalteten Rührautoklaven (1 und 2) mit je 1 l Flüssigvolumen, einem Druckabscheider (3), einem entspannten und beheizten Behälter zur Abtrennung von C4-Kohlenwasserstoffen (4) sowie einem Wischblattverdampfer (5) zur Abtrennung der katalysatorhaltigen Schwersiederphase von der Produktphase, wurde Raffinat II (29 Gew.-% 1-Buten, 52 Gew.-% 2-Buten, 19 Gew.-% Butane und sonstige C4-Kohlenwasserstoffe) mit Rhodium und Ligand 53 als Katalysator hydroformyliert. Der Katalysatorrückstrom aus der Destillation (5) in die Reaktoren betrug ca. 130 g/h, die Raffinat-Zufuhr ca. 70 g/h. Die Rhodium-Konzentration in den Reaktoren betrug etwa 100 ppm bei einem Ligand-Metall-Molverhältnis von ≈ 10:1 (mol:mol), die Temperatur des ersten Reaktors betrug 70°C und die des zweiten Reaktors 90°C. Der erste Reaktor wurde mit Synthesegas eines $CO:H_2$ Molverhältnisses von 1:1 versorgt und bei einem Gesamtdruck von ca. 22 bar betrieben. Durch zusätzliche Aufgabe von $H_2$ auf den zweiten Reaktor, der bei ca. 20 bar betrieben wurde, konnte der CO-Gehalt des Synthesegases abgasgeregelt beliebig zwischen ca. 50% CO bis ca. 1 % CO eingestellt werden. In einem repräsentativen Zeitraum von 13 Tagen stationärem Betrieb bei einem $CO:H_2$-Molverhältnis von 1:25 im zweiten Reaktor (Erster Reaktor: $CO:H_2$-Molverhältnis =1:1) wurde bei einer Aldehydausbeute von 49% ein n-Anteil von 96.1 % erreicht.

**Patentansprüche**

1. Verfahren zur kontinuierlichen Herstellung von Aldehyden mit 5 bis 21 Kohlenstoffatomen durch die isomerisierende Hydroformylierung in homogener Phase von $\alpha$-Olefine und Olefine mit internen Doppelbindungen enthaltenden Olefinzusammensetzungen mit 4 bis 20 Kohlenstoffatomen mittels Synthesegas in Gegenwart eines mit einem Sauerstoff- und/oder Stickstoffatome enthaltenden Organophosphorliganden komplexierten, homogenen Rhodium-Katalysators und freiem Liganden, bei erhöhter Temperatur und bei erhöhtem Druck in einem mehrstufigen Reaktionssystem aus mindestens zwei Reaktionszonen, **dadurch gekennzeichnet, dass** man die Olefinzusammensetzung zunächst in einer Gruppe aus einer oder mehreren ersten Reaktionszonen bei einem Gesamtdruck von 10 bis 40 bar mit Synthesegas eines $CO/H_2$-Molverhältnis von 4:1 bis 1:2 bis zu einem Umsatz der $\alpha$-Olefine von 40 bis 95 % umsetzt und den Hydroformylierungsaustrag aus dieser Gruppe aus einer oder mehreren ersten Reaktionszonen in einer Gruppe aus einer oder mehreren nachfolgenden Reaktionszonen bei einem Gesamtdruck von 5 bis 30 bar mit Synthesegas eines $CO/H_2$-Molverhältnisses von 1:4 bis 1:1000 umsetzt, wobei der Gesamtdruck in der einen oder mehreren nachfolgenden Reaktionszonen jeweils um 1 bis (G1-Gf) bar niedriger ist als in der jeweils vorausgehenden Reaktionszone, worin G1 für den Gesamtdruck in der jeweils vorausgehenden Reaktionszone und Gf für den Gesamtdruck in der jeweils der einen oder mehreren ersten Reaktionszonen jeweils nachfolgenden Reaktionszone steht, mit der Maßgabe dass die Differenz G1-Gf größer als 1 bar ist und wobei der CO-Partialdruck in der einen oder mehreren nachfolgenden Reaktionszonen jeweils niedriger ist als in der dieser vorausgehenden Reaktionszone.

2. Verfahren gemäß Anspruch 1, **dadurch gekennzeichnet, dass** man in der einen oder mehreren ersten Reaktionszonen ein $CO/H_2$-Molverhältnis von 3:2 bis 2:3 und in der einen oder mehreren nachfolgenden ein $CO/H_2$-Molverhältnis von 1:9 bis 1:100 einstellt.

3. Verfahren gemäß den Ansprüchen 1 oder 2, **dadurch gekennzeichnet, dass** man das Verfahren in zwei Reaktionszonen durchführt.

4. Verfahren gemäß den Ansprüchen 1 bis 3, **dadurch gekennzeichnet, dass** man zur Einstellung des $CO/H_2$-Molverhältnisses in der der einen oder mehreren ersten Reaktionszonen nachfolgenden Reaktionszonen wasserstoffhaltige Abgase aus Aldehyd und Enal-Hydrierverfahren verwendet.

5. Verfahren gemäß den Ansprüchen 1 bis 4, **dadurch gekennzeichnet, dass** man als homogenen Hydroformylierungskatalysator Komplexe des Rhodiums mit einem Phosphoramiditliganden der allgemeinen Formel I verwendet,

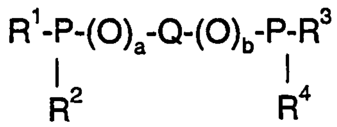

I

in der

Q eine Brückengruppe der Formel

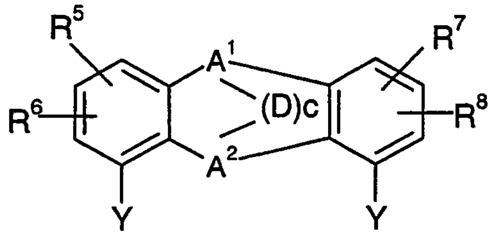

ist,
worin

$A^1$ und $A^2$ unabhängig voneinander für O, S, $SiR^aR^b$, $NR^c$ oder $CR^dR^e$ stehen, wobei
$R^a$, $R^b$ und $R^c$ unabhängig voneinander für Wasserstoff, Alkyl, Cycloalkyl, Heterocycloalkyl, Aryl oder Hetaryl stehen,
$R^d$ und $R^e$ unabhängig voneinander für Wasserstoff, Alkyl, Cycloalkyl, Heterocycloalkyl, Aryl oder Hetaryl stehen oder gemeinsam mit dem Kohlenstoffatom, an das sie gebunden sind, eine Cycloalkylidengruppe mit 4 bis 12 Kohlenstoffatomen bilden oder die Gruppe $R^d$ gemeinsam mit einer weiteren Gruppe $R^d$ oder die Gruppe $R^e$ gemeinsam mit einer weiteren Gruppe $R^e$ eine intramolekulare Brückengruppe D bilden,
D eine zweibindige Brückengruppe, ausgewählt aus den Gruppen

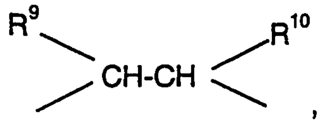 , 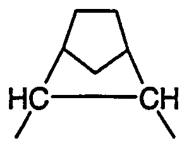 , 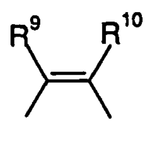 , 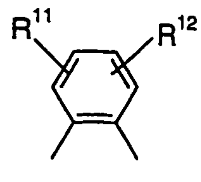 , 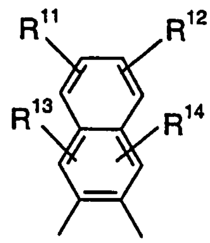

ist, in denen

$R^9$ und $R^{10}$ unabhängig voneinander für Wasserstoff, Alkyl, Cycloalkyl, Aryl, Halogen, Trifluormethyl, Carboxyl, Carboxylat oder Cyano stehen oder miteinander zu einer $C_3$- bis $C_4$-Alkylenbrücke verbunden sind,
$R^{11}$, $R^{12}$, $R^{13}$ und $R^{14}$ unabhängig voneinander für Wasserstoff, Alkyl, Cycloalkyl, Aryl, Halogen, Trifluormethyl, COOH, Carboxylat, Cyano, Alkoxy, $SO_3H$ Sulfonat, $NE^1E^2$, Alkylen-$NE^1E^2E^{3+}X^-$, Acyl oder Nitro stehen,
c 0 oder 1 ist,
Y eine chemische Bindung darstellt,
$R^5$, $R^6$, $R^7$und $R^8$ unabhängig voneinander für Wasserstoff, Alkyl, Cycloalkyl, Heterocycloalkyl, Aryl, Hetaryl, $COOR^f$, $COO^-M^+$, $SO_3R^f$, $SO^-{}_3M^+$, $NE^1E^2$, $NE^1E^2E^{3+}X^-$, Alkylen-$NE^1E^2E^{3+}X^-$, $OR^f$, $SR^f$, $(CHR^9CH_2O)_xR^f$, $(CH_2N(E^1))_xR^f$, $(CH_2CH_2N(E^1))_xR^f$, Halogen, Trifluormethyl, Nitro, Acyl oder Cyano stehen,

worin

$R^f$, $E^1$, $E^2$ und $E^3$ jeweils gleiche oder verschiedene Reste, ausgewählt unter Wasserstoff, Alkyl, Cycloalkyl oder Aryl bedeuten,
$R^9$ für Wasserstoff, Methyl oder Ethyl steht,
$M^+$ für ein Kation steht,
$X^-$ für ein Anion steht, und
x für eine ganze Zahl von 1 bis 120 steht,

oder

$R^5$ und/oder $R^7$ zusammen mit zwei benachbarten Kohlenstoffatomen des Benzolkerns, an den sie gebunden sind, für ein kondensiertes Ringsystem, mit 1, 2 oder 3 weiteren Ringen stehen,
a und b unabhängig voneinander die Zahl 0 oder 1 bedeuten
P für ein Phosphoratom steht,

und

$R^1$, $R^2$, $R^3$, $R^4$ unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine $NE^1E^2$-Gruppe stehen, mit der Maßgabe, dass $R^1$ und $R^3$ über das Stickstoffatom an das Phosphoratom P gebundene Pyrrolgruppen sind oder worin $R^1$ gemeinsam mit $R^2$ und/oder $R^3$ gemeinsam mit $R^4$ eine mindestens eine über das pyrrolische Stickstoffatom an das Phosphoratom P gebundene Pyrrolgruppe enthaltende zweibindige Gruppe E der Formel

Py-I-W

bilden, worin

Py eine Pyrrolgruppe ist,
I für eine chemische Bindung oder für O, S, $SiR^aR^b$, $NR^c$ oder $CR^hR^i$ steht,
W für Cycloalkyl, Cycloalkoxy, Aryl, Aryloxy, Hetaryl oder Hetaryloxy steht,

und

$R^h$ und $R^i$ unabhängig voneinander für Wasserstoff, Alkyl, Cycloalkyl, Heterocycloalkyl, Aryl oder Hetaryl stehen,

oder eine über die Stickstoffatome an das Phosphoratom P gebundene Bispyrrolgruppe der Formel.

Py-I-Py

bilden.

6. Verfahren gemäß den Ansprüchen 1 bis 5, **dadurch gekennzeichnet, dass** man als homogenen Hydroformylierungskatalysator Komplexe des Rhodiums mit einem Phosphoramiditliganden der allgemeinen Formel Ia verwendet

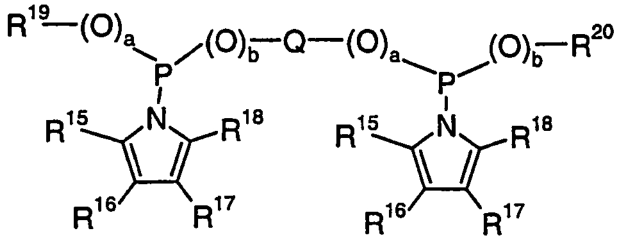

Ia

worin

$R^{15}$, $R^{16}$, $R^{17}$ und $R^{18}$ unabhängig voneinander für Wasserstoff, Alkyl, Cycloalkyl, Heterocycloalkyl, Aryl, Hetaryl, $W'COOR^k$, $W'COO^-M^+$, $W'(SO_3)R^k$, $W'(SO_3)^-M^+$, $W'PO_3(R^k)(R^l)$, $W'(PO_3)_2^-(M^+)_2$, $W'NE^4E^5$, $W'(NE^4E^5E^6)^+X^-$, $W'OR^k$, $W'SR^k$, $(CHR^lCH_2O)_yR^k$, $(CH_2NE^4)_yR^k$, $(CH_2CH_2NE^4)_yR^k$, Halogen, Trifluormethyl, Nitro, Acyl oder Cyano stehen,

worin

W' für eine Einfachbindung, ein Heteroatom oder eine zweiwertige verbrückende Gruppe mit 1 bis 20 Brückenatomen steht,
$R^k$, $E^4$, $E^5$, $E^6$ jeweils gleiche oder verschiedene Reste, ausgewählt unter Wasserstoff, Alkyl, Cycloalkyl oder Aryl bedeuten,
$R^l$ für Wasserstoff, Methyl oder Ethyl steht,
$M^+$ für ein Kationäquivalent steht,
$X^-$ für ein Anionäquivalent steht und
y für eine ganze Zahl von 1 bis 240 steht,

wobei jeweils zwei benachbarte Reste $R^{15}$, $R^{16}$, $R^{17}$ und $R^{18}$ zusammen mit den Kohlenstoffatomen des Pyrrolrings, an die sie gebunden sind, auch für ein kondensiertes Ringsystem mit 1, 2 oder 3 weiteren Ringen stehen können, mit der Maßgabe, dass wenigstens einer der Reste $R^{15}$, $R^{16}$, $R^{17}$ oder $R^{18}$ nicht für Wasserstoff steht, und dass $R^{19}$ und $R^{20}$ nicht mit einander verknüpft sind,

$R^{19}$ und $R^{20}$ unabhängig voneinander für Cycloalkyl, Heterocyloalkyl, Aryl oder Hetaryl stehen,
a und b unabhängig voneinander die Zahl 0 oder 1 bedeuten
P für ein Phosphoratom steht,
Q eine Brückengruppe der Formel

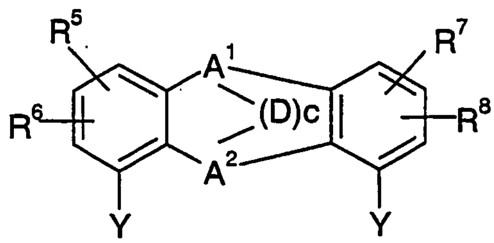

ist,
worin

$A^1$ und $A^2$ unabhängig voneinander für O, S, $SiR^aR^b$, $NR^c$ oder $CR^dR^e$ stehen, wobei
$R^a$, $R^b$ und $R^c$ unabhängig voneinander für Wasserstoff, Alkyl, Cycloalkyl, Heterocycloalkyl, Aryl oder Hetaryl stehen,
$R^d$ und $R^e$ unabhängig voneinander für Wasserstoff, Alkyl, Cycloalkyl, Heterocycloalkyl, Aryl oder Hetaryl stehen oder gemeinsam mit dem Kohlenstoffatom, an das sie gebunden sind, eine Cycloalkylidengruppe mit 4 bis 12 Kohlenstoffatomen bilden oder die Gruppe $R^d$ gemeinsam mit einer weiteren Gruppe $R^d$ oder die Gruppe $R^e$ gemeinsam mit einer weiteren Gruppe $R^e$ eine intramolekulare Brückengruppe D bilden,
D eine zweibindige Brückengruppe, ausgewählt aus den Gruppen

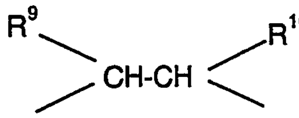 , 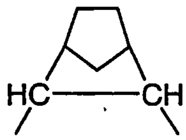 , 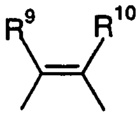 , 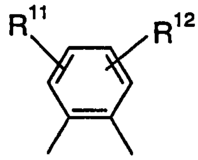 , 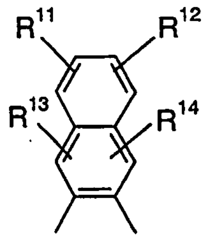

ist, in denen

$R^9$ und $R^{10}$ unabhängig voneinander für Wasserstoff, Alkyl, Cycloalkyl, Aryl, Halogen, Trifluormethyl, Carboxyl, Carboxylat oder Cyano stehen oder miteinander zu einer $C_3$- bis $C_4$-Alkylenbrücke verbunden sind,
$R^{11}$, $R^{12}$, $R^{13}$ und $R^{14}$ unabhängig voneinander für Wasserstoff, Alkyl, Cycloalkyl, Aryl, Halogen, Trifluormethyl, COOH, Carboxylat, Cyano, Alkoxy, $SO_3H$, Sulfonat, $NE^1E^2$, Alkylen-$NE^1E^2E^{3+}X^-$, Acyl oder Nitro stehen,
c 0 oder 1 ist,
$R^5$, $R^6$, $R^7$ und $R^8$ unabhängig voneinander für Wasserstoff, Alkyl, Cycloalkyl, Heterocycloalkyl, Aryl, Hetaryl, $COOR^f$, $COO^-M^+$, $SO_3R^f$, $SO^-{}_3M^+$, $NE^1E^2$, $NE^1E^2E^{3+}X^-$, Alkylen-$NE^1E^2E^{3+}X^-$, $OR^f$, $SR^f$, $(CHR^9CH_2O)_xR^f$, $(CH_2N(E^1))_xR^f$, $(CH_2CH_2N(E^1))_xR^f$, Halogen, Trifluormethyl, Nitro, Acyl oder Cyano stehen,

worin

$R^f$, $E^1$, $E^2$ und $E^3$ jeweils gleiche oder verschiedene Reste, ausgewählt unter Wasserstoff, Alkyl, Cycloalkyl oder Aryl bedeuten,
$R^g$ für Wasserstoff, Methyl oder Ethyl steht,
$M^+$ für ein Kation steht,
$X^-$ für ein Anion steht, und
x für eine ganze Zahl von 1 bis 120 steht,

oder

$R^5$ und/oder $R^7$ zusammen mit zwei benachbarten Kohlenstoffatomen des Benzolkerns, an den sie gebunden sind, für ein kondensiertes Ringsystem, mit 1, 2 oder 3 weiteren Ringen stehen.

7. Verfahren nach Anspruch 1, **dadurch gekennzeichnet, dass** man als Olefinzusammensetzung ein Raffinat 11 verwendet.

## Claims

1. A process for the continuous preparation of aldehydes having from 5 to 21 carbon atoms by isomerizing hydroformylation in the homogeneous phase of olefin compositions having from 4 to 20 carbon atoms and comprising $\alpha$-olefins and olefins having internal double bonds by means of synthesis gas in the presence of a homogeneous rhodium catalyst complexed with an oxygen- and/or nitrogencompriseing organophosphorus ligand and free ligand at elevated temperature and elevated pressure in a multistage reaction system comprising at least two reaction zones, wherein the olefin composition is firstly reacted with synthesis gas having a $CO/H_2$ molar ratio of from 4:1 to 1:2 at a total pressure of from 10 to 40 bar in a group of one or more first reaction zones to a conversion of the $\alpha$-olefins of from 40 to 95% and the hydroformylation mixture from this group of one or more first reaction zones is reacted with synthesis gas having a $CO/H_2$ molar ratio of from 1:4 to 1:1000 at a total pressure of from 5 to 30 bar in a group of one or more downstream reaction zones, where the total pressure in the one or more downstream reaction zones is in each case from 1 to (T1-Tf) bar lower than in the preceding reaction zone, where T1 is the total pressure in the preceding reaction zone and Tf is the total pressure in the reaction zone downstream of the one or more first reaction zones, with the proviso that the difference T1-Tf is greater than 1 bar, and the CO partial pressure in the one or more downstream reaction zones is in each case lower than in the reaction zone preceding this reaction zone.

2. A process as claimed in claim 1, wherein a $CO/H_2$ molar ratio of from 3:2 to 2:3 is set in the one or more first reaction zones and a $CO/H_2$ molar ratio of from 1:9 to 1:100 is set in the one or more downstream reaction zones.

3. A process as claimed in claim 1 or 2 which is carried out in two reaction zones.

4. A process as claimed in any of claims 1 to 3, wherein hydrogen-compriseing offgases from aldehyde and enal hydrogenation processes are used to set the $CO/H_2$ molar ratio in the reaction zones downstream of the one or more first reaction zones.

5. A process as claimed in any of claims 1 to 4, wherein the homogeneous hydroformylation catalyst used is a complex of rhodium with a phosphoramidite ligand of the formula I

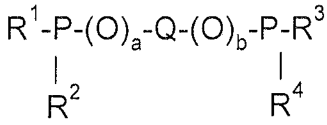

I

where

Q is a bridging group of the formula

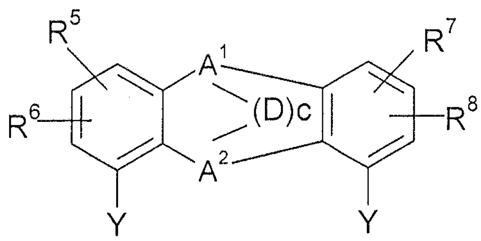

,

where

$A^1$ and $A^2$ are each, independently of one another, O, S, $SiR^aR^b$, $NR^c$ or $CR^dR^e$, where
$R^a$, $R^b$ and $R^c$ are each, independently of one another, hydrogen, alkyl, cycloalkyl, heterocycloalkyl, aryl or hetaryl,
$R^d$ and $R^e$ are each, independently of one another, hydrogen, alkyl, cycloalkyl, heterocycloalkyl, aryl or hetaryl or together with the carbon atom to which they are bound form a cycloalkylidene group having from 4 to 12 carbon atoms or the group $R^d$ together with a further group $R^d$ or the group $R^e$ together with a further group $R^e$ forms an intramolecular bridging group D,
D is a divalent bridging group selected from among the groups

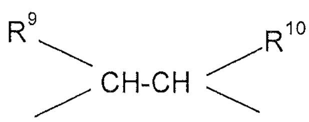

,

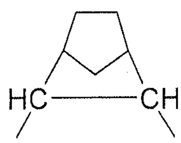

,

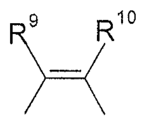

,

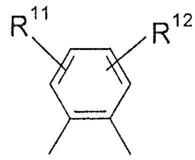

,

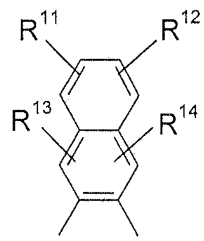

,

where

$R^9$ and $R^{10}$ are each, independently of one another, hydrogen, alkyl, cycloalkyl, aryl, halogen, trifluoromethyl, carboxyl, carboxylate or cyano or are joined to one another to form a $C_3$ - to $C_4$-alkylene bridge,
$R^{11}$, $R^{12}$, $R^{13}$ and $R^{14}$ are each, independently of one another, hydrogen, alkyl, cycloalkyl, aryl, halogen, trifluoromethyl, COOH, carboxylate, cyano, alkoxy, $SO_3H$, sulfonate, $NE^1E^2$, alkylene-$NE^1E^2E^{3+}X^-$, acyl or nitro,
c is 0 or 1,
Y is a chemical bond,
$R^5$, $R^6$, $R^7$ and $R^8$ are each, independently of one another, hydrogen, alkyl, cycloalkyl, heterocycloalkyl, aryl, hetaryl, $COOR^f$, $COO^-M^+$, $SO_3R^f$, $SO^-_3M^+$, $NE^1E^2$, $NE^1E^2E^{3+}X^-$, alkylene-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, halogen, trifluoromethyl, nitro, acyl or cyano,

where

$R^f$, $E^1$, $E^2$ and $E^3$ are identical or different radicals selected from among hydrogen, alkyl, cycloalkyl and aryl,
$R^g$ is hydrogen, methyl or ethyl,
$M^+$ is a cation,
$X^-$ is an anion and
x is an integer from 1 to 120,

or

$R^5$ and/or $R^7$ together with two adjacent carbon atoms of the benzene ring to which they are bound form a fused ring system having 1, 2 or 3 further rings,
a and b are each, independently of one another, 0 or 1,
P is a phosphorus atom,

and

$R^1$, $R^2$ $R^3$, $R^4$ are each, independently of one another, hetaryl, hetaryloxy, alkyl, alkoxy, aryl, aryloxy, cycloalkyl, cycloalkoxy, heterocycloalkyl, heterocycloalkoxy or an $NE^1E^2$ group, with the proviso that $R^1$ and $R^3$ are pyrrole groups bound via the nitrogen atom to the phosphorus atom P or $R^1$ together with $R^2$ and/or $R^3$ together with $R^4$ form a divalent group E which comprises at least one pyrrole group bound via the pyrrole nitrogen to the phosphorus atom P and has the formula

Py-I-W

where

Py is a pyrrole group,
I is a chemical bond or O, S, $SiR^aR^b$, $NR^c$ or $CR^hR^i$,
W is cycloalkyl, cycloalkoxy, aryl, aryloxy, hetaryl or hetaryloxy,

and

$R^h$ and $R^i$ are each, independently of one another, hydrogen, alkyl, cycloalkyl, heterocycloalkyl, aryl or hetaryl,

or form a bispyrrole group which is bound via the nitrogen atoms to the phosphorus atom P and has the formula

Py-I-Py.

**6.** A process as claimed in any of claims 1 to 5, wherein the homogeneous hydroformylation catalyst used is a complex of rhodium with a phosphoramidite ligand of the formula Ia

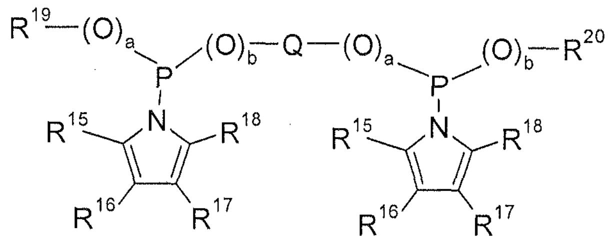

Ia

where

$R^{15}$, $R^{16}$, $R^{17}$ and $R^{18}$ are each, independently of one another, hydrogen, alkyl, cycloalkyl, heterocycloalkyl , aryl, hetaryl , $W'COOR^k$, $W'COO^-M^+$, $W'(SO_3)R^k$, $W'(SO_3)^-M^+$, $W'PO_3(R^k)(R^l)$, $W'(PO_3)_2^-(M^+)_2$, $W'NE^4E^5$, $W'(NE^4E^5E^6)^+X^-$, $W'OR^k$, $W'SR^k$, $(CHR^1CH_2O)_yR^k$, $(CH^2NE^4)_yR^k$, $(CH_2CH_2NE^4)_yR^k$, halogen, trifluoromethyl, nitro, acyl or cyano,

where

W' is a single bond, a heteroatom or a divalent bridging group having from 1 to 20 bridge atoms,
$R^k$, $E^4$, $E^5$, $E^6$ are identical or different radicals selected from among hydrogen, alkyl, cycloalkyl and aryl,
$R^1$ is hydrogen, methyl or ethyl,
$M^+$ is a cation equivalent,
$X^-$ is an anion equivalent and
y is an integer from 1 to 240,

where two adjacent radicals $R^{15}$, $R^{16}$, $R^{17}$ and $R^{18}$ together with the carbon atoms of the pyrrole ring to which they are bound may also form a fused ring system having 1, 2 or 3 further rings,
with the proviso that at least one of the radicals $R^{15}$, $R^{16}$ $R^{17}$ and $R^{18}$ is not hydrogen and $R^{19}$ and $R^{20}$ are not linked to one another,

$R^{19}$ and $R^{20}$ are each, independently of one another, cycloalkyl, heterocycloalkyl, aryl or hetaryl,
a and b are each, independently of one another, 0 or 1,
P is a phosphorus atom,
Q is a bridging group of the formula

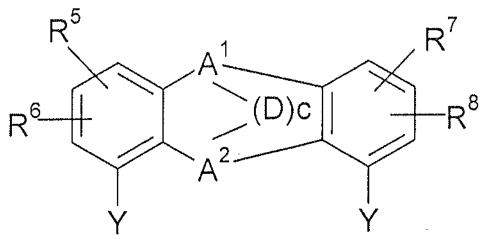

,

where

$A^1$ and $A^2$ are each, independently of one another, O, S, $SiR^aR^b$, $NR^c$ or $CR^dR^e$, where
$R^a$ $R^b$ and $R^c$ are each, independently of one another, hydrogen, alkyl, cycloalkyl, heterocycloalkyl, aryl or hetaryl,
$R^d$ and $R^e$ are each, independently of one another, hydrogen, alkyl, cycloalkyl, heterocycloalkyl, aryl or hetaryl or together with the carbon atom to which they are bound form a cycloalkylidene group having from 4 to 12 carbon atoms or the group $R^d$ together with a further group $R^d$ or the group $R^e$ together with a further group $R^e$ forms an intramolecular bridging group D,
D is a divalent bridging group selected from among the groups

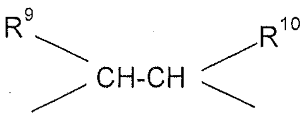 , 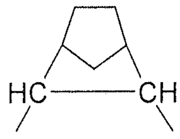 , 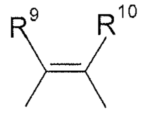 , 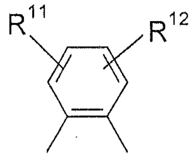 , 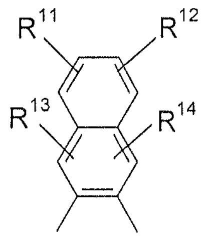 ,

where

$R^9$ and $R^{10}$ are each, independently of one another, hydrogen, alkyl, cycloalkyl, aryl, halogen, trifluoromethyl, carboxyl, carboxylate or cyano or are joined to one another to form a $C_3$- to $C_4$-alkylene bridge,
$R^{11}$, $R^{12}$, $R^{13}$ and $R^{14}$ are each, independently of one another, hydrogen, alkyl, cycloalkyl, aryl, halogen, trifluoromethyl, COOH, carboxylate, cyano, alkoxy, $SO_3H$, sulfonate, $NE^1E^2$, alkylene-$NE^1E^2E^{3+}X^-$, acyl or nitro,
c is 0 or 1,
$R^5$, $R^6$, $R^7$ and $R^8$ are each, independently of one another, hydrogen, alkyl, cycloalkyl, heterocycloalkyl, aryl, hetaryl, $COOR^f$, $COO^-M^+$, $SO_3R^f$, $SO^-_3M^+$, $NE^1E^2$, $NE^1E^2E^{3+}X^-$, alkylene-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, halogen, trifluoromethyl, nitro, acyl or cyano,

where

$R^f$, $E^1$, $E^2$ and $E^3$ are identical or different radicals selected from among hydrogen, alkyl, cycloalkyl and aryl,
$R^g$ is hydrogen, methyl or ethyl,
$M^+$ is a cation,
$X^-$ is an anion and
x is an integer from 1 to 120,

or

$R^5$ and/or $R^7$ together with two adjacent carbon atoms of the benzene ring to which they are bound form a fused ring system having 1, 2 or 3 further rings.

7. A process as claimed in claim 1, wherein the olefin composition used is a raffinate II.

## Revendications

1. Procédé pour la fabrication en continu d'aldéhydes ayant 5 à 21 atomes de carbone, par le biais d'une réaction d'hydroformylation isomérisante en phase homogène de compositions d'oléfines, contenant des α-oléfines et des oléfines présentant des doubles liaisons internes, ayant 4 à 20 atomes de carbone, au moyen d'un gaz de synthèse, en présence d'un catalyseur de rhodium homogène complexé avec un ligand organophosphoré contenant des atomes d'oxygène et/ou d'azote, et d'un ligand libre, à température élevée et sous pression élevée selon un système de réaction en plusieurs étapes constitué d'au moins deux zones réactionnelles, **caractérisé en ce que** l'on fait d'abord réagir la composition d'oléfines dans une installation constituée d'une ou de plusieurs premières zones réactionnelles sous une pression totale de 10 à 40 bars avec un gaz de synthèse présentant un rapport molaire $CO/H_2$ de 4:1 à 1:2, jusqu'à ce que l'on ait atteint un taux de conversion de 40 à 95 % % des a-oléfines, et **en ce que** l'on fait réagir l'extrait d'hydroformylation, provenant de cette installation constituée d'une ou de plusieurs premières zones réactionnelles, dans une installation constituée d'une ou de plusieurs zones réactionnelles suivantes sous une pression totale de 5 à 30 bars avec un gaz de synthèse ayant un rapport molaire $CO/H_2$ de 1:4 à 1:1000, la pression totale appliquée dans lesdites une ou plusieurs zones réactionnelles suivantes étant, respectivement, inférieure d'une valeur de 1 à (G1-Gf) bars à la pression appliquée dans la zone réactionnelle respective précédente, G1 représentant la pression totale dans la zone réactionnelle respective précédente et Gf représentant la pression totale dans la zone réactionnelle suivant, respectivement, lesdites une ou plusieurs premières zones réactionnelles, à condition que la différence G1-Gf soit supérieure à 1 bar et que la pression partielle en CO dans lesdites une ou plusieurs zones réactionnelles suivantes soit, respectivement, inférieure à celle de cette zone

réactionnelle précédente.

2. Procédé selon la revendication 1, **caractérisé en ce que** l'on règle un rapport molaire $CO/H_2$ de 3:2 à 2:3 dans lesdites une ou plusieurs premières zones réactionnelles, et un rapport molaire $CO/H_2$ de 1:9 à 1:100 dans lesdites une ou plusieurs zones réactionnelles suivantes.

3. Procédé selon les revendications 1 ou 2, **caractérisé en ce que** l'on met en oeuvre le procédé dans deux zones réactionnelles.

4. Procédé selon les revendications 1 à 3, **caractérisé en ce que** l'on utilise, pour régler le rapport molaire $CO/H_2$ dans les zones réactionnelles suivant lesdites une ou plusieurs premières zones réactionnelles, des gaz d'évacuation contenant de l'hydrogène, provenant d'un procédé d'hydrogénation d'aldéhyde et d'énal.

5. Procédé selon les revendications 1 à 4, **caractérisé en ce que** l'on utilise, comme catalyseur d'hydroformylation homogène, des complexes du rhodium avec un ligand de phosphoramidite de formule générale I :

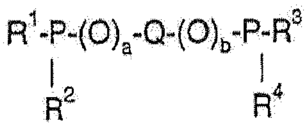
I

dans laquelle :

Q est un groupe de pontage de formule :

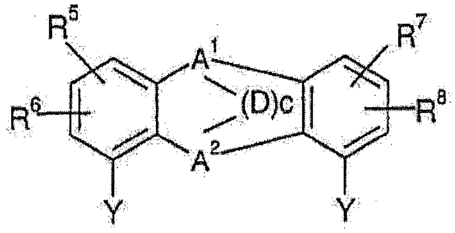

dans laquelle :

$A^1$ et $A^2$ représentent, indépendamment l'un de l'autre, O, S, $SiR^aR^b$, $NR^c$ ou $CR^dR^e$, où
$R^a$, $R^b$ et $R^c$ représentent, indépendamment l'un de l'autre, l'hydrogène, un groupe alkyle, cycloalkyle, hétérocycloalkyle, aryle ou hétaryle,
$R^d$ et $R^e$ représentent, indépendamment l'un de l'autre, l'hydrogène, un groupe alkyle, cycloalkyle, hétérocycloalkyle, aryle ou hétaryle ou forment, conjointement avec l'atome de carbone auquel ils sont liés, un groupe cycloalkylidène ayant 4 à 12 atomes de carbone, ou le groupe $R^d$ conjointement avec un autre groupe $R^d$, ou le groupe $R^e$ conjointement avec un autre groupe $R^e$, forment un groupe de pontage intramoléculaire D,
D représente un groupe de pontage divalent, choisi parmi les groupes suivants :

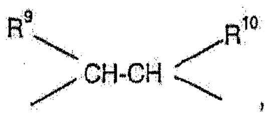 , 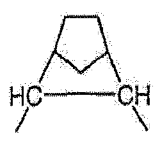 , 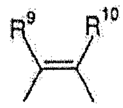 , 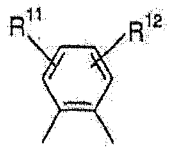 , 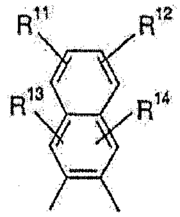

dans lesquels :

$R^9$ et $R^{10}$ représentent, indépendamment l'un de l'autre, l'hydrogène, un groupe alkyle, cycloalkyle, aryle, un halogène, un groupe trifluorométhyle, carboxyle, carboxylate ou cyano, ou sont reliés l'un à l'autre de manière à former un pont alkylène en $C_3$-$C_4$,
$R^{11}$, $R^{12}$, $R^{13}$ et $R^{14}$ représentent, indépendamment l'un de l'autre, l'hydrogène, un groupe alkyle, cycloalkyle, aryle, un halogène, un groupe trifluorométhyle, COOH, carboxylate, cyano, alcoxy, $SO_3H$, sulfonate, $NE^1E^2$, alkylène-$NE^1E^2E^{3+}X^-$, acyle ou nitro,
c est égal à 0 ou 1,
Y représente une liaison chimique,
$R^5$, $R^6$, $R^7$ et $R^8$ représentent, indépendamment l'un de l'autre, l'hydrogène, un groupe alkyle, cycloalkyle, hétérocycloalkyle, aryle, hétaryle, $COOR^f$, $COO^-M^+$, $SO_3R^f$, $SO^-_3M^+$, $NE^1E^2$, $NE^1E^2E^{3+}X^-$, alkylène-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, un halogène, un groupe trifluorométhyle, nitro, acyle ou cyano,

où

$R^f$, $E^1$, $E^2$ et $E^3$ représentent, respectivement, des radicaux identiques ou différents, choisis parmi l'hydrogène, un groupe alkyle, cycloalkyle ou aryle,
$R^g$ représente l'hydrogène, un méthyle ou un éthyle,
$M^+$ représente un cation,
$X^-$ représente un anion, et
x représente un nombre entier de 1 à 120,

ou

$R^5$ et/ou $R^7$ représentent, conjointement avec deux atomes de carbone adjacents du noyau benzénique auxquels ils sont liés, un système cyclique condensé avec 1, 2 ou 3 autres cycles,
a et b représentent, indépendamment l'un de l'autre, le chiffre 0 ou 1,
P représente un atome de phosphore,

et

$R^1$, $R^2$, $R^3$ et $R^4$ représentent, indépendamment l'un de l'autre, un groupe hétaryle, hétaryloxy, alkyle, alcoxy, aryle, aryloxy, cycloalkyle, cycloalcoxy, hétérocycloalkyle, hétérocycloalcoxy ou un groupe $NE^1E^2$, à condition que $R^1$ et $R^3$ soient des groupes pyrrole reliés à l'atome de phosphore P via l'atome d'azote, ou dans lequel $R^1$ conjointement avec $R^2$, et/ou $R^3$ conjointement avec $R^4$, forment un groupe E divalent contenant au moins un groupe pyrrole relié à l'atome de phosphore P via l'atome d'azote du pyrrole, de formule suivante :

Py-I-W

dans laquelle :

Py représente un groupe pyrrole,
I représente une liaison chimique ou 0, S, $SiR^aR^b$, $NR^c$ ou $CR^hR^i$,
W représente un groupe cycloalkyle, cycloalcoxy, aryle, aryloxy, hétaryle ou hétaryloxy,

et

$R^h$ et $R^i$ représentent, indépendamment l'un de l'autre, l'hydrogène, un groupe alkyle, cycloalkyle, hétérocycloalkyle, aryle ou hétaryle,

ou forment un groupe bispyrrole relié à l'atome de phosphore P via l'atome d'azote, de formule suivante :

Py-I-Py

**6.** Procédé selon les revendications 1 à 5, **caractérisé en ce que** l'on utilise, comme catalyseur d'hydroformylation homogène, des complexes du rhodium avec un ligand de phosphoramidite de formule générale Ia :

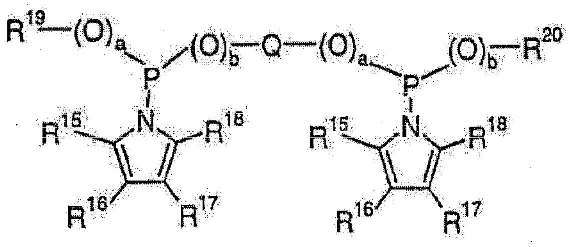

Ia

dans laquelle :

$R^{15}$, $R^{16}$, $R^{17}$ et $R^{18}$ représentent, indépendamment l'un de l'autre, l'hydrogène, un groupe alkyle, cycloalkyle, hétérocycloalkyle, aryle, hétaryle, $W'COOR^k$, $W'COO^-M^+$, $W'(SO_3)R^k$, $W'(SO_3)^-M^+$, $W'PO_3(R^k)(R^l)$, $W'(PO_3)_2^-(M^+)_2$, $W'NE^4E^5$, $W'(NE^4E^5E^6)^+X^-$, $W'OR^k$, $W'SR^k$, $(CHR^1CH_2O)_yR^k$, $(CH_2NE^4)_yR^k$, $(CH_2CH_2NE^4)_yR^k$, un halogène, un groupe trifluorométhyle, nitro, acyle ou cyano,

où

W' représente une liaison simple, un hétéroatome ou un groupe de pontage divalent ayant 1 à 20 atomes de pontage,
$R^k$, $E^4$, $E^5$ et $E^6$ représentent, respectivement, des radicaux identiques ou différents, choisis parmi l'hydrogène, un groupe alkyle, cycloalkyle ou aryle,
$R^l$ représente l'hydrogène, un méthyle ou un éthyle,
$M^+$ représente un équivalent cationique,
$X^-$ représente un équivalent anionique, et
y représente un nombre entier de 1 à 240,

où, respectivememt, deux radicaux adjacents $R^{15}$, $R^{16}$, $R^{17}$ et $R^{18}$, conjointement avec les atomes de carbone du cycle pyrrole auxquels ils sont liés, peuvent également représenter un système cyclique condensé avec 1, 2 ou 3 autres cycles,
à condition qu' au moins l'un des radicaux $R^{15}$, $R^{16}$, $R^{17}$ ou $R^{18}$ ne représente pas l'hydrogène et que $R^{19}$ et $R^{20}$ ne soient pas reliés l'un à l'autre,

$R^{19}$ et $R^{20}$ représentent, indépendamment l'un de l'autre, un groupe cycloalkyle, hétérocycloalkyle, aryle ou hétaryle,
a et b représentent, indépendamment l'un de l'autre, le chiffre 0 ou 1,
P représente un atome de phosphore,
Q est un groupe de pontage de formule suivante :

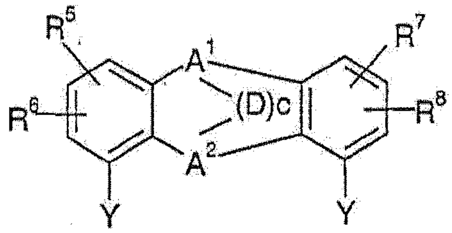

dans laquelle :

$A^1$ et $A^2$ représentent, indépendamment l'un de l'autre, 0, S, $SiR^aR^b$, $NR^c$ ou $CR^dR^e$, où
$R^a$, $R^b$ et $R^c$ représentent, indépendamment l'un de l'autre, l'hydrogène, un groupe alkyle, cycloalkyle, hétérocycloalkyle, aryle ou hétaryle,
$R^d$ et $R^e$ représentent, indépendamment l'un de l'autre, l'hydrogène, un groupe alkyle, cycloalkyle, hétérocycloalkyle, aryle ou hétaryle ou forment, conjointement avec l'atome de carbone auquel ils sont liés, un groupe cycloalkylidène ayant 4 à 12 atomes de carbone, ou le groupe $R^d$ conjointement avec un autre groupe $R^d$, ou

le groupe $R^e$ conjointement avec un autre groupe $R^e$, forment un groupe de pontage intramoléculaire D, D représente un groupe de pontage divalent, choisi parmi les groupes suivants :

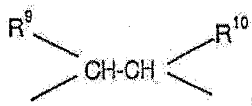, 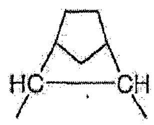, 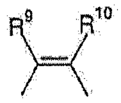, 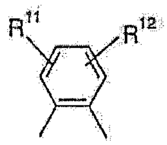, 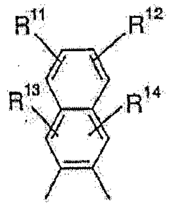

dans lesquels :

$R^9$ et $R^{10}$ représentent, indépendamment l'un de l'autre, l'hydrogène, un groupe alkyle, cycloalkyle, aryle, un halogène, un groupe trifluorométhyle, carboxyle, carboxylate ou cyano, ou sont reliés l'un à l'autre de manière à former un pont alkylène en $C_3$-$C_4$,
$R_{11}$, $R_{12}$, $R_{13}$ et $R_{14}$ représentent, indépendamment l'un de l'autre, l'hydrogène, un groupe alkyle, cycloalkyle, aryle, un halogène, un groupe trifluorométhyle, COOH, carboxylate, cyano, alcoxy, $SO_3H$, sulfonate, $NE^1E^2$, alkylène-$NE^1E^2E^{3+}X^-$, acyle ou nitro,
c est égal à 0 ou 1,
$R^5$, $R^6$, $R^7$ et $R^8$ représentent, indépendamment l'un de l'autre, l'hydrogène, un groupe alkyle, cycloalkyle, hétérocycloalkyle, aryle, hétaryle, $COOR^f$, $COO^-M^+$, $SO_3R^f$, $SO^-_3M^+$, $NE^1E^2$, $NE^1E^2E^{3+}X^-$, alkylène-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, un halogène, un groupe trifluorométhyle, nitro, acyle ou cyano,

où

$R^f$, $E^1$, $E^2$ et $E^3$ représentent, respectivement, des radicaux identiques ou différents, choisis parmi l'hydrogène, un groupe alkyle, cycloalkyle ou aryle,
$R^g$ représente l'hydrogène, un méthyle ou un éthyle,
$M^+$ représente un cation,
$X^-$ représente un anion, et
x représente un nombre entier de 1 à 120,

ou

$R^5$ et/ou $R^7$ représentent, conjointement avec deux atomes de carbone adjacents du noyau benzénique auxquels ils sont liés, un système cyclique condensé avec 1, 2 ou 3 autres cycles.

**7.** Procédé selon la revendication 1, **caractérisé en ce que** l'on utilise, comme composition d'oléfines, un produit de type raffinat II.

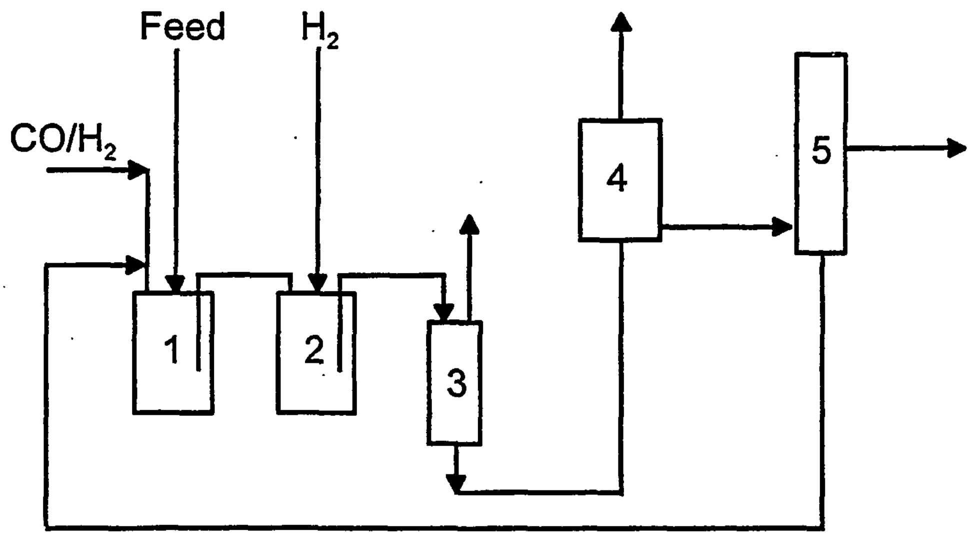
Feed
H2
CO/H2
1
2
3
4
5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3527809 A
- US 4668651 A
- WO 0158589 A
- WO 0283695 A
- US 4885401 A
- US 4599206 A
- EP 188426 A
- WO 9720801 A
- US 5744650 A
- US 5874639 A
- US 5728893 A
- JP 2000143572 A
- EP 423769 A
- EP 423769 B
- EP 1008580 A
- US 4716250 A
- WO 0268371 A
- US 4778929 A
- EP 1114017 A
- EP 1231198 A
- EP 1069101 A
- EP 1134271 A
- US 5849972 A
- WO 0053546 A
- US 4148830 A
- US 6100432 A
- WO 0158844 A
- WO 02083695 A
- WO 9819985 A
- WO 9952632 A
- WO 02068371 A
- EP 982314 A
- EP 213369 A
- US 4769498 A
- EP 155508 A
- WO 9950214 A


### In der Beschreibung aufgeführte Nicht-Patentliteratur


- **REINIUS et al.** *J. Mol. Cat. A: Chemical,* 2000, vol. 158, 499
- *Catalysis Today,* 2002, vol. 74, 111
- **WEISSERMEL, ARPE.** Industrielle Organische Chemie. Wiley-VCH, 1998, vol. 5, 82-99